# EUROPÄISCHE PATENTANMELDUNG

(11) **EP 0 736 569 A1**
(43) Veröffentlichungstag der Anmeldung: **09.10.1996**
(21) Anmeldenummer: 96810172.5
(22) Anmeldetag: 19.03.1996
(51) Int. Cl.: C08K 5/3462, C07D 487/04

(54) **Pyrrolodiazin-Derivate als Stabilisatoren für chlorhaltige Polymerisate**

(30) Priorität: 28.03.1995 EP 95810204
(71) Anmelder: CIBA-GEIGY AG, 4002 Basel (CH)
(72) Erfinder: Wehner, Wolfgang, Dr., 64372 Ober-Ramstadt (DE); Friedrich, Hans-Helmut, 64686 Lautertal (DE); Drewes, Rolf, Dr., 64678 Lindenfels (DE)

(57) **Zusammenfassung**

Es werden Zusammensetzungen beschrieben, die
a) ein chlorhaltiges Polymerisat und
b) mindestens eine Verbindung, die mindestens einen Rest der Formel I 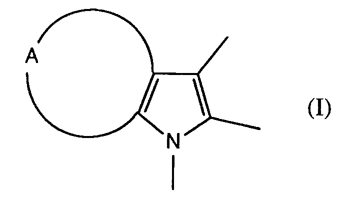 enthält, worin A die Ergänzung zu einem unsubstituierten oder substituierten sechs-gliedrigen heterocyclischen Ring mit zwei Stickstoffringatomen bedeutet.

## Beschreibung

Die vorliegende Erfindung betrifft Zusammensetzungen enthaltend ein chlorhaltiges Polymerisat, bevorzugt PVC, und ein Pyrrolodiazin-Derivat als Stabilisator, die Verwendung derselben zum Stabilisieren von chlorhaltigen Polymerisaten gegen oxidativen, thermischen oder lichtinduzierten Abbau, ein Verfahren zum Stabilisieren von PVC-Formmassen, ein Verfahren zur Herstellung der Stabilisatoren sowie neue Pyrrolodiazin-Derivate.

Es ist bekannt, dass chlorhaltige Polymerisate gegen den schädigenden Einfluss von Licht und Wärme, insbesondere bei der Verarbeitung zu Formteilen, geschützt werden müssen. Einige Pyrrol-Derivate und deren Verwendung als Costabilisatoren werden beispielsweise in US-A-4 369 276, US-A-5 155 152 oder US-A-5 288 776 beschrieben.

Diese bekannten Stabilisatoren und Stabilisatorengemische können in den chlorhaltigen Polymerisaten nicht allen gestellten Anforderungen entsprechen.

Einige Pyrrolodiazin-Derivate sind bekannt und werden beispielsweise in folgenden Publikationen beschrieben: N.M. Smirnova, L.F. Linberg, V.M. Nesterov und T.S. Safanova, Chemistry of Heterocyclic Compounds 1978, 443-446; H. Ogura, M. Sakaguchi und K. Takeda, Chem. Pharm. Bull. 20 (2), 404-408 (1972); S. Senda und K. Hirota, Chem. Pharm. Bull. 22 (12), 2921-28 (1974); S. Senda und K. Hirota, Chem. Pharm. Bull. 22 (7), 1459-1467 (1974); oder F. Yoneda, M. Higuchi, K. Senga, M. Kanohori und S. Nishigaki, Chem. Pharm. Bull. 21 (3), 473-477 (1973).

Es wurde nun gefunden, dass sich Pyrrolodiazin-Derivate besonders gut als Stabilisatoren für chlorhaltige Polymerisate, insbesondere für PVC, eignen.

Die vorliegende Erfindung betrifft daher Zusammensetzungen enthaltend
a) ein chlorhaltiges Polymerisat und
b) mindestens eine Verbindung, die mindestens einen Rest der Formel I 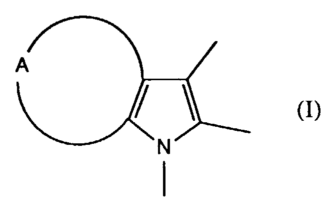
enthält, worin A die Ergänzung zu einem unsubstituierten oder substituierten sechs-gliedrigen heterocyclischen Ring mit zwei Stickstoffringatomen bedeutet.

A als Ergänzung zu einem unsubstituierten oder substituierten sechs-gliedrigen heterocyclischen Ring mit zwei Stickstoffringatomen bedeutet in der Formel I beispielsweise eine Gruppe der Formel a, b, c, d, e, f oder g 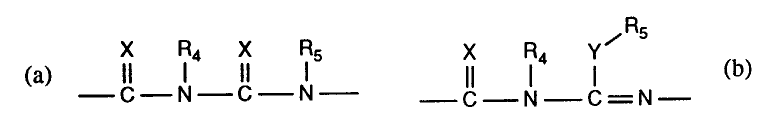 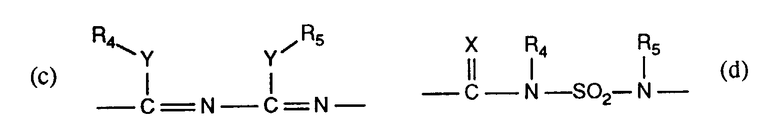 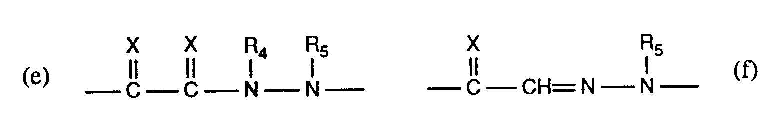 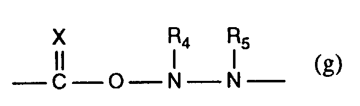 worin
X Sauerstoff oder Schwefel ist,
Y Sauerstoff, Schwefel oder 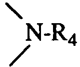 bedeutet,
R₄ und R₅ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl;
unsubstituiertes oder am Phenylring durch ein bis drei Reste R₆ substituiertes C₇-C₉-Phenylalkyl bedeuten, und
R₆ C₁-C₄-Alkyl, C₁-C₄-Alkoxy, C₅-C₈-Cycloalkyl, Hydroxy oder Chlor darstellt.

Die Verbindungen, die mindestens einen Rest der Formel I enthalten, zeichnen sich durch eine sehr gute stabilisierende Wirkung in chlorhaltigen Polymerisaten sowohl gegen oxidativen, thermischen als auch lichtinduzierten Abbau aus. Besonders bemerkenswert ist die farbstabilisierende Wirkung bei thermischer Belastung.

Von Interesse sind Zusammensetzungen enthaltend als Komponente b) mindestens eine Verbindung der Formel II 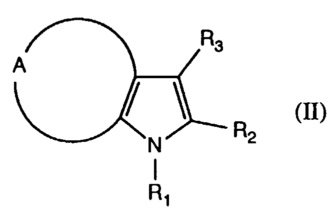 worin
A eine Gruppe der Formel III oder IV 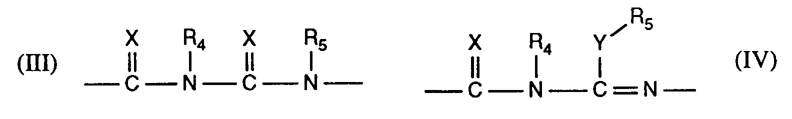 bedeutet,
X Sauerstoff oder Schwefel ist,
Y Sauerstoff, Schwefel oder 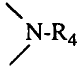 bedeutet,
R₁ Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; ein Alkalimetall oder ein Erdalkalimetall darstellt,
R₂ und R₃ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; 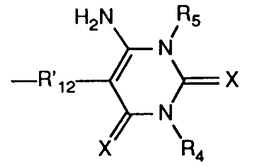 oder einen Rest der Formel V oder VI 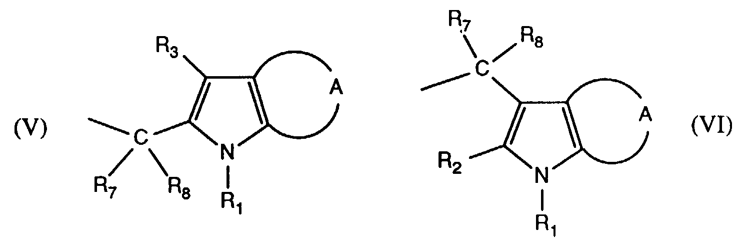 darstellen, worin A und R₁ die oben angegebene Bedeutung besitzen, oder ferner einer der Reste R₂ und R₃ Hydroxy, Formyl, C₁-C₁₂-Alkoxy, unsubstituiertes oder durch ein bis drei Reste R₆ substituiertes Phenoxy; C₁-C₁₂-Alkanoyloxy, unsubstituiertes oder durch ein bis drei Reste R₆ substituiertes Benzoyloxy; 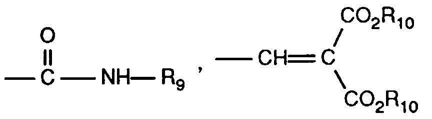 oder -CH=N-R₁₁ darstellt,
R₄ und R₅ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeuten,
R₆ C₁-C₄-Alkyl, C₁-C₄-Alkoxy, C₅-C₈-Cycloalkyl, Hydroxy oder Chlor darstellt,
R₇ und R₈ unabhängig voneinander Wasserstoff, CF₃, C₁-C₁₂-Alkyl, Phenyl oder C₇-C₉-Phenylalkyl darstellen, oder R₇ und R₈ zusammen mit dem C-Atom, an das sie gebunden sind, einen unsubstituierten oder durch C₁-C₄-Alkyl substituierten C₅-C₁₂-Cycloalkylidenring bilden,
R₉ unsubstituiertes oder durch ein bis drei Reste R₆ substituiertes Phenyl bedeutet;
R₁₀ Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeutet,
R₁₁ C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl darstellt,
R'₁₂ eine direkte Bindung oder 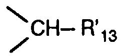 bedeutet, und
R'₁₃ Wasserstoff oder C₁-C₈-Alkyl darstellt.

Die Verbindungen der Formel II, worin A einen Rest der Formel III oder IV darstellt, bedeuten die Formeln VII, VIIa, VIII oder VIIIa.

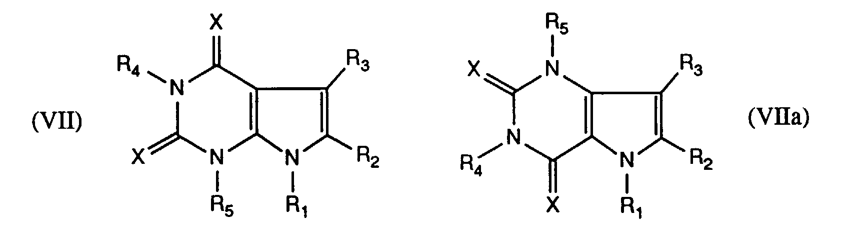

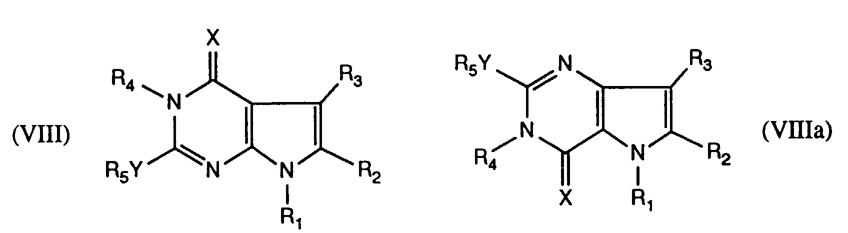

Alkyl mit bis zu 12 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl oder 1,1,3,3,5,5-Hexamethylhexyl. Eine der bevorzugten Bedeutungen von R₁, R₂, R₃, R₄, R₅, R₇, R₈, R₁₀ und R₁₁ ist beispielsweise C₁-C₁₀-Alkyl, insbesondere C₁-C₈-Alkyl, z.B. C₁-C₆-Alkyl. Eine speziell bevorzugte Bedeutung von R₁, R₂, R₃, R₄, R₅ ist C₁-C₄-Alkyl.

Durch Sauerstoff, Schwefel oder Carboxy unterbrochenes Alkyl mit 2 bis zu 12 Kohlenstoffatomen kann ein- oder mehrfach unterbrochen sein und bedeutet beispielsweise CH₃-O-CH₂-, CH₃-S-CH₂-, CH₃-O-CH₂CH₂-O-CH₂-, CH₃-(O-CH₂CH₂-)₂O-CH₂-, CH₃-(O-CH₂CH₂-)₃O-CH₂-, CH₃-(O-CH₂CH₂-)₄O-CH₂-, CH₃CO₂CH₂CH₂- oder CH₃CH₂CO₂CH₂CH₂-. Eine besonders bevorzugte Bedeutung von R₁, R₂, R₃, R₄, R₅, R₁₀ und R₁₁ ist beispielsweise durch Sauerstoff unterbrochenes C₂-C₁₂-Alkyl, insbesondere durch Sauerstoff unterbrochenes C₄-C₁₂-Alkyl, z.B. durch Sauerstoff unterbrochenes C₄-C₁₀-Alkyl.

Durch Hydroxy substituiertes Alkyl mit bis zu 12 Kohlenstoffatomen, das vorzugsweise 1 bis 3, insbesondere 1 oder 2 Hydroxygruppen enthält, bedeutet einen verzweigten oder unverzweigten Rest wie beispielsweise 2-Hydroxyethyl, 2-Hydroxypropyl, 3-Hydroxypropyl, 2,3-Dihydroxypropyl, 2-Hydroxybutyl, 2-Hydroxypentyl, 2-Hydroxyhexyl, 2-Hydroxyheptyl, 2-Hydroxyoctyl oder 2,5,6-Trihydroxyhexyl. Eine bensonders bevorzugte Bedeutung von R₁, R₂, R₃, R₄, R₅, R₁₀ und R₁₁ ist beispielsweise durch Hydroxy substituiertes C₁-C₁₀-Alkyl, insbesondere C₂-C₈-Alkyl. Speziell bevorzugt ist 2-Hydroxyethyl.

Alkenyl mit 3 bis 6 Kohlenstoffatomen bedeutet einen verzweigten oder unverzweigten Rest wie beispielsweise Propenyl, 2-Butenyl, 3-Butenyl, Isobutenyl, n-2,4-Pentadienyl, 3-Methyl-2-butenyl, 2-Pentenyl oder 2-Hexenyl. Eine besonders bevorzugte Bedeutung von R₁, R₂, R₃, R₄, R₅, R₁₀ und R₁₁ ist Propenyl.

Unsubstituiertes oder durch ein bis drei Reste R₆ substituiertes C₅-C₈-Cycloalkyl, das insbesondere 1 oder 2 verzweigte oder unverzweigte C₁-C₄-Alkylgruppen-Reste enthält, bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl.

Durch 1 bis 3 Reste R₆ substituiertes Phenyl, das vorzugsweise 1 oder 2 C₁-C₄-Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-butylphenyl, 2-Ethylphenyl oder 2,6-Diethylphenyl.

Unsubstituiertes oder am Phenylring durch 1 bis 3 Reste R₆ substituiertes C₇-C₉-Phenylalkyl, das insbesondere 1 oder 2 verzweigte oder unverzweigte C₁-C₄-Alkylgruppen-Reste enthält, bedeutet beispielsweise Benzyl, α-Methylbenzyl, α,α-Dimethylbenzyl, 2-Phenylethyl, 2-Methylbenzyl, 3-Methylbenzyl, 4-Methylbenzyl, 2,4-Dimethylbenzyl, 2,6-Dimethylbenzyl oder 4-tert-Butylbenzyl. Benzyl ist bevorzugt.

Alkoxy mit bis zu 12 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Eine bevorzugte Bedeutung von R₂ und R₃ ist Alkoxy mit 1 bis 10, insbesondere 1 bis 8 Kohlenstoffatomen. Eine bevorzugte Bedeutung von R₆ ist Methoxy.

Durch ein bis drei Reste R₆ substituiertes Phenoxy, das vorzugsweise 1 oder 2 C₁-C₄-Alkylgruppen enthält, bedeutet beispielsweise o-, m- oder p-Methylphenoxy, 2,3-Dimethylphenoxy, 2,4-Dimethylphenoxy, 2,5-Dimethylphenoxy, 2,6-Dimethylphenoxy, 3,4-Dimethylphenoxy, 3,5-Dimethylphenoxy, 2-Methyl-6-ethylphenoxy, 4-tert-Butylphenoxy, 2-Ethylphenoxy oder 2,6-Diethylphenoxy.

Alkanoyloxy mit bis zu 12 Kohlenstoffatomen bedeutet einen verzweigten oder unverzweigten Rest wie beispielsweise Formyloxy, Acetoxy, Propionyloxy, Butanoyloxy, Pentanoyloxy, Hexanoyloxy, Heptanoyloxy, Octanoyloxy, Nonanoyloxy, Decanoyloxy, Undecanoyloxy oder Dodecanoyloxy. Bevorzugt ist Alkanoyloxy mit 1 bis 10, insbesondere 1 bis 8, z.B. 2 bis 6 Kohlenstoffatomen. Besonders bevorzugt ist Acetoxy.

Durch ein bis drei Reste R₆ substituiertes Benzoyloxy, das insbesondere 1 oder 2 C₁-C₄-Alkylgruppen trägt, bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy.

Ein Alkalimetall bedeutet beispielsweise Lithium, Natrium, Kalium, Rubidium oder Cäsium. Bevorzugt sind Natrium und Kalium.

Ein Erdalkalimetall bedeutet beispielsweise Magnesium, Calcium, Strontium oder Barium. Bevorzugt sind Magnesium und Calcium.

Durch C₁-C₄-Alkyl substituierter C₅-C₁₂-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

Bevorzugt sind Zusammensetzungen enthaltend als Komponente b) mindestens eine Verbindung der Formel II, worin die Verbindung der Formel II eine Verbindung der Formel VII, VIIa oder VIII 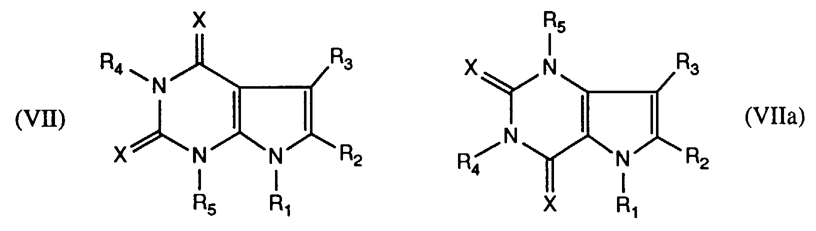 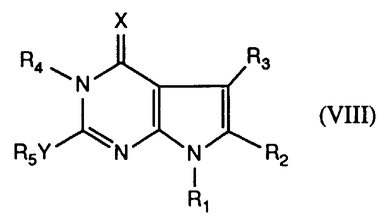 bedeutet, worin
X Sauerstoff oder Schwefel ist,
Y Sauerstoff, Schwefel oder 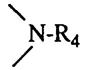 bedeutet,
R₁ Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, C₇-C₉-Phenylalkyl, ein Alkalimetall oder ein Erdalkalimetall darstellt,
R₂ und R₃ unabhängig voneinander Wasserstoff, C₁-C₁₀-Alkyl durch Sauerstoff unterbrochenes C₂-C₁₂-Alky; durch Hydroxy substituiertes C₁-C₁₀-Alkyl; C₃-C₆-Alkenyl, C₅-C₈-Cycloalkyl, Phenyl, unsubstituiertes oder am Phenylring durch ein bis drei Reste R₆ substituiertes C₇-C₉-Phenylalkyl; 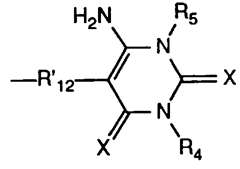 oder einen Rest der Formel IX, X, XI oder XII 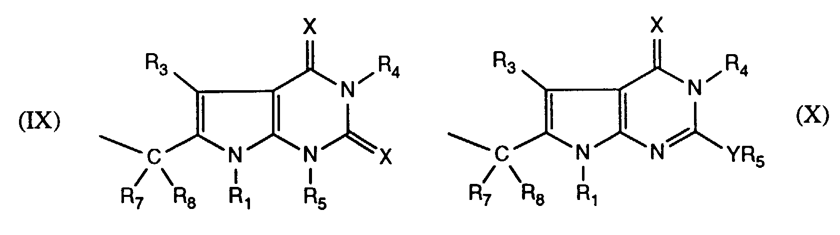 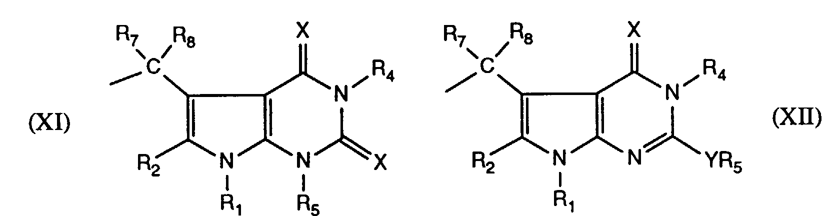 darstellt, worin X, Y, R₁, R₂ und R₃ die oben angegebene Bedeutung besitzen, oder ferner einer der Reste R₂ und R₃ Hydroxy, Formyl, C₁-C₁₀-Alkoxy, Phenoxy, C₁-C₁₀-Alkanoyloxy, Benzoyloxy, 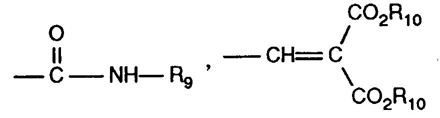 oder -CH=N-R₁₁ darstellt,
R₄ und R₅ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl oder C₇-C₉-Phenylalkyl bedeuten,
R₆ C₁-C₄-Alkyl, C₁-C₄-Alkoxy, Cyclohexyl, Hydroxy oder Chlor darstellt,
R₇ und R₈ unabhängig voneinander Wasserstoff, C₁-C₁₀-Alkyl, Phenyl oder C₇-C₉-Phenylalkyl darstellen,
R₉ Phenyl bedeutet,
R₁₀ C₁-C₁₀-Alkyl, durch Sauerstoff unterbrochenes C₂-C₁₂-Alkyl; C₃-C₆-Alkenyl, C₅-C₈-Cycloalkyl, Phenyl oder C₇-C₉-Phenylalkyl bedeutet,
R₁₁ C₁-C₁₀-Alkyl, C₅-C₈-Cycloalkyl, Phenyl oder C₇-C₉-Phenylalkyl darstellt,
R'₁₂ eine direkte Bindung oder 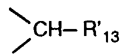 bedeutet, und
R'₁₃ Wasserstoff oder C₁-C₄-Alkyl darstellt.

Bevorzugt sind auch Zusammensetzungen, worin in Formel II X und Y Sauerstoff bedeuten.

Bevorzugt sind ebenfalls Zusammensetzungen, worin in Formel II R₁ Wasserstoff, C₁-C₄-Alkyl, Benzyl, 2-Hydroxyethyl, Natrium, Magnesium oder Calcium darstellt.

Ebenfalls von Interesse sind Zusammensetzungen, worin in Formel II R₂ Wasserstoff, C₁-C₄-Alkyl, Phenyl, 2,6-Di-tert-butyl-4-hydroxybenzyl, Hydroxy, 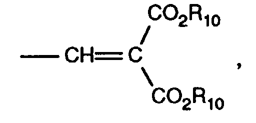 -CH=N-R₁₁ oder einen Rest der Formel IX 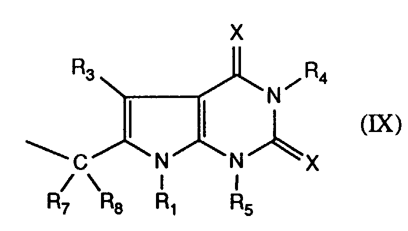 darstellt, worin X, R₁, R₄, R₅, R₇, R₈, R₁₀ und R₁₁ wie in Anspruch 2 definiert sind, und R₃ Wasserstoff, Hydroxy, C₁-C₄-Alkyl, Phenyl, 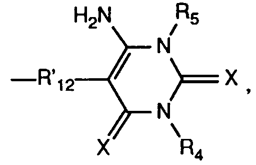 worin X, R₄, R₅ und R'₁₂ wie in Anspruch 2 definiert sind, oder 2,6-Di-tert-butyl-4-iydroxybenzyl bedeutet.

Von besonderem Interesse sind Zusammensetzungen, worin in Formel II R₄ und R₅ unabhängig voneinander Wasserstoff oder C₁-C₄-Alkyl bedeuten.

Von speziellem Interesse sind Zusammensetzungen enthaltend als Komponente b) mindestens eine Verbindung der Formel VII, VIIa oder VIII, worin
X und Y Sauerstoff sind,
R₁ Wasserstoff, C₁-C₄-Alkyl, durch Hydroxy substituiertes C₂-C₆-Alkyl, ein Alkalimetall oder Erdalkalimetall darstellt,
R₂ Wasserstoff, C₁-C₈-Alkyl, unsubstituiertes oder mit C₁-C₄-Alkyl substituiertes Phenyl; 2,6-Di-tert-butyl-4-hydroxybenzyl, Hydroxy, 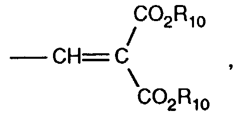 -CH=N-R₁₁ oder einen Rest der Formel IX 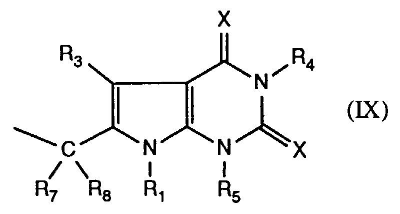 darstellt, worin X und R₁ die oben angegebene Bedeutung besitzen,
R₃ Wasserstoff, Hydroxy, C₁-C₈-Alkyl, unsubstituiertes oder mit C₁-C₄-Alkyl substituiertes Phenyl; 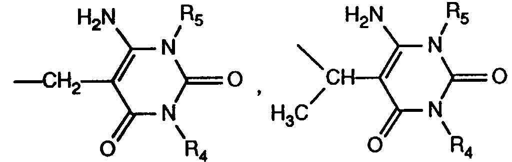 oder 2,6-Di-tert-butyl-4-hydroxybenzyl bedeutet,
R₄ und R₅ unabhängig voneinander Wasserstoff, C₁-C₄-Alkyl, C₃-C₆-Alkenyl oder Benzyl bedeuten,
R₇ und R₈ unabhängig voneinander Wasserstoff, C₁-C₉-Alkyl oder Phenyl darstellen, oder
R₇ und R₈ zusammen mit dem C-Atom, an das sie gebunden sind, einen C₅-C₈-Cycloalkylidenring bilden,
R₁₀ C₁-C₈-Alkyl bedeutet, und
R₁₁ C₇-C₉-Phenylalkyl darstellt.

Von ganz speziellem Interesse sind Zusammensetzungen enthaltend als Komponente b) mindestens eine Verbindung der Formel VII, VIIa oder VIII, worin
X und Y Sauerstoff sind,
R₁ Wasserstoff, 2-Hydroxyethyl, Natrium, Magnesium oder Calcium darstellt,
R₂ Wasserstoff, C₁-C₄-Alkyl, Phenyl, 2,6-Di-tert-butyl-4-hydroxybenzyl, Hydroxy, 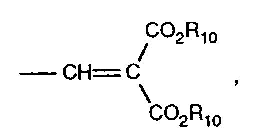 -CH=N-R₁₁ oder einen Rest der Formel IX 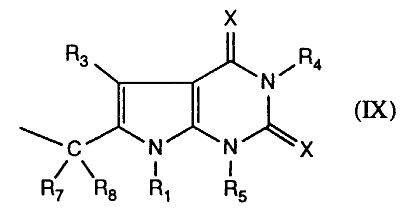 darstellt, worin X und R₁ die oben angegebene Bedeutung besitzen,
R₃ Wasserstoff, Hydroxy, C₁-C₄-Alkyl, Phenyl, 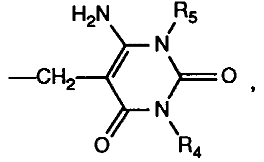 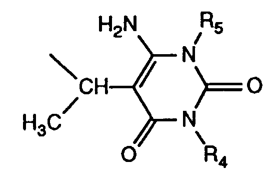 oder 2,6-Di-tert-butyl-4-hydroxybenzyl bedeutet,
R₄ und R₅ unabhängig voneinander Wasserstoff oder C₁-C₄-Alkyl bedeuten,
R₇ und R₈ unabhängig voneinander Wasserstoff, C₁-C₉-Alkyl oder Phenyl darstellen,
R₁₀ C₁-C₄-Alkyl bedeutet, und
R₁₁ Benzyl darstellt.

Speziell bevorzugt sind Zusammensetzungen enthaltend als Komponente b) mindestens eine Verbindung der Formel VII, worin die Verbindung der Formel VII 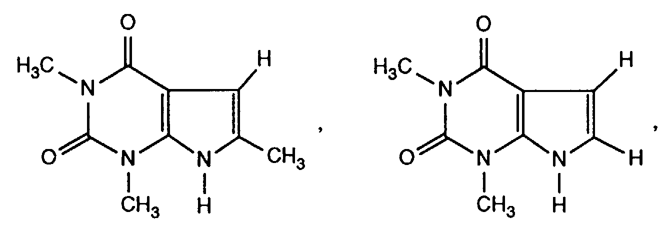 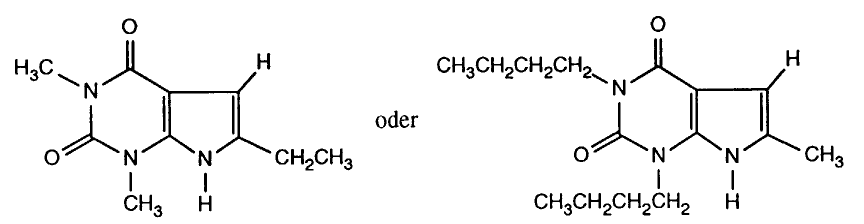 bedeutet.

Als chlorhaltige Polymerisate können beispielsweise aufgezählt werden: Polymere des Vinylchlorides, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methycrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenechlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alpha-substituierten Acrylsäure; Polymere von chlorierten Styrolen, zum Beispiel Dichlorstyrol; chlorierte Gummis; chlorierte Polymere des Ethylens; Polymere und nachchlorierte Polymere von Chlorbutadiens und deren Copolymere mit Vinylchlorid, Gummi-Hydrochlorid und chloriertes Gummi-Hydrochlorid; sowie Mischungen der genannten Polymere unter sich oder mit anderen polymerisierbaren Verbindungen.

Ferner sind umfasst die Pfropfpolymerisate von PVC mit EVA, ABS und MBS. Bevorzugte Substrate sind auch Mischungen der vorstehend genannten Homo-und Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen.

Als chlorhaltiges Polymerisat ist Polyvinylchlorid besonders bevorzugt, insbesondere Suspensionspolymerisat und Massepolymerisat.

Weiterhin sind im Rahmen dieser Erfindung unter der Komponente (a) auch insbesondere Recyclate chlorhaltiger Polymerisate zu verstehen, wobei es sich hierbei um die oben näher beschriebenen Polymere handelt, welche durch Verarbeitung, Gebrauch oder Lagerung eine Schädigung erfahren haben. Besonders bevorzugt ist PVC-Recyclat. In den Recyclaten können auch kleine Mengen an Fremdstoffen enthalten sein, wie z.B. Papier, Pigmente, Klebstoffe, die oft schwierig zu entfernen sind. Diese Fremdstoffe können auch aus dem Kontakt mit diversen Stoffen während des Gebrauchs oder der Aufarbeitung stammen, wie z.B. Treibstoffreste, Lackanteile, Metallspuren, Initiatorreste oder auch Wasserspuren.

Es ist möglich, die erfindungsgemässen Verbindungen, die mindestens einen Rest der Formel I enthalten, allein oder vorzugsweise zusammen mit bekannten Thermostabilisatoren einzusetzen, wie z.B. Organozinnverbindungen, organischen Antimonverbindungen, Me(II)-Phenolaten, insbesondere C₇-C₂₀-Alkylphenolaten, beispielsweise Nonylphenolat, oder Me(II)-Carboxylaten. Me(II) bedeutet z.B. Ba, Ca, Mg oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate, Dihydroxystearate oder 2-Ethylhexanoate. Besonders bevorzugt sind Stearate, Oleate und p-tert-Butylbenzoate. Beispiele für Organozinnverbindungen und organische Antimonverbindungen sind die in US-A-4 743 640 Spalte 3, Zeile 48 bis Spalte 5, Zeile 38 genannten Verbindungen.

Zusätzlich können die mit den Verbindungen, die mindestens einen Rest der Formel I enthalten, stabilisierten chlorhaltigen Polymerisate in üblichen Mengen weitere herkömmliche PVC-Stabilisatoren bzw. PVC-Costabilisatoren enthalten, wie beispielsweise Phosphite oder Phosphonite, Epoxyverbindungen wie Glycidylverbindungen, Perchloratsalze, Hydrotalcite und/oder Zeolithe.

Bei den Phosphiten handelt es sich bevorzugt um solche der Formeln 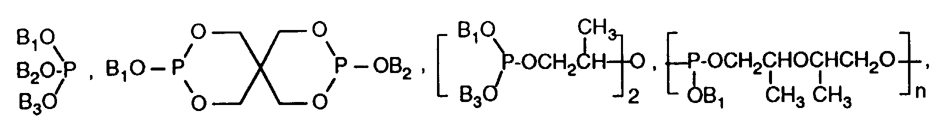 worin B₁, B₂ und B₃ unabhängig voneinander C₄-C₁₈-Alkyl, C₆-C₁₈-Alkenyl, C₅-C₇-Cycloalkyl, Phenyl oder durch ein bis drei C₁-C₁₂-Alkylgruppen substituiertes Phenyl bedeuten.

Beispiele sind Trioctyl-, Tridecyl-, Tridodecyl-, Tritetradecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiaryl-phosphite, wie z.B. Phenyldidecyl-, (2,4-Di-tert-butylphenyl)didodecyl-, (2,6-Di-tert-butylphenyl)didodecyl-phosphit und die Dialkyl- und Diaryl-pentaerythrit-diphosphite, wie z.B. Distearylpentaerythrit-diphosphit. Ebenfalls bevorzugt sind die Tetraphenyl- und Tetraalkyl-[dipropylenglykol-1,2]-diphosphite und die Poly-[dipropylenglykol-1,2-phenylphosphite] sowie die Poly-[dipropylenglykol-1,2-alkylphosphite].

Besonders bevorzugte organische Phosphite sind Distearyl-pentaerythritdiphosphit, Tris(nonylphenyl)phosphit, Phenyldidecylphosphit, Tetraphenyl-[dipropylenglykol-1,2]-diphosphit und Poly-[dipropylenglykol-1,2-phenylphosphit].

Speziell bevorzugt sind auch Phosphite oder Phosphonite der folgenden Formeln.

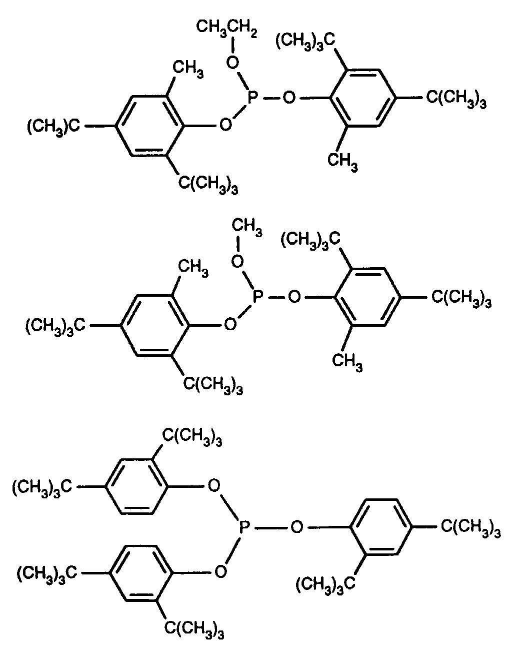

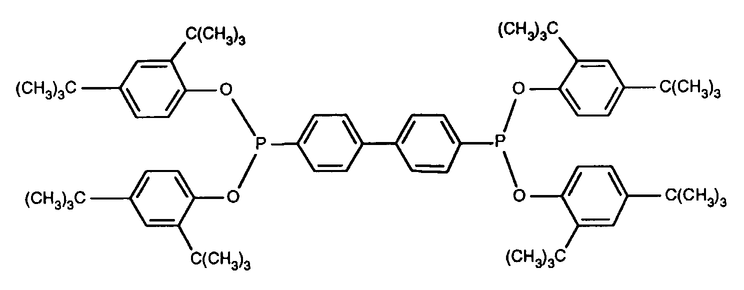

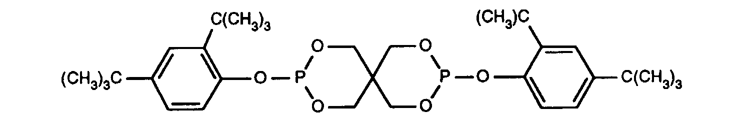

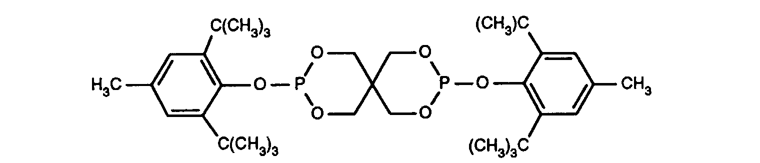

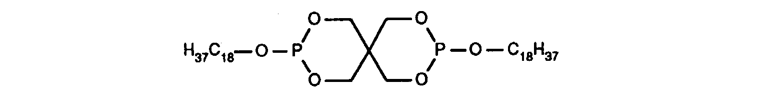

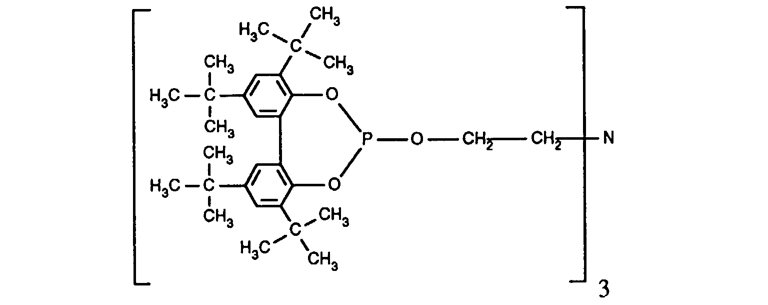

Die Phosphite bzw. Phosphonite werden in einer Menge von beispielsweise 0,3 bis 5, vorzugsweise 0,5 bis 1 Gew.-Teilen bezogen auf 100 Gew.-Teile PVC eingesetzt.

Bei der Epoxyverbindung handelt es sich bevorzugt um epoxidierte Oele und epoxidierte Fettsäureester, z.B. epoxidiertes Polybutadien, epoxidiertes Sojabohnenöl, epoxidiertes Leinsamenöl, epoxidiertes Rapsöl, epoxidierter Talg, Methylbutyl- oder 2-Ethylhexylepoxystearat, Tris(epoxypropyl)isocyanurat, epoxidiertes Ricinusöl, epoxidiertes Sonnenblumenöl, 3-(2-Xenoxy)-1,2-epoxypropan, Bisphenol-A-polyglycidylether, Vinylcyclohexendiepoxyd, Dicyclopentadiendiepoxyd und 3,4-Epoxycyclohexylmethyl-3,4-epoxycyclohexancarboxylat.

Die im Rahmen der Erfindung verwendbaren Glycidylverbindungen können eine aliphatische, aromatische, cycloaliphatische, araliphatische oder heterocyclische Struktur haben; sie enthalten Epoxidgruppen als Seitengruppen. Die Epoxidgruppen sind vorzugsweise als Glycidylgruppen über Ether- oder Esterbindungen mit dem Restmolekül verbunden, oder es handelt sich um N-Glycidylderivate von heterocyclischen Aminen, Amiden oder Imiden. Epoxidverbindungen dieser Typen sind allgemein bekannt und im Handel erhältlich.

Die endständigen Epoxidverbindungen enthalten wenigstens einen Epoxyrest, insbesondere solche der Formel I' 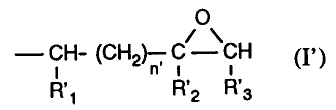 wobei dieser direkt an Kohlenstoff, Sauerstoff-, Stickstoff- oder Schwefelatome gebunden ist, worin R'₁ und R'₃ beide Wasserstoff sind, R'₂ Wasserstoff oder Methyl und n' = 0 ist, oder worin R'₁ und R'₃ zusammen -CH₂-CH₂- oder -CH₂-CH₂-CH₂- bedeuten, R'₂ dann Wasserstoff und n' = 0 oder 1 ist.

Beispielhaft für endständige Epoxidverbindungen sind zu erwähnen:

I) Glycidyl- und β-Methylglycidylester erhältlich durch Umsetzung einer Verbindung mit mindestens einer Carboxylgruppe im Molekül und Epichlorhydrin bzw. Glycerindichlorhydrin bzw. β-Methyl-epichlorhydrin. Die Umsetzung erfolgt zweckmässig in der Gegenwart von Basen.

Als Verbindungen mit mindestens einer Carboxylgruppe im Molekül können aliphatische Carbonsäuren verwendet werden. Beispiele für diese Carbonsäuren sind Glutarsäure, Adipinsäure, Pimelinsäure, Korksäure, Azelainsäure, Sebazinsäure oder dimerisierte bzw. trimerisierte Linolsäure, Acryl- und Methacrylsäure, Capron-, Capryl-, Laurin-, Myristin-, Palmitin-, Stearin- und Pelargonsäure, sowie die bei den organischen Zinkverbindungen erwähnten Säuren.

Es können aber auch cycloaliphatische Carbonsäuren eingesetzt werden, wie beispielsweise Cyclohexancarbonsäure, Tetrahydrophthalsäure, 4-Methyltetrahydrophthalsäure, Hexahydrophthalsäure oder 4-Methylhexahydrophthalsäure.

Weiterhin können aromatische Carbonsäuren Verwendung finden, wie beispielsweise Benzoesäure, Phthalsäure, Isophthalsäure, Trimellithsäure oder Pyromellithsäure.

Ebenfalls können auch carboxylterminierte Addukte, z.B. von Trimellithsäure und Polyolen, wie beispielsweise Glycerin oder 2,2-Bis-(4-hydroxycyclohexyl)-propan verwendet werden.

Weitere im Rahmen dieser Erfindung verwendbare Epoxidverbindungen finden sich in der EP 0 506 617.

II) Glycidyl- oder (β-Methylglycidyl)-ether erhältlich durch Umsetzung einer Verbindung mit mindestens einer freien alkoholischen Hydroxygruppe und/oder phenolischen Hydroxygruppe und einem geeignet substituierten Epichlorhydrin unter alkalischen Bedingungen, oder in Anwesenheit eines sauren Katalysators und anschliessender Alkalibehandlung.

Ether dieses Typs leiten sich beispielsweise ab von acyclischen Alkoholen, wie Ethylenglykol, Diethylenglykol und höheren Poly-(oxyethylen)-glykolen, Propan-1,2-diol, oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Bistrimethylolpropan, Pentaerythrit, Sorbit, sowie von Polyepichlorhydrinen, Butanol, Amylalkohol, Pentanol, sowie von monofunktionellen Alkoholen wie Isooctanol, 2-Ethylhexanol, Isodecanol sowie C₇-C₉-Alkanol- und C₉-C₁₁-Alkanolgemischen.

Sie leiten sich aber auch beispielsweise ab von cycloaliphatischen Alkoholen wie 1,3-oder 1,4-Dihydroxycyclohexan, Bis-(4-hydroxycyclohexyl)-methan, 2,2-Bis-(4-hydroxycyclohexyl)-propan oder l,l-Bis-(hydroxymethyl)-cyclohex-3-en oder sie besitzen aromatische Kerne wie N,N-Bis-(2-hydroxyethyl)-anilin oder p,p'-Bis-(2-hydroxyethylamino)-diphenylmethan.

Die endständigen Epoxidverbindungen können sich auch von einkernigen Phenolen ableiten, wie beispielsweise von Phenol, Resorcin oder Hydrochinon; oder sie basieren auf mehrkernigen Phenolen wie beispielsweise auf Bis-(4-hydroxyphenyl)-methan, 2,2-Bis(4-hydroxyphenyl)-propan, 2,2-Bis-(3,5-dibrom-4-hydroxyphenyl)-propan, 4,4'-Dihydroxydiphenylsulfon oder auf unter sauren Bedingungen erhaltene Kondensationsprodukte von Phenolen mit Formaldehyd wie Phenol-Novolake.

Weitere mögliche endständige Epoxide sind beispielsweise: Glycidyl-1-naphthylether, Glycidyl-2-phenylphenylether, 2-Biphenylglycidylether, N-(2,3-epoxypropyl)-phthalimid und 2,3-Epoxypropyl-4-methoxyphenylether.

III) (N-Glycidyl)-Verbindungen erhältlich durch Dehydrochlorierung der Reaktionsprodukte von Epichlorhydrin mit Aminen, die mindestens ein Aminowasserstoffatom enthalten. Bei diesen Aminen handelt es sich zum Beispiel um Anilin, N-Methylanilin, Toluidin, n-Butylamin, Bis-(4-aminophenyl)-methan, m-Xylylendiamin oder Bis-(4-methylaminophenyl)-methan, aber auch N,N,O-Triglycidyl-m-aminophenol oder N,N,O-Triglycidyl-p-aminophenol.

Zu den (N-Glycidyl)-Verbindungen zählen aber auch N,N'-Di-, N,N',N"-Tri- und N,N',N'',N''' -Tetraglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin oder Glykoluril und Triglycidylisocyanurat.

IV) S-Glycidyl-Verbindungen, wie beispielsweise Di-S-glycidylderivate, die sich von Dithiolen, wie beispielsweise Ethan-1,2-dithiol oder Bis-(4-mercaptomethylphenyl)-ether ableiten.

V) endständige Epoxidverbindungen mit einem Rest der Formel I', worin R'₁ und R'₃ zusammen -CH₂-CH₂- bedeuten und n' 0 ist, sind Bis-(2,3-epoxycyclopentyl)-ether, 2,3-Epoxycyclopentylglycidylether oder 1,2-Bis-(2,3-epoxycyclopentyloxy)-ethan. Ein endständiges Epoxidharz mit einem Rest der Formel I', worin R'₁ und R'₃ zusammen -CH₂-CH₂- sind und n' 1 bedeutet, ist beispielsweise 3,4-Epoxy-6-methyl-cyclohexancarbonsäure-(3',4'-epoxy-6'-methyl-cyclohexyl)-methylester.

Geeignete endständige Epoxide sind beispielsweise:
a) flüssige Bisphenol-A-diglycidylether wie Araldit®GY 240, Araldit®GY 250, Araldit®GY 260, Araldit®GY 266, Aradit®GY 2600, Araldit®MY 790;
b) feste Bisphenol-A-diglycidylether wie Araldit®GT 6071, Araldit®GT 7071, Araldit®GT 7072, Aradit®GT 6063, Araldit®GT 7203, Araldit®GT 6064, Araldit®GT 7304, Araldit®GT 7004, Aradit®GT 6084, Araldit®GT 1999, Araldit®GT 7077, Araldit®GT 6097, Araldit®GT 7097, Araldit®GT 7008, Araldit®GT 6099, Araldit®GT 6608, Araldit®GT 6609, Aradit®GT 6610;
c) flüssige Bisphenol-F-diglycidylether wie Araldit®GY 281, Aradit®PY 302, Araldit®PY 306;
d) feste Polyglycidylether von Tetraphenylethan wie CG Epoxy Resin®0163;
e) feste und flüssige Polyglycidylether von Phenolformaldehyd Novolak wie EPN 1138, EPN 1139, GY 1180, PY 307;
f) feste und flüssige Polyglycidylether von o-Cresolformaldehyd Novolak wie ECN 1235, ECN 1273, ECN 1280, ECN 1299;
g) flüssige Glycidylether von Alkoholen wie Shell® Glycidylether 162, Araldit®DY 0390, Araldit®DY 0391;
h) flüssige Glycidylether von Carbonsäuren wie Shell®Cardura E Terephthalsäureester, Trimellithsäureester, Aradit®PY 284;
i) feste heterocyclische Epoxidharze (Triglycidylisocyanurat) wie Araldit® PT 810;
j) flüssige cycloaliphatische Epoxidharze wie Aradit®CY 179;
k) flüssige N,N,O-Triglycidylether von p-Aminophenol wie Aradit®MY 0510;
l) Tetraglycidyl-4-4'-methylenbenzamin oder N,N,N',N'-Tetraglycidyldiaminophenylmethan wie Araldit®MY 720, Araldit®MY 721.

Vorzugsweise finden endständige Epoxidverbindungen mit zwei funktionellen Gruppen Verwendung. Es können aber auch prinzipiell endständige Epoxidverbindungen mit einer, drei oder mehr funktionellen Gruppen eingesetzt werden.

Vorwiegend werden endständige Epoxidverbindungen, vor allem Diglycidylverbindungen, mit aromatischen Strukturen eingesetzt.

Gegebenenfalls kann auch ein Gemisch von endständigen Epoxidverbindungen unterschiedlicher Struktur eingesetzt werden.

Besonders bevorzugt sind als endständige Epoxidverbindungen Diglycidylether auf der Basis von Bisphenolen, wie beispielsweise von 2,2-Bis-(4-hydroxyphenyl)-propan (Bisphenol A), Bis-(4-hydroxyphenyl)-methan oder Mischungen von Bis-(ortho/para-hydroxyphenyl)-methan (Bisphenol F).

Die endständigen Epoxidverbindungen können in einer Menge von vorzugsweise mindestens 0,1 Teil, beispielsweise 0,1 bis 50, zweckmässig 1 bis 30 und insbesondere 1 bis 20 Gew.-Teilen, bezogen auf 100 Gew.-Teile PVC, eingesetzt werden.

Perchloratsalze sind beispielsweise Verbindungen der Formel M(ClO₄)ₘ, wobei M für NH₄⁺, Na⁺, K⁺, Mg⁺⁺, Ca⁺⁺, Ba⁺⁺ oder Al³⁺ steht. Der Index m ist entsprechend der Wertigkeit von M 1, 2 oder 3.

Die Perchloratsalze können dabei in verschiedenen gängigen Darreichungsformen eingesetzt werden; z.B. als Salz oder wässrige Lösung aufgezogen auf ein Trägermaterial wie PVC, Ca-Silikat, Zeolith oder Hydrotalcit, oder eingebunden durch chemische Reaktion in ein Hydrotalcit.

Die Perchloratsalze können in einer Menge von beispielsweise 0,001 bis 5, zweckmässig 0,01 bis 3, besonders bevorzugt 0,01 bis 2 Gew.-Teilen, bezogen auf 100 Gew.-Teile PVC, angewendet werden.

Ein bevorzugter Gegenstand der Erfindung sind daher auch Zusammensetzungen enthaltend ausser der Komponente a) und der Komponente b) (Verbindung der Formel I, die mindestens einen Rest der Formel I enthält) zusätzlich mindestens ein Me(II)-Carboxylat und/oder Me(II)-Phenolat, wobei Me(II) Ba, Ca, Mg oder Zn bedeutet.

Gemäss einer weiteren Bevorzugung enthalten die erfindungsgemässen Zusammensetzungen ausser der Komponente a) und der Komponente b) mindestens ein Me(II)-Carboxylat, wobei Me(II) Ba, Ca, Mg oder Zn bedeutet. Gemische aus Ba/Zn- oder Ca/Zn-Carboxylaten werden dabei als Costabilisatoren besonders bevorzugt.

Ebenfalls bevorzugt sind Zusammensetzungen enthaltend ausser den Komponenten a) und b) eine Epoxyverbindung bzw. Glycidylverbindung und/oder ein Phosphit und gegebenenfalls ein Me(II)-Carboxylat und/oder Me(II)-Phenolat.

Weiterhin bevorzugt sind Zusammensetzungen enthaltend ausser den Komponenten a) und b) eine Epoxyverbindung bzw. Glycidylverbindung und ein Perchloratsalz und/oder ein Phosphit oder Phosphonit.

Die bekannten Thermostabilisatoren (z.B. Carboxylate) können in dem zu stabilisierenden Material in einer dem Fachmann bekannten Konzentration vorliegen, wie zum Beispiel in Mengen von 0,05 bis 5 Gew.%.

Zweckmässig sind Zusammensetzungen, wie vorstehend beschrieben, worin die Komponente b) in einer Menge von 0,05 bis 5 %, insbesondere 0,1 bis 5 %, beispielsweise 0,1 bis 2 %, bezogen auf das Gewicht der Komponente a) vorliegt.

Je nach dem Verwendungszweck der Polymerisate können vor oder bei der Einarbeitung der Stabilisatoren auch weitere Zusätze eingearbeitet werden, wie zum Beispiel Antioxidantien, Gleitmittel (bevorzugt Montanwachse oder Glycerinester), Fettsäureester, Paraffine, Weichmacher, Füllstoffe, Russ, Asbest, Kaolin, Talk, Glasfasern, Modifikatoren (wie etwa Schlagzäh-Zusätze), optische Aufheller, Pigmente, Lichtschutzmittel, UV-Absorber, Flammschutzmittel, Verarbeitungshilfen oder Antistatika. Besonders bevorzugt sind Lichtschutzmittel mit gehinderter Aminstruktur wie z.B. in EP-A-421 933 definiert.

Weitere mögliche Zusätze sind ferner β-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Polyole, z.B. die in DE-A-3 019 910 beschriebenen Verbindungen, β-Diketone, z.B. die in DE-A-2 600 516 angegebenen Verbindungen, oder auch Gemische aus β-Diketonen und Hydrotalciten, wie z.B. in EP-A-63 180 beschrieben.

Bevorzugt sind Zusammensetzungen enthaltend zusätzlich zu den Komponenten a) und b) Gleitmittel, Pigmente, Modifikatoren, Verarbeitungshilfen, Füllstoffe, Antioxidantien und/oder Lichtschutzmittel.

Enthalten die erfindungsgemässen Zusammensetzungen Weichmacher, insbesondere organische Weichmacher, kommen beispielsweise solche aus den folgenden Gruppen in Betracht.
A) Phthalate (Phthalsäureester)
   Beispiele für solche Weichmacher sind Dimethyl-, Diethyl-, Dibutyl-, Dihexyl-, Di-2- ethylhexyl-, Di-n-octyl-, Di-iso-octyl-, Di-iso-nonyl-, Di-iso-decyl-, Di-iso-tridecyl-, Dicyclohexyl-, Di-methylcyclohexyl-, Dimethylglycol-, Dibutylglycol-, Benzylbutyl- und Diphenyl-phthalat sowie Mischungen von Phthalaten wie C₇-C₉- und C₉-C₁₁-Alkylphthalate aus überwiegend linearen Alkoholen, C₆-C₁₀-n-Alkylphthalate und C₈-C₁₀-n-Alkylphthalate. Bevorzugt sind davon Dibutyl-, Dihexyl-, Di-2-ethylhexyl-, Di-n-octyl-, Di-iso-octyl-, Di-iso-nonyl-, Di-iso-decyl-, Di-iso-tridecyl- und Benzylbutyl-phthalat sowie die genannten Mischungen von Alkylphthalaten. Besonders bevorzugt ist Di-2-ethylhexylphthalat (DOP).
B) Ester aliphatischer Dicarbonsäuren, insbesondere Ester von Adipin-, Azelain- und Sebazinsäure.
   Beispiele für solche Weichmacher sind Di-2-ethylhexyladipat, Di-isooctyladipat (Gemisch), Di-iso-nonyladipat (Gemisch), Di-iso-decyladipat (Gemisch), Benzylbutyladipat, Benzyloctyladipat, Di-2-ethylhexylazelat, Di-2-ethylhexylsebacat und Di-isodecylsebacat (Gemisch). Bevorzugt sind Di-2-ethylhexyladipat und Di-iso-octyladipat.
C) Trimellithsäureester,
   beispielsweise Tri-2-ethylhexyltrimellithat, Tri-iso-decyltrimellithat (Gemisch), Tri-isotridecyltrimellithat, Tri-iso-octyltrimellithat (Gemisch) sowie Tri-C₆-C₈-alkyl, Tri-C₆-C₁₀-alkyl-, Tri-C₇-C₉-alkyl- und Tri-C₉-C₁₁-alkyl-trimellithate. Die letztgenannten Trimellithate entstehen durch Veresterung der Trimellithsäure mit den entsprechenden Alkanolgemischen. Bevorzugte Trimellithate sind Tri-2-ethylhexyltrimellithat und die genannten Trimellithate aus Alkanolgemischen.
D) Polymerweichmacher
   Eine Definition dieser Weichmacher und Beispiele für solche sind im "Plastics Additives Handbook", Herausgeber H. Gächter und H. Müller, Hanser Publishers, 1985, Seite 284, Kapitel 5.7.10 sowie in "PVC Technology ", Herausgeber W.V. Titow, 4th. Ed., Elsevier Publ., 1984, Seiten 165-170 angegeben. Die gebräuchlichsten Ausgangsmaterialien für die Herstellung der Polyesterweichmacher sind: Dicarbonsäuren wie Adipin-, Phthal-, Azelain- und Sebacinsäure; Diole wie 1,2-Propandiol, 1,3-Butandiol, 1,4-Butandiol, 1,6-Hexandiol, Neopentylglycol und Diethylenglykol; Monocarbonsäuren wie Essig-, Capron-, Capryl-, Laurin-, Myristin-, Palmitin-, Stearin-, Pelargon-und Benzoesäure; monofunktionelle Alkohole wie Isooctanol, 2-Ethylhexanol, Isodecanol sowie C₇-C₉-Alkanol und C₉-C₁₁-Alkanolgemische. Besonders vorteilhaft sind Polyesterweichmacher aus den genannten Dicarbonsäuren und monofunktionellen Alkoholen.
E) Phosphorsäureester
   Eine Definition dieser Ester ist im vorstehend genannten "Plastics Additives Handbook" auf Seite 271, Kapitel 5.7.2 zu finden. Beispiele für solche Phosphorsäureester sind Tributylphosphat, Tri-2-ethylbutylphosphat, Tri-2-ethylhexylphosphat, Trichlorethylphosphat, 2-Ethylhexyl-di-phenylphosphat, Kresyldiphenylphosphat, Triphenylphosphat, Trikresylphosphat und Trixylenylphosphat. Bevorzugt ist Tri-2-ethylhexyl-phosphat.
F) Chlorierte Kohlenwasserstoffe (Paraffine)
G) Kohlenwasserstoffe
H) Monoester, z.B. Butyloleat, Phenoxyethyloleat, Tetrahydrofurfuryloleat und Alkylsulfonsäureester.
I) Glycolester, z.B. Diglykolbenzoate.

Definitionen und Beispiele für Weichmacher der Gruppen F) bis I) sind den folgenden Handbüchern zu entnehmen:

"Plastics Additives Handbook", Herausgeber H. Gächter und H. Müller, Hanser Publishers, 1985, Seite 284, Kapitel 5.7.11 (Gruppe F)), und Kapitel 5.7.13 (Gruppe G)).

"PVC Technology", Herausgeber W.V. Titow, 4th. Ed., Elsevier Publishers, 1984, Seiten 171-173, Kapitel 6.10.2 (Gruppe F)), Seite 174, Kapitel 6.10.5 (Gruppe G)), Seite 173, Kapitel 6.10.3 (Gruppe H)) und Seiten 173-174, Kapitel 6.10.4 (Gruppe I)).

Besonders bevorzugt sind Weichmacher aus den Gruppen A) bis E), insbesondere A) bis C), vor allem die in diesen Gruppen als bevorzugt herausgestellten Weichmacher. Besonders günstig ist Di-2-ethylhexylphthalat (DOP).

Die Weichmacher können in einer Menge von beispielsweise 15 bis 70, zweckmässig 15 bis 60 und insbesondere 20 bis 50 Gew.-Teilen, bezogen auf 100 Gew.-Teile Polymerzusammensetzung, angewendet werden.

Die erfindungsgemässen Zusammensetzungen können auch weitere, für chlorhaltige Polymerisate übliche Stabilisatoren enthalten.

Ferner können die erfindungsgemässen Zusammensetzungen übliche Antioxidantien, Lichtstabilisatoren und UV-Absorber enthalten. Beispiele dafür sind:

### 1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.12. Ester der β-(3,5-Di-tert-butyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicydo-[2.2.2]-octan.

1.13. Ester der β-(5-tert-Butvl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

### 2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylenbis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; 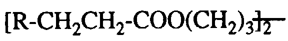 mit R = 3'-tert-Butyl-4'-hydroxy-5'-2H-benzotriazol-2-yl-phenyl.

2.2. 2-Hedroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.5. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

Die erfindungsgemässen Zusammensetzungen können auf an sich bekannte Weise hergestellt werden. In der Regel wird das Stabilisatorsystem in das Polymer eingearbeitet, wozu sich an sich bekannte Vorrichtungen, wie Kalander, Mischer, Kneter und dergleichen, anbieten.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Die nach vorliegender Erfindung stabilisierten Zusammensetzungen können auf bekannte Weisen in die gewünschte Form gebracht werden. Solche Verfahren sind beispielsweise Mahlen, Kalandrieren, Extrudieren oder Spritzgiessen, ferner Extrusions-Blasen oder eine Verarbeitung nach dem Plastisol-Verfahren. Die Zusammensetzungen können auch zu Schaumstoffen verarbeitet werden.

Bevorzugte stabilisierte chlorhaltige Polymerzusammensetzungen sind nicht weichgemachte, resp. weichmacherfreie oder im wesentlichen weichmacherfreie Zusammensetzungen.

Die erfindungsgemässen Zusammensetzungen eignen sich insbesondere, in Form von Hart-Rezepturen, für Hohlkörper (Flaschen), Verpackungsfolien (Tiefziehfolien), Blasfolien, Crash pad-Folien (Automobile), Rohre, Schaumstoffe, Schwerprofile (Fensterrahmen), Lichtwandprofile, Bauprofile, Sidings, Fittings, Bürofolien und Apparatur-Gehäuse (Computer, Haushaltgeräte).

Andere Zusammensetzungen, in Form von Weich-Rezepturen sind für Drahtummantelungen, Kabelisolierungen, Dekorationsfolien, Dachfolien, Schaumstoffe, Agrarfolien, Schläuche, Dichtungsprofile, Bürofolien, Folien für Traglufthallen und Folien für Kraftfahrzeuginnenräume geeignet.

Beispiele für die Anwendung der erfindungsgemässen Zusammensetzungen als Plastisole sind Kunstleder, Fussböden, Textilbeschichtungen, Tapeten, Coil-Coatings und Unterbodenschutz für Kraftfahrzeuge.

Beispiele für Sinter-PVC-Anwendungen der erfindungsgemässen Zusammensetzungen sind Slush, Slush Mould und Coil-Coatings.

Bevorzugt wird die Verwendung der oben beschriebenen Polymerzusammensetzungen zur Herstellung von Hartprofilen für Aussenanwendungen und von Hartfolien.

Ein weiterer Gegenstand der Erfindung ist die Verwendung von mindestens einer Verbindung, die mindestens einen Rest der Formel I enthält, als Stabilisator für chlorhaltige Polymerisate gegen oxidativen, thermischen oder lichtinduzierten Abbau.

Die vorliegende Erfindung betrifft auch ein Verfahren zum Stabilisieren eines chlorhaltigen Polymerisats gegen oxidativen, thermischen oder lichtinduzierten Abbau, dadurch gekennzeichnet, dass man diesem mindestens eine Verbindung, die mindestens einen Rest der Formel I enthält, einverleibt oder auf dieses aufbringt.

Die bevorzugten Verbindungen, die mindestens einen Rest der Formel I enthalten, für die Verwendung als Stabilisatoren und das Verfahren zum Stabilisieren sind die gleichen, wie sie für die Zusammensetzung mit einem chlorhaltigen Polymerisat beschrieben werden.

Ebenfalls Gegenstand der Erfindung sind neue Verbindungen der Formel II 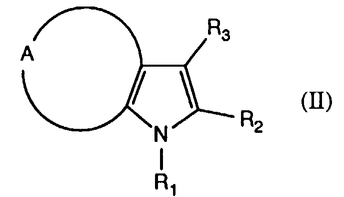 worin
A eine Gruppe der Formel III oder IV 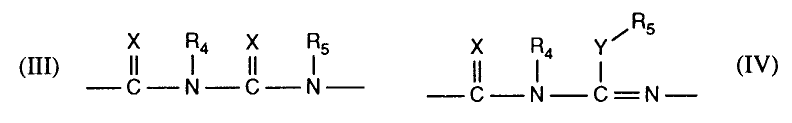 bedeutet,
X Sauerstoff oder Schwefel ist,
Y Sauerstoff, Schwefel oder 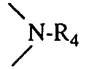 bedeutet,
R₁ Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; ein Alkalimetall oder ein Erdalkalimetall darstellt,
R₂ und R₃ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl;
unsubstituiertes oder am Phenylring durch ein bis drei Reste R₆ substituiertes C₇-C₉-Phenylalkyl; 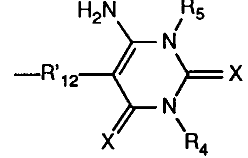 oder einen Rest der Formel V oder VI 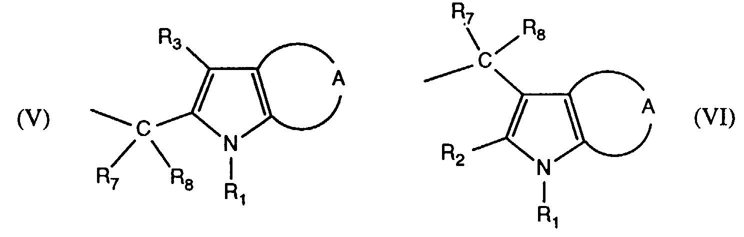
darstellen, worin A und R₁ die oben angegebene Bedeutung besitzen, oder ferner einer der Reste R₂ und R₃ Hydroxy, Formyl, C₁-C₁₂-Alkoxy, unsubstituiertes oder durch ein bis drei Reste R₆ substituiertes Phenoxy; C₁-C₁₂-Alkanoyloxy,
unsubstituiertes oder durch ein bis drei Reste R₆ substituiertes Benzoyloxy; 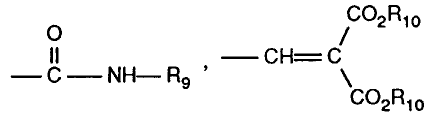 oder -CH=N-R₁₁ darstellt,
R₄ und R₅ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeuten,
R₆ C₁-C₄-Alkyl, C₁-C₄-Alkoxy, C₅-C₈-Cycloalkyl, Hydroxy oder Chlor darstellt,
R₇ und R₈ unabhängig voneinander Wasserstoff, CF₃, C₁-C₁₂-Alkyl, Phenyl oder C₇-C₉-Phenylalkyl darstellen, oder R₇ und R₈ zusammen mit dem C-Atom, an das sie gebunden sind, einen unsubstituierten oder durch C₁-C₄-Alkyl substituierten C₅-C₁₂-Cycloalkylidenring bilden,
R₉ unsubstituiertes oder durch ein bis drei Reste R₆ substituiertes Phenyl bedeutet;
R₁₀ Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeutet,
R₁₁ C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phennylalkyl darstellt,
R'₁₂ eine direkte Bindung oder 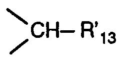 bedeutet, und
R'₁₃ Wasserstoff oder C₁-C₈-Alkyl darstellt; mit der Bedingung, dass die Verbindungen der Formel VII 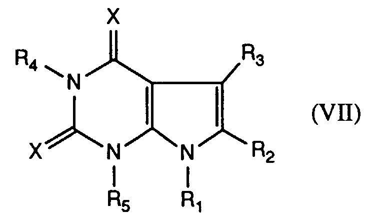 worin
X Sauerstoff bedeutet,
R₁ Wasserstoff, Methyl oder Phenyl darstellt,
R₂ Wasserstoff, Hydroxy, Formyl, C₁-C₃-Alkyl, Phenyl, 4-Chlorphenyl, 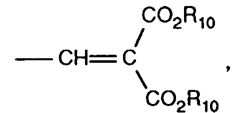 -CH=N-R₁₁ oder -CH₂CH(CO₂CH₂CH₃)₂ bedeutet,
R₃ Wasserstoff, Hydroxy, Formyl, C₁-C₅-Alkyl, Phenyl oder -CH₂CO₂R₁₀ darstellt,
R₄ und R₅ unabhängig voneinander Wasserstoff, C₁-C₄-Alkyl, Allyl, Phenyl, 4-Methylphenyl oder Benzyl bedeuten,
R₁₀ C₁-C₄-Alkyl darstellt,
R₁₁ Methyl, Allyl, Benzyl oder Phenyl bedeutet, und die Verbindung (152) 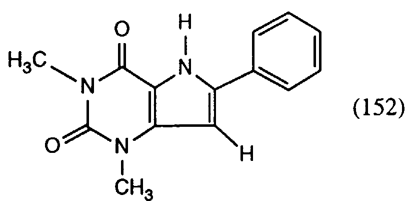 ausgeschlossen sind, wobei die Verbindungen 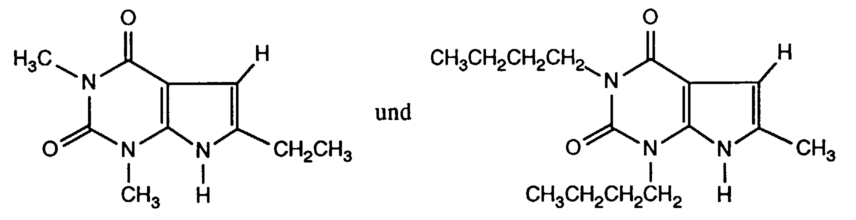 nicht ausgeschlossen sind.

Bevorzugte Gruppen von neuen Verbindungen der Formel II entsprechen den in den oben für die erfindungsgemässen Zusammensetzungen ausgedrückten Bevorzugungen.

Von besonderem Interesse sind die Verbindungen der Formel II, worin die Verbindung der Formel II eine Verbindung der Formel VII, VIIa oder VIII 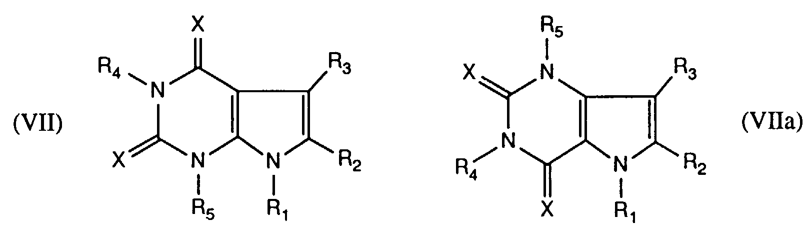 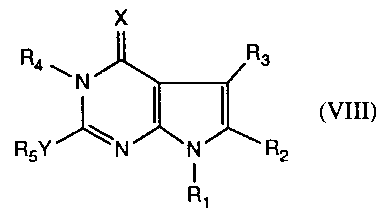 bedeutet, worin
X Sauerstoff oder Schwefel ist,
Y Sauerstoff, Schwefel oder 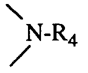 bedeutet,
R₁ Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, C₇-C₉-Phenylalkyl, ein Alkalimetall oder ein Erdalkalimetall darstellt,
R₂ und R₃ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, unsubstituiertes oder am Phenylring durch ein bis drei Reste R₆ substituiertes C₇-C₉-Phenylalkyl; 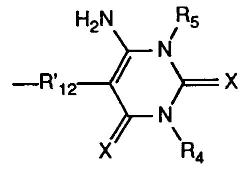 oder einen Rest der Formel IX, X, XI oder XII 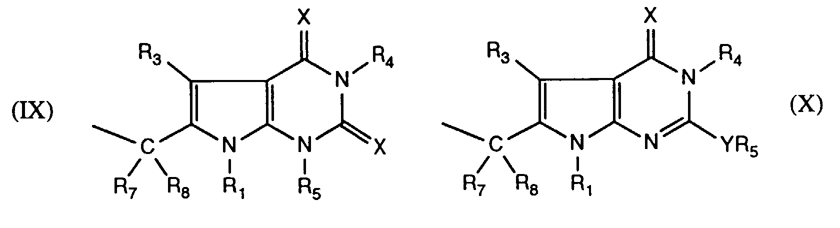 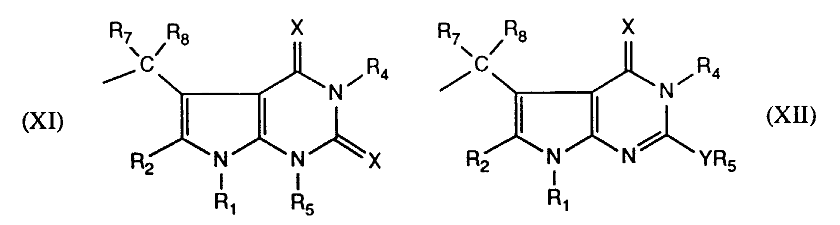 darstellt, worin X, Y, R₁, R₂ und R₃ die oben angegebene Bedeutung besitzen, oder ferner einer der Reste R₂ und R₃ Hydroxy, Formyl, C₁-C₁₀-Alkoxy, Phenoxy, C₁-C₁₀-Alkanoyloxy, Benzoyloxy, 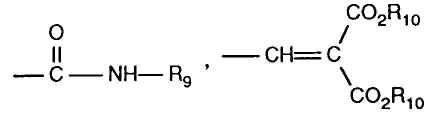 oder -CH=N-R₁₁ darstellt,
R₄ und R₅ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl oder C₇-C₉-Phenylalkyl bedeuten,
R₆ C₁-C₄-Alkyl, C₁-C₄-Alkoxy, Cyclohexyl, Hydroxy oder Chlor darstellt,
R₇ und R₈ unabhängig voneinander Wasserstoff, C₁-C₁₀-Alkyl, Phenyl oder C₇-C₉-Phenylalkyl darstellen,
R₉ Phenyl bedeutet,
R₁₀ C₁-C₁₀-Alkyl, durch Sauerstoff unterbrochenes C₂-C₁₂-Alkyl; C₃-C₆-Alkenyl, C₅-C₈-Cycloalkyl, Phenyl oder C₇-C₉-Phenylalkyl bedeutet,
R₁₁ C₁-C₁₀-Alkyl, C₅-C₈-Cycloalkyl, Phenyl oder C₇-C₉-Phenylalkyl darstellt,
R'₁₂ eine direkte Bindung oder 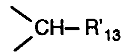 bedeutet, und
R'₁₃ Wasserstoff oder C₁-C₄-Alkyl darstellt.

Bevorzugt sind Verbindungen der Formel II, worin R₄ und R₅ unabhängig voneinander Wasserstoff oder C₁-C₄-Alkyl bedeuten.

Speziell bevorzugt sind Verbindungen der Formel II, worin
X und Y Sauerstoff sind,
R₁ Wasserstoff, 2-Hydroxyethyl, Natrium, Magnesium oder Calcium darstellt,
R₂ Wasserstoff, C₁-C₄-Alkyl, Phenyl, 2,6-Di-tert-butyl-4-hydroxybenzyl, Hydroxy, 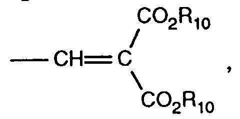 -CH=N-R₁₁ oder einen Rest der Formel IX 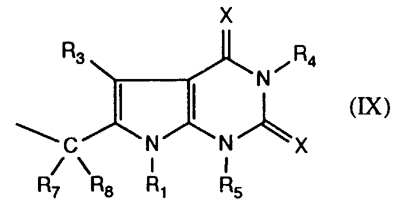 darstellt, worin X und R₁ die oben angegebene Bedeutung besitzen,
R₃ Wasserstoff, Hydroxy, C₁-C₄-Alkyl, Phenyl, 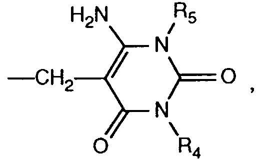 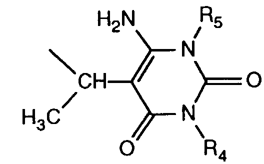 oder 2,6-Di-tert-butyl-4-hydroxybenzyl bedeutet,
R₄ und R₅ unabhängig voneinander Wasserstoff oder C₁-C₄-Alkyl bedeuten,
R₇ und R₈ unabhängig voneinander Wasserstoff, C₁-C₉-Alkyl oder Phenyl darstellen,
R₁₀ C₁-C₄-Alkyl bedeutet, und
R₁₁ Benzyl darstellt.

Die Verbindungen, die mindestens einen Rest der Formel I enthalten, beziehungsweise die Verbindungen der Formel II, sind teilweise bekannt und deren Herstellung wird beispielsweise in folgenden Publikationen beschrieben: N.M. Smirnova, L.F. Linberg, V.M. Nesterov und T.S. Safanova, Chemistry of Heterocyclic Compounds 1978, 443-446; H. Ogura, M. Sakaguchi und K. Takeda, Chem. Pharm. Bull. 20 (2), 404-408 (1972); S. Senda und K. Hirota, Chem. Pharm. Bull. 22 (12), 2921-28 (1974); S. Senda und K. Hirota, Chem. Pharm. Bull. 22 (7), 1459-1467 (1974); F. Yoneda, M. Higuchi, K. Senga, M. Kanohori und S. Nishigaki, Chem. Pharm. Bull. 21 (3), 473-477 (1973); oder K. Hirota et al., Synthesis 1982, 1097-1099. Die neuen erfindungsgemässen Verbindungen, die mindestens einen Rest der Formel I enthalten, beziehungsweise die Verbindungen der Formel II, können in Analogie zu den oben erwähnten Literaturvorschriften hergestellt werden.

Diese bekannten Methoden liefern die gewünschten erfindungsgemässen Stabilisatoren jedoch nicht immer in guten Ausbeuten.

Ein weiterer Gegenstand der Erfindung ist daher auch ein neues Verfahren zur Herstellung von Verbindungen der Formel XIII oder XIV 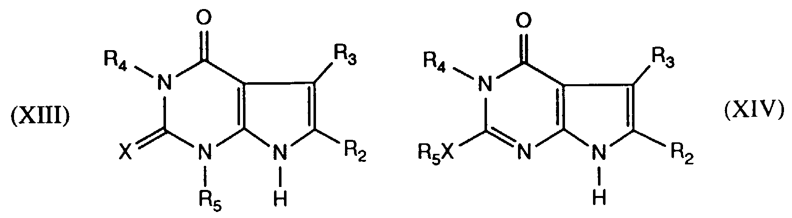 worin die allgemeinen Symbole wie in Formel II definiert sind, dadurch gekennzeichnet, dass eine Verbindung der Formel XV oder XVI 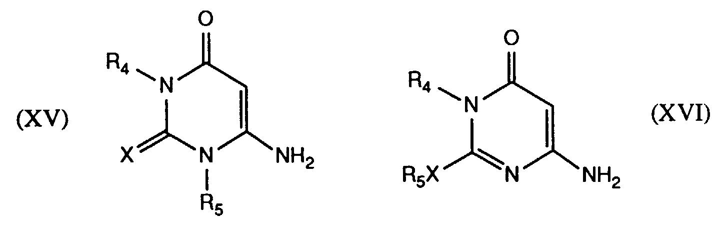 worin die allgemeinen Symbole wie in Formel II definiert sind, mit einer Verbindung der Formel XVIIa, XVIIb, XVIIc, XVIId, XVIIe oder XVIIf 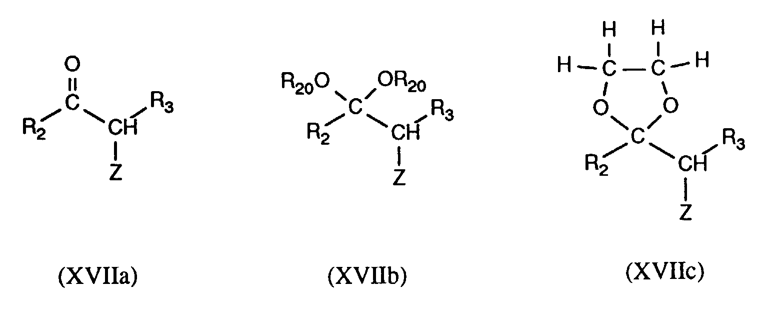 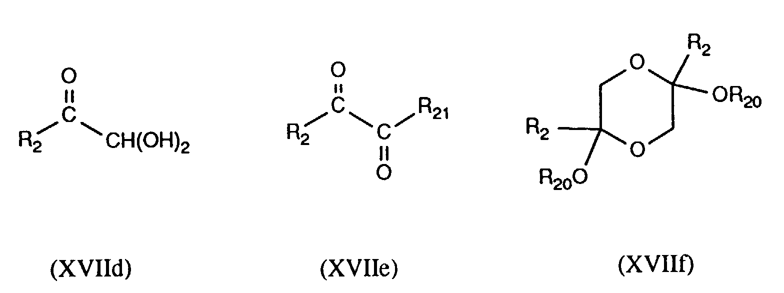 worin R₂ und R₃ wie in Formel II definiert sind, R₂₀ C₁-C₄-Alkyl darstellt, R₂₁ C₁-C₄-Alkyl bedeutet, und Z eine Abgangsgruppe darstellt, in Gegenwart eines Ammoniumsalzes umgesetzt wird.

R₂₀ und R₂₁ bedeuten als C₁-C₄-Alkyl beispielsweise Methyl, Ethyl, Propyl, Isopropyl, n-Butyl, sec-Butyl, Isobutyl oder tert-Butyl. Eine besonders bevorzugte Bedeutung von R₂₀ und R₂₁ ist Methyl oder Ethyl.

Die Umsetzung erfolgt bevorzugt in Gegenwart eines Lösungsmittels. Geeignete Lösungsmittel zur Durchführung der Reaktion sind sowohl protische wie auch dipolar aprotische Lösungsmittel.

Als protische Lösungsmittel eignen sich besonders Alkohole (beispielsweise Methanol, Ethanol, Propanol oder Isopropanol), Ketone (beispielweise Aceton oder Methylethylketon), Ether (beispielsweise Tetrahydrofuran, Dioxan, Diethylenglykol oder 2-Methoxyethanol) und Carbonsäuren (beispielsweise Essigsäure) die sich mit Wasser in jedem beliebigen Verhältnis mischen lassen. Bevorzugt wird das erfindungsgemässe Verfahren in einem wässsrigen Lösungsmittel, insbesondere wässriger Essigsäure oder nur in Wasser, durchgeführt.

Als dipolar aprotische Lösungsmittel eignen sich besonders solche, die sich mit Wasser in jedem beliebigen Verhältnis mischen lassen wie beispielsweise Acetonitril, Dimethylformamid, Dimethylacetamid, Dimethylsulfoxid oder N-Methylpyrrolidon.

Die Reaktion wird bevorzugt bei erhöhter Temperatur, insbesondere in einem Temperaturbereich von 40 bis 160°C, durchgeführt. Ein speziell bevorzugter Temperaturbereich ist 40 bis 140°C, insbesondere 60 bis 120°C.

Bei der Umsetzung der Verbindung der Formel XV oder XVI mit der Verbindung der Formel XVIIa, XVIIb oder XVIIc wird die Verbindung der Formel XVIIa, XVIIb oder XVIIc bevorzugt stöchiometrisch oder im Ueberschuss bezüglich der eingesetzten Verbindung der Formel XV oder XVI eingesetzt. Von Interesse ist ein Ueberschuss von 1,1 bis 4 Aequivalenten, insbesondere 1,1 bis 3,0 Aequivalenten, bezüglich der eingesetzten Verbindung der Formel XV oder XVI. Ein ganz besonders bevorzugtes Reaktandenverhältnis ist 1,0 bis 1,5.

Die Abgangsgruppe Z in der Verbindungen der Formel XVIIa, XVIIb oder XVIIc bedeutet beispielsweise Halogen, Hydroxy oder 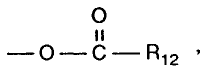 worin
R₁₂ Wasserstoff, C₁-C₄-Alkyl, unsubstituiertes oder mit R₁₃ substituiertes Phenyl darstellt; und
R₁₃ Halogen, Nitro, CF₃, C₁-C₄-Alkyl oder C₁-C₄-Alkoxy bedeutet.

Halogen bedeutet beispielsweise Chlor, Brom oder Jod. Von besonderem Interesse ist Chlor oder Brom, insbesondere Chlor.

Ist R₁₂ Alkyl mit bis zu 4 Kohlenstoffatomen, bedeutet dies einen verzweigten oder unverzweigten Rest wie beispielsweise Methyl, Ethyl, Propyl, Isopropyl, n-Butyl, sec-Butyl, Isobutyl, tert-Butyl. Eine bevorzugte Bedeutung von R₁₂ ist Methyl.

Ist R₁₂ ein unsubstituiertes oder mit R₁₃ substituiertes Phenyl bedeutet dies beispielsweise Phenyl, p-Nitrophenyl, p-Chlorphenyl, p-Methylphenyl, p-Methoxyphenyl oder p-Tri-fluormethylphenyl.

Bevorzugte Ammoniumsalze für das erfindungsgemässe Verfahren sind Verbindungen der Formel XVIII 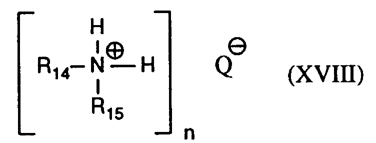 worin
n 1 oder 2 bedeutet,
R₁₄ und R₁₅ unabhängig voneinander Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₁₆ substituiertes C₅-C₈-Cycloalkyl; unsubstituiertes oder durch ein bis drei Reste R₁₆ substituiertes Phenyl; unsubstituiertes oder am Phenylring durch ein bis drei Reste R₁₆ substituiertes C₇-C₉-Phenylalkyl darstellt; oder R₁₄ und R₁₅ zusammen mit dem Stickstoffatom an das sie gebunden sind, einen C₄-C₇-Cycloalkylenring bilden, der zusätzlich mit Sauerstoff oder Schwefel unterbrochen sein kann,
R₁₆ C₁-C₄-Alkyl, C₁-C₄-Alkoxy, C₅-C₈-Cycloalkyl, Hydroxy oder Chlor darstellt, und
wenn n = 1 ist,
Q^{⊖} R₁₇-Co₂^{⊖} oder 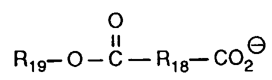 bedeutet,
wenn n = 2 ist,
Q^{⊖} Carbonat oder ^{⊖}O₂C-R₁₈-CO₂^{⊖} darstellt,
R₁₇ Wasserstoff, Hydroxy, C₁-C₁₈-Alkyl, Phenyl oder Benzyl bedeutet,
R₁₈ eine direkte Bindung oder C₁-C₆-Alkylen darstellt, und
R₁₉ Wasserstoff oder C₁-C₁₈-Alkyl bedeutet.

Speziell bevorzugte Ammoniumsalze für das erfindungsgemässe Verfahren sind beispielsweise Ammoniumformiat, Ammoniumacetat, Ammoniumpropionat, Methylammoniumacetat, Ethylammoniumacetat, n-Propylammoniumacetat, n-Butylammoniumacetat, 2-Hydroxyethylammoniumacetat, Dimethylammoniumacetat, Diethylammoniumacetat, Di-n-Butylammoniumacetat, Ammoniumcarbonat, Ammoniumhydrogencarbonat, Ammoniumoxalat, Ammoniummalonat oder Ammoniumadipat. Von besonderem Interesse ist Ammoniumacetat und Di-n-Butylammoniumacetat.

Das für das erfindungsgemässe Verfahren benötigte Ammoniumsalz kann in einem beliebigen molaren Verhältnis, d.h. im Ueberschuss oder Unterschuss, gegenüber der eingesetzten Verbindung der Formel XV oder XVI eingesetzt werden. Von Interesse ist eine Menge des Ammoniumsalzes von 0,1 bis 3 Aequivalenten, insbesondere 0,25 bis 1,5 Aequivalenten, bezüglich der eingesetzten Verbindung der Formel XV oder XVI.

Die Isolierung der Produkte nach dem erfindungsgemässen Verfahren erfolgt bevorzugt durch Filtration der ausgefallenen Produkte. Das dabei gebildete Filtrat bzw. die Mutterlauge kann als Ausgangsmaterial für eine weitere Umsetzung nach dem erfindungsgemässen Verfahren wieder eingesetzt werden.

Die vorliegende Erfindung betrifft deshalb auch ein Verfahren, worin nach Beendigung der Umsetzung ein Gemisch enthaltend einen Niederschlag der Verbindung der Formel XIII oder XIV und eine Mutterlauge gebildet wird, die Mutterlauge von dem Niederschlag getrennt wird und als Reaktionsmedium in einer weiteren Umsetzung gemäss dem oben erwähnten erfindungsgemässen Verfahren verwendet wird.

Die Verbindungen der Formel XV oder XVI sind teilweise bekannt oder können in Analogie zu N.M. Smirnova, L.F. Linberg, V.M. Nesterov und T.S. Safanova, Chemistry of Heterocyclic Compounds 1978, 443-446; H. Ogura, M. Sakaguchi und K. Takeda, Chem. Pharm. Bull. 20 (2), 404408 (1972); S. Senda und K. Hirota, Chem. Pharm. Bull. 22 (12), 2921-28 (1974); S. Senda und K. Hirota, Chem. Pharm. Bull. 22 (7), 1459-1467 (1974); oder F. Yoneda, M. Higuchi, K. Senga, M. Kanohori und S. Nishigaki, Chem. Pharm. Bull. 21 (3), 473-477 (1973) hergestellt werden.

Bevorzugte Verbindungen der Formel XVIIa, XVIIb, XVIIc, XVIId, XVIIe oder XVIIf sind beispielsweise Glyoxal, Methylglyoxal, Phenylglyoxal, Diacetyl, 2,3-Hexandion, 3,4-Hexandion, Diethoxyessigsäureethylester, Hydroxyaceton, Hydroxy-3,3-dimethylbutanon, 3-Hydroxy-2-butanon, ω-Hydroxyacetophenon, 2,5-Dimethyl-2,5-dimethoxy-1,4-dioxan, Chloraceton, Phenacylchlorid, Phenacylbromid, l-Chlor-3,3-dimethyl-2-butanon, α-Brompropiophenon, Chloracetaldehyd oder Bromacetaldehyd sowie deren Methyl- oder Ethylacetale; oder Acetoxyaceton. Diese Verbindungen sind in der Literatur bekannt oder bei Fluka bzw. Aldrich käuflich.

Die Herstellung von Verbindungen der Formel VII oder VIII worin R₂ einen Rest der Formel IX oder X bedeutet und/oder R₃ einen Rest der Formel XI oder XII darstellt, erfolgt bevorzugt ausgehend von den nach dem oben geschilderten erfindungsgemässen Verfahren hergestellten Verbindungen der Formel XIII oder XIV, worin R₂ und/oder R₃ Wasserstoff darstellt, durch Umsetzung mit einer Verbindung der Formel XIX 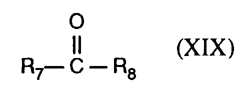 worin R₇ und R₈ die angegebenen Bedeutungen haben.

Bedeutet die Verbindung der Formel XIX einen Aldehyd, wird die Umsetzung bevorzugt in wässriger Ameisensäure, Essigsäure oder Propionsäure unter Rückfluss durchgeführt. Bedeutet die Verbindung der Formel XIX ein Keton, wird die Umsetzung bevorzugt in einem Gemisch von wässriger Jodwasserstoffsäure und Essigsäureanhydrid in einem Temperaturbereich von 20 bis 60°C, insbesondere 30 bis 50°C, durchgeführt.

Bevorzugte Verbindungen der Formel XIX sind beispielsweise Formaldehyd, Acetaldehyd, Propionaldehyd, Benzaldehyd, Aceton, Methylethylketon, Di-n-Butylketon, Methylnonylketon, Acetophenon, Cyclopentanon, Cyclohexanon oder 5-Methyl-2-hexanon.

Die folgenden Beispiele erläutern die Erfindung weiter. Angaben in Teilen oder Prozenten beziehen sich auf das Gewicht.

### Beispiel 1: Herstellung der Verbindung (101) (Tabelle 1).

Die Herstellung kann in Analogie zu dem von N.M. Smirnova, L.F. Linberg, V.M. Nesterov und T.S. Safanova, Chemistry of Heterocyclic Compounds 1978, 443-446 beschriebenen Verfahren erfolgen.

Eine Mischung von 23,1 g (0,10 Mol) 1,3-Dimethyl-5-chloroacetyl-4-aminouracil, 14,2 g (0,10 Mol) Kaliumcarbonat und 250 ml Dioxan wird während 14 Stunden unter Rühren am Rückfluss gekocht. Das Reaktionsgemisch wird abgekühlt, mit 300 ml Wasser verdünnt, am Vakuumrotationsverdampfer auf ein Volumen von 130 ml eingeengt und auf 10°C abgekühlt. Die ausgefallenen Kristalle werden abfiltriert, zweimal mit je 80 ml Wasser gewaschen und getrocknet. Es resultiert 13,0 g (61 %) der Verbindung (101) (Tabelle 1), orangefarbene Kristalle, Smp. 228-232°C.

### Beispiel 2: Herstellung der Verbindung (102) (Tabelle 1).

Die Herstellung kann in Analogie zu dem von H. Ogura, M. Sakaguchi und K. Takeda, Chem. Pharm. Bull. 20 (2), 404-408 (1972) beschriebenen Verfahren erfolgen.

Eine Mischung aus 16,7 g (0,10 Mol) Bromessigsäureethylester, 15,5 g (0,10 Mol) 4-Amino-1,3-dimethyluracil und 100 ml Dimethylformamid wird 4 Stunden unter Rühren am Rückfluss gekocht. Das Reaktionsgemisch wird dann 48 Stunden beim Raumtemperatur stehengelassen. Die Kristalle werden abfiltriert, mit Ether gewaschen und getrocknet. Kristallisation aus n-Butanol liefert 13,4 g (72 %) der Verbindung (102) (Tabelle 1), rote Kristalle, Smp. 310°C.

### Beispiel 3: Herstellung der Verbindung (103) (Tabelle 1).

Ein Gemisch aus 155,2 g (1,0 Mol) 4-Amino-1,3-dimethyluracil, 101,8 g (1,1 Mol) Chloraceton, 154,2 g (2,0 Mol) Ammoniumacetat und 1200 ml Wasser werden 14 Stunden unter Rühren am Rückfluss gekocht. Nach dem Abkühlen auf 10°C wird der Niederschlag abgesaugt, zweimal mit 100 ml Wasser gewaschen und bis zur Gewichtskonstanz getrocknet. Es resultiert 106 g (55 %) der Verbindung (103) (Tabelle 1), gelbe Kristalle, Smp. 313-314°C. Die Mutterlauge kann mehrfach wiedereingesetzt werden. Die Ausbeute erhöht sich dann auf 60 % der Theorie bei Einsatz gleicher Molmengen der Edukte.

### Beispiel 4: Herstellung der Verbindung (104) (Tabelle 1).

Die Herstellung kann in Analogie zu dem von S. Senda und K. Hirota, Chem. Pharm. Bull. 22 (12), 2921-28 (1974) beschriebenen Verfahren erfolgen.

In eine Suspension von 13,5 g (0,05 Mol) 6-Dimethylmethylen(1,3-dimethyl-2,4-dioxo-1,2,3,4-tetrahydropyrrolo[2,3-d]pyrimidin)-ammoniumchlorid [S. Senda und K. Hirota, Chem. Pharm. Bull. 22 (12), Seite 2924 (1974), Verbindung 2a] und 100 ml absolutem Ethanol werden 5,4 g (0,05 Mol) Benzylamin gegeben. Das Reaktionsgemisch wird 30 Minuten bei 25°C gerührt, dann am Vakuumrotationsverdampfer eingeengt und der Rückstand mit 200 ml Wasser versetzt Das Unlösliche wird abfiltriert und getrocknet. Kristallisation des Filterrückstandes aus i-Propanol/Wasser liefert 14,7 g (94 %) der Verbindung (104) (Tabelle 1), Smp. 121-124°C.

### Beispiel 5: Herstellung der Verbindung (105) (Tabelle 1).

Die Herstellung kann in Analogie zu dem von S. Senda und K. Hirota, Chem. Pharm. Bull. 22 (12), 2921-28 (1974) beschriebenen Verfahren erfolgen.

Zu einer Lösung von 210 ml absolutem Ethanol, 2 g (0,065 Mol) Natriumhydrid (20 % in Paraffinöl) und 9,6 g (0,06 Mol) Malonsäurediethylester werden bei Raumtemperatur 8,1 g (0,03 Mol) 6-Dimethylmethylen(1,3-dimethyl-2,4-dioxo-1,2,3,4-tetrahydropyrrolo-[2,3-d]pyrimidin)-ammoniumchlorid [siehe Beispiel 4] gegeben. Das Reaktionsgemisch wird 30 Minuten bei Raumtemperatur gerührt und anschliessend am Vakuumrotationsverdampfer eingeengt. Der Rückstand wird mit einer Mischung von 90 ml Wasser und 5 ml Eisessig angeteigt und 15 Minuten Rühren gelassen. Der gelbe Niederschlag wird abfiltriet, mit Wasser gewaschen und getrocknet. Kristallisation aus Dimethylformamid/-Ethanol liefert 6,9 g (66 %) der Verbindung (105), Smp. 249-251°C.

### Beispiel 6: Herstellung der Verbindung (106) (Tabelle 1).

In eine Suspension von 18 g (0,10 Mol) 1,3-Dimethyl-2H-pyrrolo[2,3-d]pyrimidin-2,4(3H)-dion [Verbindung (122), Beispiel 10] in 60 g Wasser wird unter Rühren am Rückfluss eine Lösung von 5,7 g (0,105 Mol) Ameisensäure (85 %ig) und 13,4 g (0,165 Mol) Formaldehydlösung (37 %ig) innerhalb 60 Minuten eingetropft. Anschliessend wird das Reaktionsgemisch weitere 6 Stunden bei 100°C gerührt, dann auf 20°C abgekühlt und mit 100 ml Wasser verdünnt. Der Niederschlag abfiltriet, mit Wasser gewaschen und bis zur Gewichtskonstanz getrocknet. Kristallisaton des Rückstandes aus Dimethylformamid liefert 18,0 g (97 %) der Verbindung (106) (Tabelle 1), rosa Kristalle, Smp. >320°C.

Wird in Analogie zu Beispiel 6 anstelle von 1,3-Dimethyl-2H-pyrrolo[2,3-d]pyrimidin-2,4(3H)-dion [Verbindung (122)] das 2H-Pyrrolo[2,3-d]pyrimidin-2,4(3H)-dion [Verbindung (123), Beispiel 10] eingesetzt, wird die Verbindung (107) (Tabelle 1) erhalten.

Wird in Analogie zu Beispiel 6 anstelle von Formaldehydlösung Acetaldehyd, Propionaldehyd oder Benzaldehyd eingesetzt, werden die Verbindungen (108), (109) und (110) erhalten (Tabelle 1).

### Beispiel 7: Herstellung der Verbindung (111) (Tabelle 1).

Die Herstellung kann in Analogie zu dem von S. Senda und K. Hirota, Chem. Pharm. Bull. 22 (7), 1459-1467 (1974) beschriebenen Verfahren erfolgen.

13,5 g (0,06 Mol) einer "Schiff"-Base aus 4-Hydrazino-1,3-dimethyluracil und Ethylmethylketon werden unter Rühren mit 60 ml Ethylenglykol versetzt und während 1 Stunde bei 190°C gehalten. Das Reaktionsgemisch wird abgekühlt und mit 50 ml i-Propanol versetzt. Der ausgefallene Niederschlag wird abfiltriert, mit i-Propanol/Petrolether gewaschen und getrocknet. Kristallisation des Filterrückstandes aus Ethanol/Wasser liefert 9,8 g (79 %) der Verbindung (111) (Tabelle 1), weisse Kristalle, Smp. 318-320°C.

### Beispiel 8: Herstellung der Verbindung (112) (Tabelle 1).

Die Herstellung kann in Analogie zu dem von H. Ogura, M. Sakaguchi und K. Takeda, Chem. Pharm. Bull. 20 (2), 404-408 (1972) beschriebenen Verfahren erfolgen.

Eine Mischung von 31 g (0,20 Mol) 4-Amino-1,3-dimethyluracil, 40 g (0,20 Mol) Phenacylbromid und 250 ml Dimethylformamid wird während 2 Stunden bei 135°C gerührt. Anschliessend wird das Reaktionsgemisch am Vakuumrotationsverdampfer eingeengt. Der Rückstand wird mit 200 ml Wasser aufgerührt, der Festkörper abfiltriert, zweimal mit 50 ml Wasser gewaschen und bis zur Gewichtskonstanz getrocknet. Kristallisation aus Eisessig/Alkohol liefert 46,2 g (91 %) der Verbindung (112) (Tabelle 1), hellbraune Kristalle, Smp. 289-296°C.

### Beispiel 9: Herstellung der Verbindung (112) und (113) (Tabelle 1).

Die Herstellung kann in Analogie zu dem von F. Yoneda, M. Higuchi, K. Senga, M. Kanohori und S. Nishigaki, Chem. Pharm. Bull. 21 (3), 473-477 (1973) beschriebenen Verfahren erfolgen.

Eine Mischung aus 15,5 g (0,10 Mol) 4-Amino-1,3-dimethyluracil, 19,9 g (0,10 Mol) Phenacylbromid und 150 ml Eisessig wird während 5 Stunden bei 90°C gerührt. Anschliessend wird das Reaktionsgemisch am Vakuumrotationsverdampfer eingeengt. Der Rückstand wird mit 300 ml Wasser angeteigt, das Ungelöste abfiltriert, mit Wasser gewaschen und getrocknet. Kristallisation des Rückstandes aus Ethanol liefert 16,7 g (65 %) der Verbindung (112) (Tabelle 1), weisse Kristalle. Die Mutterlauge wird anschliessend mittels Ammoniaklösung (25 %ig) auf pH 10 eingestellt und 10 Minuten gerührt. Der erhaltene Niederschlag wird abfiltriert, mit Wasser gewaschen und getrocknet. Kristallisation des Filterrückstandes aus Ethanol/Wasser liefert 3,5 g (14 %) der Verbindung (113), weisse Kristalle, Smp. 126-127°C.

In Analogie zu Beispiel 9 werden anstelle vom 4-Amino-1,3-dimethyluracil mit 4-Amino-uracil, 4-Amino-3-n-propyluracil, 4-Amino-1,3-di-n-propyluracil, 4-Amino-1,3-di-n-butyl-2-thio-uracil und 4-Amino-1,3-diethyl-2-thio-uracil die Verbindungen (114), (115), (116), (117) und (118) erhalten (Tabelle 1).

### Beispiel 10: Herstellung der Verbindung (122) (Tabelle 1).

Zu einer gerührten Mischung von 31 g (0,10 Mol) 4-Amino-1,3-dimethyluracil, 15,4 g (0,20 Mol) Ammoniumacetat und 150 ml Wasser wird bei einer Temperatur von 60-65°C eine Lösung von 52,4 g (0,30 Mol) Chloracetaldehyd (45 %ige wässrige Lösung) und 15,4 g (0,20 Mol) Ammoniumacetat in 50 ml Wasser innerhalb 10 Minuten getropft. Es bildet sich ein Niederschlag. Das Reaktionsgemisch wird noch 15 Minuten nachgerührt, der Rührer abgestellt und 12 Stunden bei Raumtemperatur stehengelassen. Der Niederschlag wird abfiltriert, mit Wasser gewaschen und bis zur Gewichtskonstanz getrocknet. Es resultiert 23,6 g (66 %) der Verbindung (122) (Tabelle 1) als weisses Pulver, Smp. 288-292°C.

In Analogie zu Beispiel 10 werden anstelle von 4-Amino-1,3-dimethyluracil mit 4-Amino-uracil, 4-Amino-3-n-propyluracil und 4-Amino-1,3-diethyluracil die Verbindungen (123), (124) und (125) erhalten (Tabelle 1).

Wird in Analogie zu Beispiel 10 anstelle von Chloracetaldehyd α-Chlorpropionaldehyd verwendet, so wird die Verbindung (126) erhalten (Tabelle 1).

### Beispiel 11: Herstellung eines Gemisches der Verbindungen (103) und (127).

Ein Gemisch von 31 g (0,20 Mol) 4-Amino-1,3-dimethyluracil, 25,5 g (0,22 Mol) Acetoxyaceton, 12,2 g (0,20 Mol) Ethanolamin und 12 g (0,20 Mol) Eisessig in 200 ml Wasser wird während 9 Stunden unter Rückfluss gekocht. Anschliessend wird das Reaktionsgemisch auf 10°C abgekühlt, der ausgefallene Niederschlag abfiltriert und bis zur Gewichtskonstanz getrocknet. Es resultiert 18,9 g eines ca. 1:1 Gemisches der Verbindungen (103) und (127), weisse Kristalle, Smp. 246°C. Die Bestimmung der Zusammensetzung erfolgt mittels ¹H-NMR.

### Beispiel 12: Herstellung der Verbindung (103) (Tabelle 1).

Eine Mischung von 31,0 g (0,10 Mol) 4-Amino-1,3-dimethyluracil, 25,5 g (0,22 Mol) Acetoxyaceton und 15,4 g (0,20 Mol) Ammoniumacetat in 200 ml Wasser wird während 12 Stunden unter Rückfluss gekocht. Anschliessend wird das Reaktionsgemisch auf 10°C abgekühlt, der ausgefallene Niederschlag abgesaugt, zweimal mit je 50 ml Wasser gewaschen und bis zur Gewichtskonstanz im Vakuum bei 60°C getrocknet. Es resultiert 28,6 g (74 %) der Verbindung (103), hellbeige Kristalle, Smp. 313-314°C. Wird die Mutterlauge als Reaktionsmedium bei gleicher Reaktionsführung wiederverwendet, erhöht sich die Ausbeute auf über 80 % der Theorie.

In Analogie zu Beispiel 12 werden anstelle von 4-Amino-1,3-dimethyluracil mit 4-Amino-1,3-diethyluracil, 4-Amino-3-n-propyluracil, 4-Amino-1,3-di-n-butyluracil und 4-Amino-1,3-di-n-propyluracil die Verbindungen (129), (130), (131) und (133) erhalten (Tabelle 1).

Wird in Analogie zu Beispiel 12 anstelle von Acetoxyaceton 1-Acetoxy-2-butanon [Beilstein EIII, Band 2, Seite 360] verwendet, so wird die Verbindung (128) erhalten (Tabelle 1).

Wird in Analogie zu Beispiel 12 anstelle von 4-Amino-1,3-dimethyluracil und Acetoxyaceton 4-Amino-1,3-diethyluracil und 1-Acetoxy-2-butanon [Beilstein EIII, Band 2, Seite 360] verwendet, so wird die Verbindung (132) erhalten (Tabelle 1).

Bei höher alkylierten Aminouracilen kann dem Reaktionsgemisch ein mit Wasser mischbares Lösungsmittel wie z.B. n-Propanol, Dioxan oder Dimethylformamid zugesetzt werden.

### Beispiel 13: Herstellung der Verbindungen (134) und (135).

Ein Gemisch aus 9,7 g (0,05 Mol) 1,3-Dimethyl-2H-pyrrolo[2,3-d]pyrimidin-2,4(3H)-dion [Verbindung (122), Beispiel 10] und 13,2 g (0,05 Mol) 4-(Dimethylaminomethylen)-2,6-di-tert-butylphenol in 60 ml Essigsäure wird während 4 Stunden bei 120°C gerührt. Das Reaktionsgemisch wird anschliessend noch 30 Minuten nachgerührt und die ausgefallenen Kristalle filtriert. Kristallisation des Rückstandes aus Methanol liefert 5,5 g (23 %) der Verbindung (134) (Tabelle 1), weisse Kristalle, Smp. 233°C. Die Mutterlauge wird mit 200 ml Wasser versetzt, 1,5 Stunden gerührt, der Niederschlag abfiltriert und getrocknet. Kristallisation des Rückstandes aus Toluol liefert 8,5 g (43 %) der Verbindung (135) (Tabelle 1), weisse Kristalle, Smp. 250-260°C.

### Beispiel 14: Herstellung der Verbindung (134) (Tabelle 1).

Eine Mischung von 9,0 g (0,05 Mol) 1,3-Dimethyl-2H-pyrrolo[2,3-d]pyrimidin-2,4(3H)-dion [Verbindung (122), Beispiel 10], 22,6 g (0,086 Mol) 4-(Dimethylaminomethylen)-2,6-di-tert-butylphenol und 1 ml Natriumethylatlösung (30 %ig) in 100 ml absolutem Ethanol wird während 8 Stunden unter Rühren am Rückfluss gekocht. Anschliessend wird das Reaktionsgemisch auf 10°C abgekühlt, das ausgefallene Produkt abfiltriert, mit Ethanol/Petrolether gewaschen und getrocknet. Es resultiert 21,6 g (88 % der Verbindung (134) (Tabelle 1), weisse Kristalle, Smp. 235-238°C.

### Beispiel 15: Herstellung der Verbindung (136) (Tabelle 1).

Zu einer frisch zubereiteten Magnesiummethylat-Lösung, hergestellt aus 1,34 g (0,055 Mol) Magnesium-Spänen und 150 ml absolutem Methanol, werden 17,9 g (0,10 Mol) 1,3-Dimethyl-2H-pyrrolo[2,3-d]pyrimidin-2,4(3H)-dion [Verbindung (122), Beispiel 10] portionsweise unter Rühren bei 40°C gegeben. Das Reaktionsgemisch wird anschliessend 2 Stunden am Rückfluss gekocht und dann auf 20°C abgekühlt. Das ausgefallene Produkt wird abfiltriert, mit Methanol und Petrolether gewaschen und getrocknet Es resultiert 20 g (95 %) der Verbindung (136) (Tabelle 1), hellgraues Pulvers, Smp. >340°C. Mg-Bestimmung: berechnet: 6,4 % Mg; gefunden: 7,0 % Mg.

In Analogie zu Beispiel 15 wird anstelle von 1,4 g Magnesium mit 2,2 g (0,055 Mol) Calcium die Verbindung (138) erhalten (Tabelle 1). Ausbeute: 20,2 g (95 %), helles Pulver, Smp. >340°C. Ca-Bestimmung: berechnet: 10,1 %; gefunden: 9,5 %.

### Beispiel 16: Herstellung der Verbindung (137) (Tabelle 1).

Ein Gemisch von 5,4 g (0,03 Mol) 1,3-Dimethyl-2H-pyrrolo[2,3-d]pyrimidin-2,4(3H)-di-on [Verbindung (122), Beispiel 10] in 100 ml absolutem Methanol wird mit 5,4 g (0,03 Mol) einer 30 %igen Natriummethylatlösung versetzt. Die klare Lösung wird anschliessend am Vakuumrotationsverdampfer eingeengt. Es resultiert 6,0 g (95 %) der Verbindung (137) (Tabelle 1), graues Pulvers, Smp. >340°C.

### Beispiel 17: Herstellung der Verbindung (139) (Tabelle 1).

Zu 57,5 g (0,25 Mol) Jodwasserstoffsäure (57%-ig in Wasser) wird innerhalb von 75 Minuten bei ca. 10°C unter Rühren 50 ml Essigsäureanhydrid getropft. Anschliessend werden 9,0 g (0,05 Mol) 1,3-Dimethyl-2H-pyrrolo[2,3-d]pyrimidin-2,4(3H)-dion [Verbindung (122), Beispiel 10] und 5,7 g (0,04 Mol) Di-n-Butylketon zugegeben. Dann wird das Reaktionsgemisch 15 Minuten bei 20°C und 120 Minuten bei ca. 40°C gerührt. Nach dem Abkühlen wird das Reaktionsgemisch in 500 ml Wasser eingerührt, die wässrige Phase abdekantiert und der Rückstand mit 500 ml Diethylether 1 Stunde gerührt. Der Niederschlag wird filtriert und getrocknet. Kristallisation aus n-Propanol und Reinigung mit Aktivkohle liefert 10,0 g (83 %) der Verbindung (139) (Tabelle 1), braunes Pulver, Smp. 232-234°C.

In Analogie zu Beispiel 17 werden anstelle von Di-n-Butylketon mit Aceton, Methylnonylketon und Acetophenon die Verbindungen (140), (141) und (142) erhalten (Tabelle 1).

### Beispiel 18: Herstellung der Verbindung (119) (Tabelle 1).

a) Zu einer Lösung von 86,08 g (0,50 Mol) O-Methylisoharnstoff-Hydrogensulfat in 300 ml absolutem Methanol wird unter Stickstoff-Atmosphäre 69,0 g (0,61 Mol) Cyanessigsäureethylester gegeben. Anschliessend wird 216 g (1,20 Mol) einer 30 %igen Lösung von Natriummethylat in Methanol zugetropft Das Reaktionsgemisch erwärmt sich auf 57,5°C. Anschliessend wird das Reaktionsgemisch während 5 Stunden unter Rückluss gekocht, dann abgekühlt und über Nacht stehen gelassen. Die ausgefallenen Salze (NaHSO₄) werden abfiltriert und das Filtrat am Vakuumrotationsverdampfer eingeengt. Der Rückstand wird in ca. 300 ml Wasser unter Erwärmen aufgenommen. Sobald eine klare Lösung vorliegt, wird mit ca. 35 ml Eisessig angesäuert (pH ca. 6). Anschliessend wird im Eis/-Wasser-Bad abgekühlt, das ausgefallene Produkt abfiltriert und mit Wasser gewaschen. Trocknen des Rückstandes im Vakuumtrockenschrank liefert 69,2 g (70,6 %) der Verbindung (119a), Smp. 232-235°C. 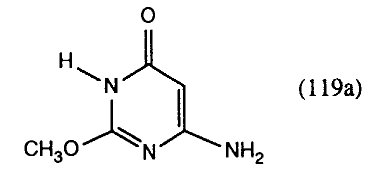
b) Ein Gemisch von 7,1 g (0,05 Mol) der Verbindung (119a) [Herstellung siehe Beispiel 18a] und 10,0 g (0,05 Mol) Phenacylbromid in 70 ml Dimethylformamid wird während 3 Stunden bei ca. 130°C gerührt. Das Reaktionsgemisch wird anschliessend auf Raumtemperatur abgekühlt Der Niederschlag wird filtriert und getrocknet. Es resultiert 3,7 g (30 %) der Verbindung (119) (Tabelle 1), Smp. >350°C.

### Beispiel 19: Herstellung der Verbindung (120) (Tabelle 1).

Eine Lösung von 15,5 g (0,10 Mol) 4-Amino-1,3-dimethyluracil und 20,2 g (0,15 Mol) 1-Chlor-3,3-dimethyl-2-butanon (Aldrich) in 75 ml Dimethylacetamid wird 2,5 Stunden bei 160°C gerührt. Nach dem Abkühlen wird eine Klärfiltration vorgenommen und das Filtrat am Vakuumrotationsverdampfer eingeengt. Der Rückstand wird in Methylenchlorid gelöst und zweimal mit Wasser gewaschen. Die organische Phase wird über Natriumsulfat getrocknet und am Vakuumrotationsverdampfer eingeengt. Chromatographie des Rückstandes an Kieselgel liefert 3,5 g (15 %) der Verbindung (120) (Tabelle 1), gelbe Kristalle, Smp. 226-232°C.

### Beispiel 20: Herstellung der Verbindung (121) (Tabelle 1).

Ein Gemisch von 15,5 g (0,10 Mol) 4-Amino-1,3-dimethyluracil und 23,4 g (0,11 Mol) α-Brompropiophenon (Aldrich) in 100 ml Dimethylformamid wird während 3 Stunden bei ca. 130°C gerührt. Anschliessend wird das Reaktionsgemisch abgekühlt und am Vakuumrotationsverdampfer eingeengt. Der Rückstand wird mit 100 ml Ethanol versetzt. Der ausgefallene Niederschlag wird filtriert und mit Ethanol und Methyl-tert-butylether gewaschen. Kristallisation aus n-Butanol liefert 11,4 g (42 %) der Verbindung (121) (Tabelle 1), gelbe Kristalle, Smp. 270-272°C.

### Beispiel 21: Herstellung der Verbindung (103) (Tabelle 1).

Zu einer Mischung aus 186 g (1,20 Mol) 4-Amino-1,3-dimethyluracil und 23,1 g (0,30 Mol) Ammoniumacetat in 72 g (1,20 Mol) Eisessig und 185 g Wasser werden unter Rühren und unter Rückfluss während 90 Minuten 101 g (1,32 Mol) Hydroxyaceton getropft. Anschliessend wird während 3 Stunden unter Rückfluss gekocht. Das Reaktionsgemisch wird abgekühlt, der Niederschlag filtriert, der Rückstand dreimal mit je 80 ml Wasser gewaschen und anschliessend getrocknet. Es resultieren 188 g (81 %) der Verbindung (103), beige Kristalle, Smp, 313-314°C.

In Analogie zu Beispiel 21 werden anstelle von Hydroxyaceton mit Hydroxy-3,3-dimethylbutanon [J. Org. Chem. 1993 (58), 7557], 3-Hydroxy-2-butanon (Aldrich), ω-Hydroxyacetophenon [J. Org. Chem. 1993 (58), 7557] und 2,5-Dimethyl-2,5-di-methoxy-1,4-dioxan [Houben-Weyl VII/2a, Seite 828] die Verbindungen (128) (Ausbeute 84%); (120) (Ausbeute 25%); (111) (Ausbeute 32%); (112) (Ausbeute 94%); und (103) (Ausbeute 84 %) erhalten.

### Beispiel 22: Herstellung der Verbindung (131) (Tabelle 1).

Zu einer Mischung aus 48 g (0,20 Mol) 4-Amino-1,3-di-n-butyluracil und 15,4 g (0,20 Mol) Ammoniumacetat in 36 g Eisessig und 130 g Wasser werden unter Rühren und unter Rückfluss während 100 Minuten eine Lösung von 19,2 g (0,22 Mol) Hydroxyaceton (85 %ig) in 15 g n-Propanol getropft. Anschliessend wird während 6 Stunden unter Rückfluss gekocht Das Reaktionsgemisch wird abgekühlt und mit 300 ml Methylenchlorid versetzt. Die organische Phase wird abgetrennt, mit Wasser, Natriumhydrogencarbonat-Lösung und nochmals mit Wasser gewaschen, über Natriumsulfat getrocknet und am Vakuumrotationsverdampfer eingeengt Der Rückstand liefert 46,0 g eines Gemisches enthaltend 84 Mol-% der Verbindung (131) und 16 Mol-% des Ausgangsmaterials (4-Amino-1,3-di-n-butyluracil) als hellbraune Kristalle. Die Bestimmung der Zusammensetzung erfolgt mittels ¹H-NMR-Spektrum.

### Beispiel 23: Herstellung der Verbindung (143) (Tabelle 1).

Zu einer Mischung aus 22,9 g (0,10 Mol) 4-Amino-1,3-di-n-propyluracil und 7,7 g (0,10 Mol) Ammoniumacetat in 25 g Eisessig und 50 g Wasser werden unter Rühren und unter Rückfluss während 50 Minuten eine Lösung von 10,2 g (1,10 Mol) Hydroxy-2-butanon in 10 ml Wasser getropft Anschliessend wird während 3 Stunden unter Rückfluss gekocht. Das Reaktionsgemisch wird abgekühlt und mit 500 ml Wasser versetzt. Die organische Phase wird mit 100 ml Methylenchlorid extrhiert. Die organische Phase wird abgetrennt, mit Wasser, Natriumhydrogencarbonat-Lösung und nochmals mit Wasser gewaschen, über Natriumsulfat getrocknet und am Vakuumrotationsverdampfer eingeengt. Kristallisation des Rückstandes aus Essigsäureethylester liefert 8,8 g (34 %) der Verbindung (143) (Tabelle 1), weisse Kristalle, Smp. 141-142°C.

### Beispiel 24: Herstellung der Verbindung (144) (Tabelle 1).

Ein Gemisch aus 39,9 g (0,20 Mol) 4-Amino-1,3-diethylthiouracil, 22,9 g (0,30 Mol) Hydroxyaceton, 23,1 g (0,30 Mol) Ammoniumacetat, 30 g Essigsäure und 50 g Wasser wird 18 Stunden unter Rühren am Rückfluss gekocht. Das Reaktionsgemisch wird anschliessend abgekühlt und der Niederschlag abfiltriert. Der klebrige Niederschlag wird mit 50 ml n-Propanol aufgekocht, dann abgekühlt, das Ungelöste filtriert und der Rückstand mit Petrolether gewaschen. Nach dem Trocknen werden 3,3 g (6,9 %) der Verbindung (144) (Tabelle 1), beige Kristalle, Smp. 301-303°C, erhalten.

### Beispiel 25: Herstellung der Verbindung (146) (Tabelle 1).

Ein Gemisch aus 31,1 g (0,20 Mol) 4-Amino-1,3-dimethyluracil, 39,6 g (0,22 Mol) einer 40 %-wässrigen Methylglyoxal-Lösung und 150 ml Wasser wird während 3 Stunden bei Raumtemperatur gerührt. Der Niederschlag wird anschliessend filtriert, mit Wasser und n-Propanol gewaschen und bis zur Gewichtskonstanz getrocknet. Es resultieren 31,6 g (91,3 %) der Verbindung (146) (Tabelle 1), weisse Kristalle, Smp. > 330°C.

In Analogie zu Beispiel 25 werden anstelle von Methylglyoxal-Lösung mit Glyoxal (Aldrich) und Diacetyl (Aldrich) die Verbindungen (145) und (147) (Tabelle 1) erhalten.

In Analogie zu Beispiel 25 werden des weitern anstelle von Methylglyoxal-Lösung mit 2,3-Hexandion, 3,4-Hexandion (Aldrich) und Phenylglyoxal plus zusätzlich jeweils 50 ml Essigsäure die Verbindungen (148), (149) und (151) erhalten. Die Aufarbeitung der Verbindung (149) erfolgt nach der Umsetzung durch Einengen des Reaktionsgemisches auf Rückstand.

### Beispiel 26: Herstellung der Verbindung (150) (Tabelle 1).

Ein Gemisch aus 31,0 g (0,20 Mol) 4-Amino-1,3-dimethyluracil, 38,8 g (0,22 Mol) Diethoxyessigsäureethylester (Aldrich), 7,7 g (0,10 Mol) Ammoniumacetat, 150 ml Wasser und 50 g Essigsäure wird während 7,5 Stunden unter Rückfluss gekocht. Anschliessend wird das Reaktionsgemisch abgekühlt, der Niederschlag filtriert, der Rückstand mit Wasser gewaschen und dann mit 100 ml Aceton aufgeschlämmt. Das Produkt wird erneut filtriert, der Rückstand mit Aceton gewaschen und bis zur Gewichtskonstanz getrocknet. Es resultieren 14,2 g (41 %) der Verbindung (150), beige Kristalle, Smp. 276-279°C.

### Beispiel 27: Herstellung der Verbindung (152) (Tabelle 1).

a) 50,5 g (0,40 Mol) 6-Methyluracil (Merck) werden unter Rühren während einer Stunde portionenweise bei 0 bis +5°C in eine Lösung aus 200 ml 100 %iger Salpetersäure (rauchend) und 50 g Phosphorpentoxid eingetragen. Nach Beendigung der exothermen Reaktion wird während 5 Stunden bei +5°C weiter gerührt Anschliessend wird das Reaktionsgemisch auf 1 kg Eiswasser gegossen. Der gebildete Niederschlag wird filtriert, mit Wasser gewaschen und anschliessend bis zur Gewichtskonstanz getrocknet. Es resultieren 37 g (54 %) 5-Nitro-6-methyl-uracil, blassgelbes Pulver, Smp. 281°C unter Zersetzung.
b) In 40 g (1,0 Mol) 2 N Natronlauge werden bei Raumtemperatur unter Rühren portionenweise 171 g (1,00 Mol) 5-Nitro-6-methyl-uracil (Beispiel 27a) eigetragen, wobei sich die anfangs klare Lösung unter leichter Exothermie breiartig verändert. Bei strikter Temperatur- und pH-Kontrolle (T = 40°C; pH = 8-9) werden gleichzeitig unter Rühren während 3 Stunden 400 g (3,2 Mol) Dimethylsulfat und 4 N Natronlauge zugetropft. Anschliessend wird das Reaktionsgemisch nachgerührt und der gebildete Niederschlag nach Stehen über Nacht filtriert. Der Rückstand wird mit Wasser gewaschen und anschliessend bis zur Gewichtskonstanz getrocknet. Es resultieren 97 g (49 %) 5-Nitro-1,3,6-trimethyl-uracil, hellgelbes Pulver, Smp. 152°C.
c) 39,8 g (0,20 Mol) 5-Nitro-1,3,6-trimethyl-uracil (Beispiel 27b) werden in 150 ml absolutem Ethanol, das 17,4 g Natriumethylat enthält, unter Rühren am Rückfluss während einer Stunde gekocht. Anschliessen wird auf Raumtemperatur abgekühlt, filtriert, der Rückstand mit warmem Ethanol gewaschen und anschliessend bis zur Gewichtskonstanz getrocknet. Es resultieren 44,3 g (99 %) Natriumsalz von 5-Nitro-1,3,6-trimethyl-uracil als gelbes Pulver.
d) Ein Gemisch von 22,1 g (0,10 Mol) Natriumsalz von 5-Nitro-1,3,6-trimethyl-uracil (Beispiel 27c), 15,2 g (0,12 Mol) Benzylchlorid, 15,2 g (0,11 Mol) Kaliumcarbonat und 6,1 g (0,036 Mol) Kaliumiodid in 150 ml Dimethylformamid wird während 4 Stunden unter Rühren bei 120°C gehalten. Anschliessend werden am Vakuumrotationsverdampfer die flüchtigen Anteile abgezogen, der Rückstand mit 400 g Eiswasser versetzt und mit 50 g Essigsäure unter Rühren angesäuert. Der gebildete Niederschlag wird filtriert, der Rückstand mit Wasser neutral gewaschen und bis zur Gewichtskonstanz getrocknet. Dieser Rückstand wird in 250 ml Dimethylformamid gelöst und 2 Stunden unter Rückfluss gekocht. Das Reaktionsgemisch wird anschliessend am Vakuumrotationsverdampfer eingeengt. Der Rückstand wird in 150 ml Ethanol heiss aufgenommen. Der Niederschlag wird filtriert, der Filterrückstand mit Ethanol gewaschen und bis zur Gewichtskonstanz getrocknet. Es resultieren 8,94 g (35%) der Verbindung (152) (Tabelle 1), beiges Pulver, Smp. 314°C.

In Analogie zu Beispiel 27d wird anstelle von Benzylchlorid mit 4-tert-Butylbenzylchlorid die Verbindung (153) (Tabelle 1), Ausbeute 20 %, Smp. 307°C, erhalten.

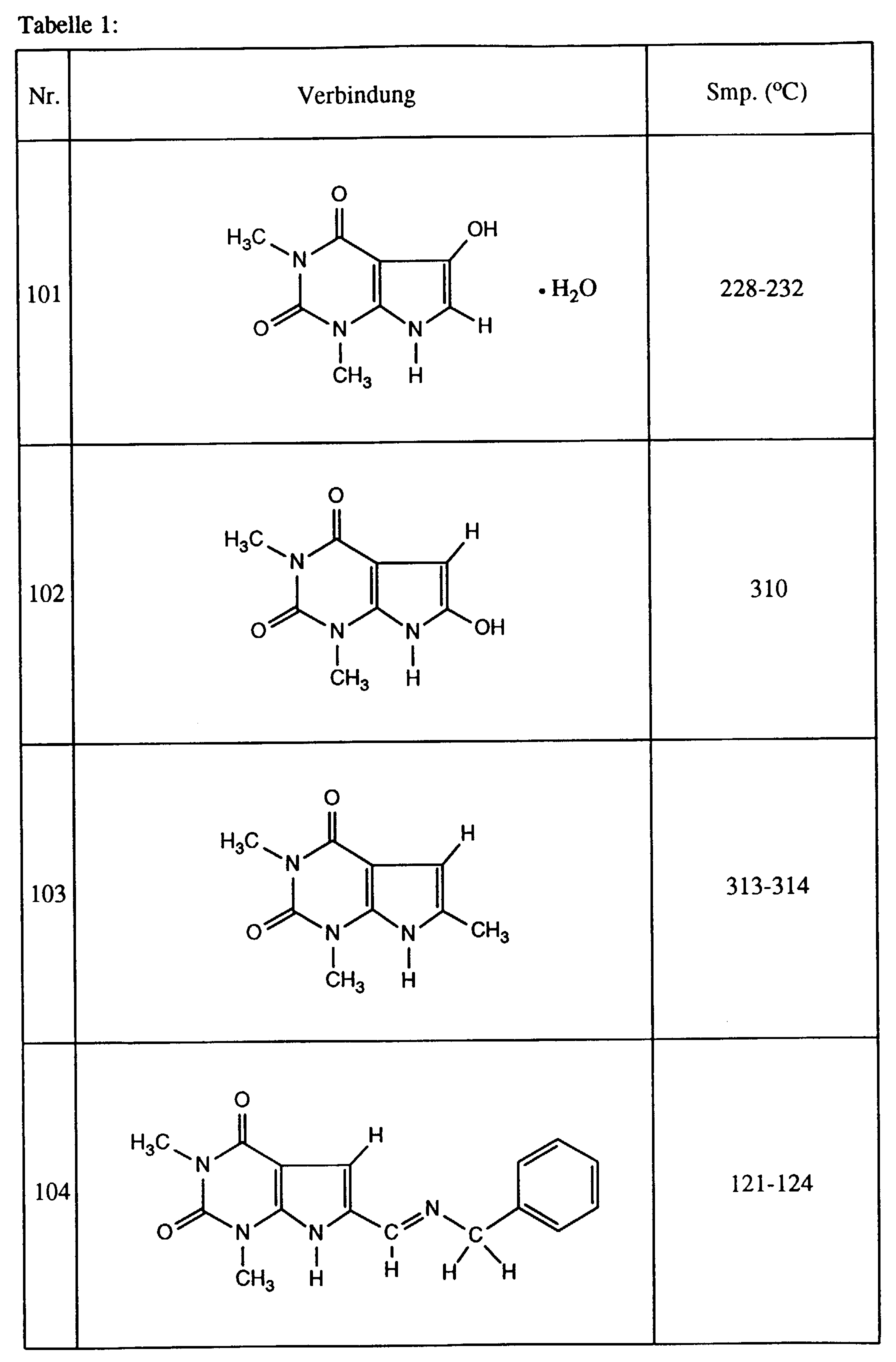

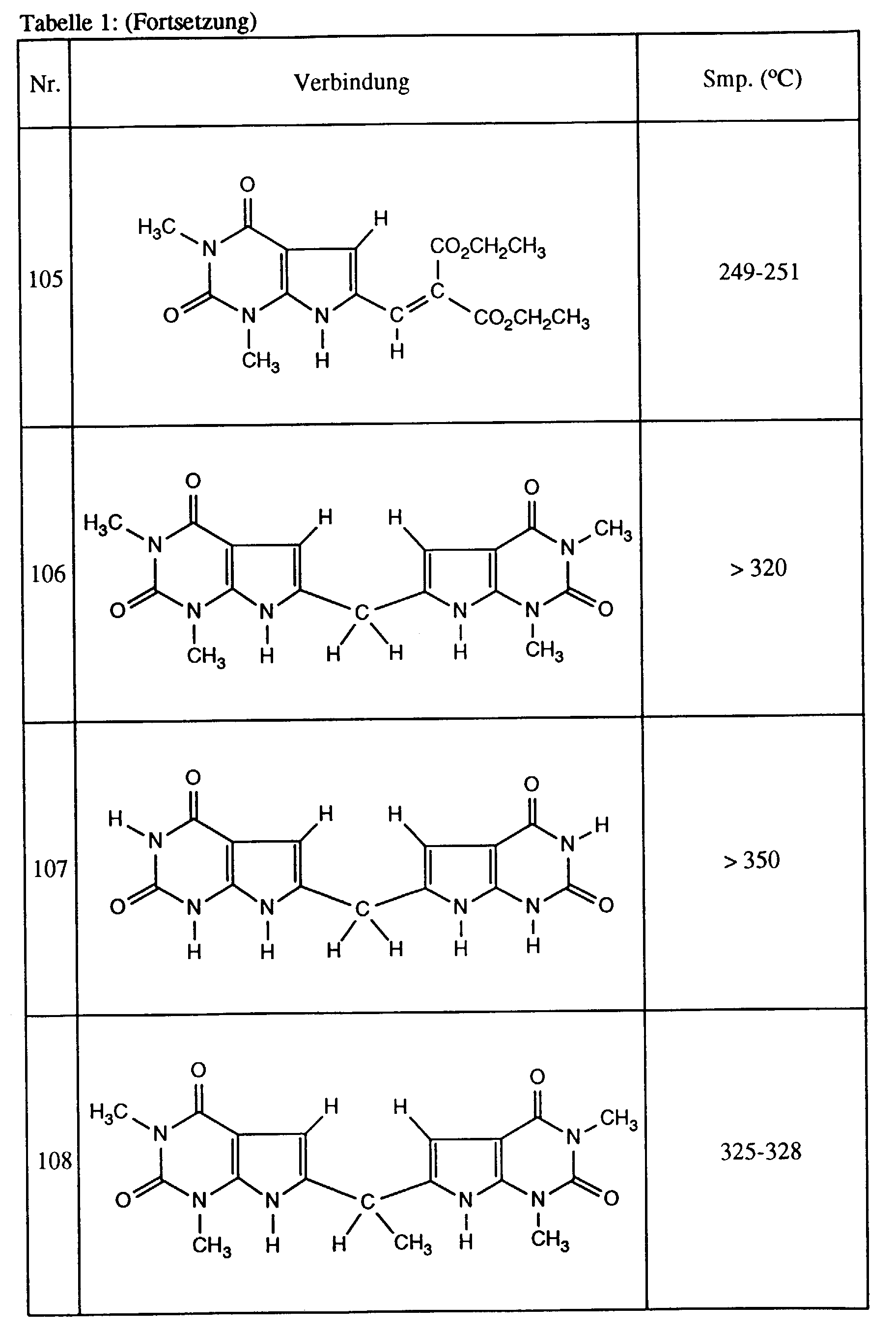

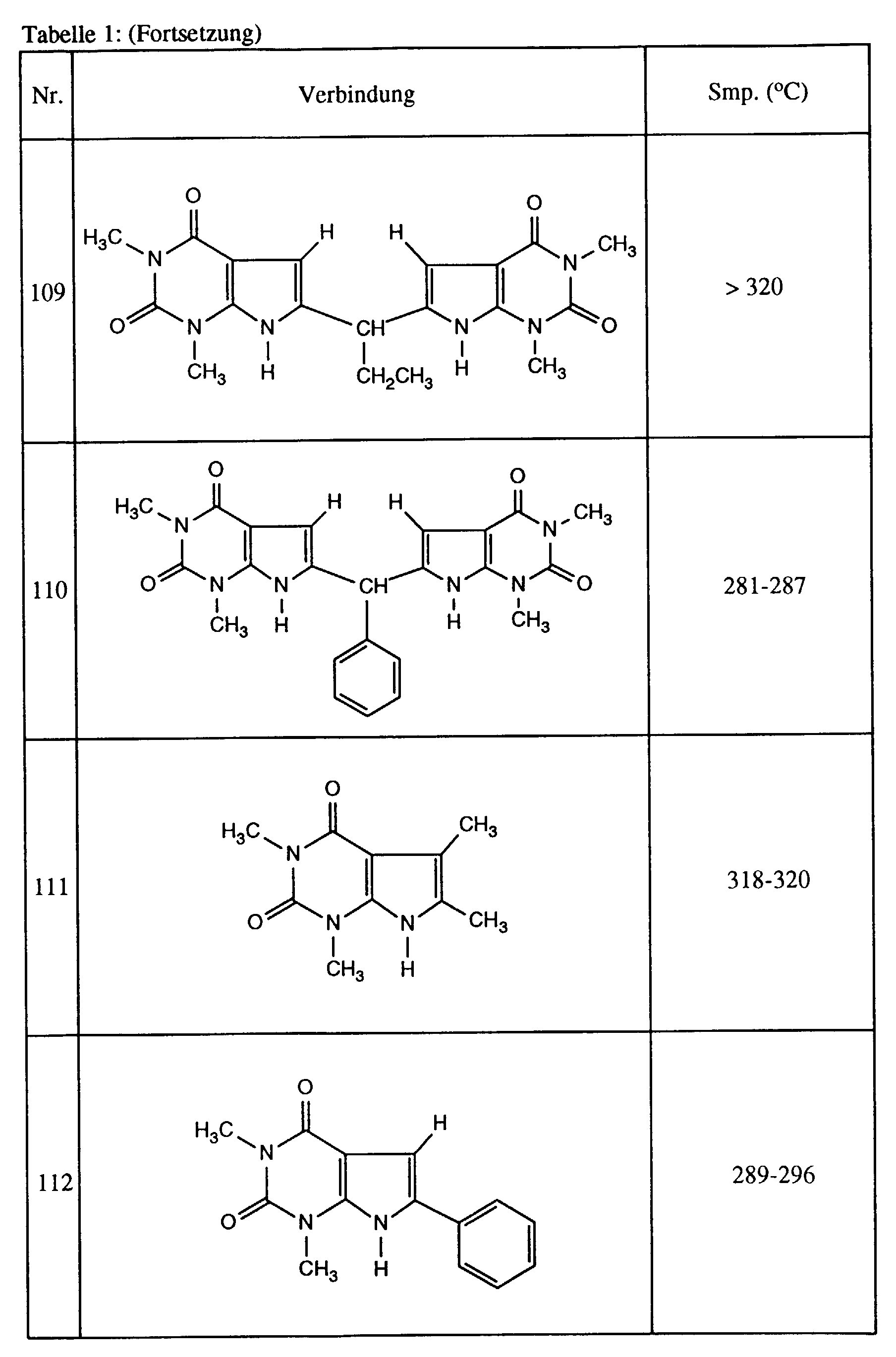

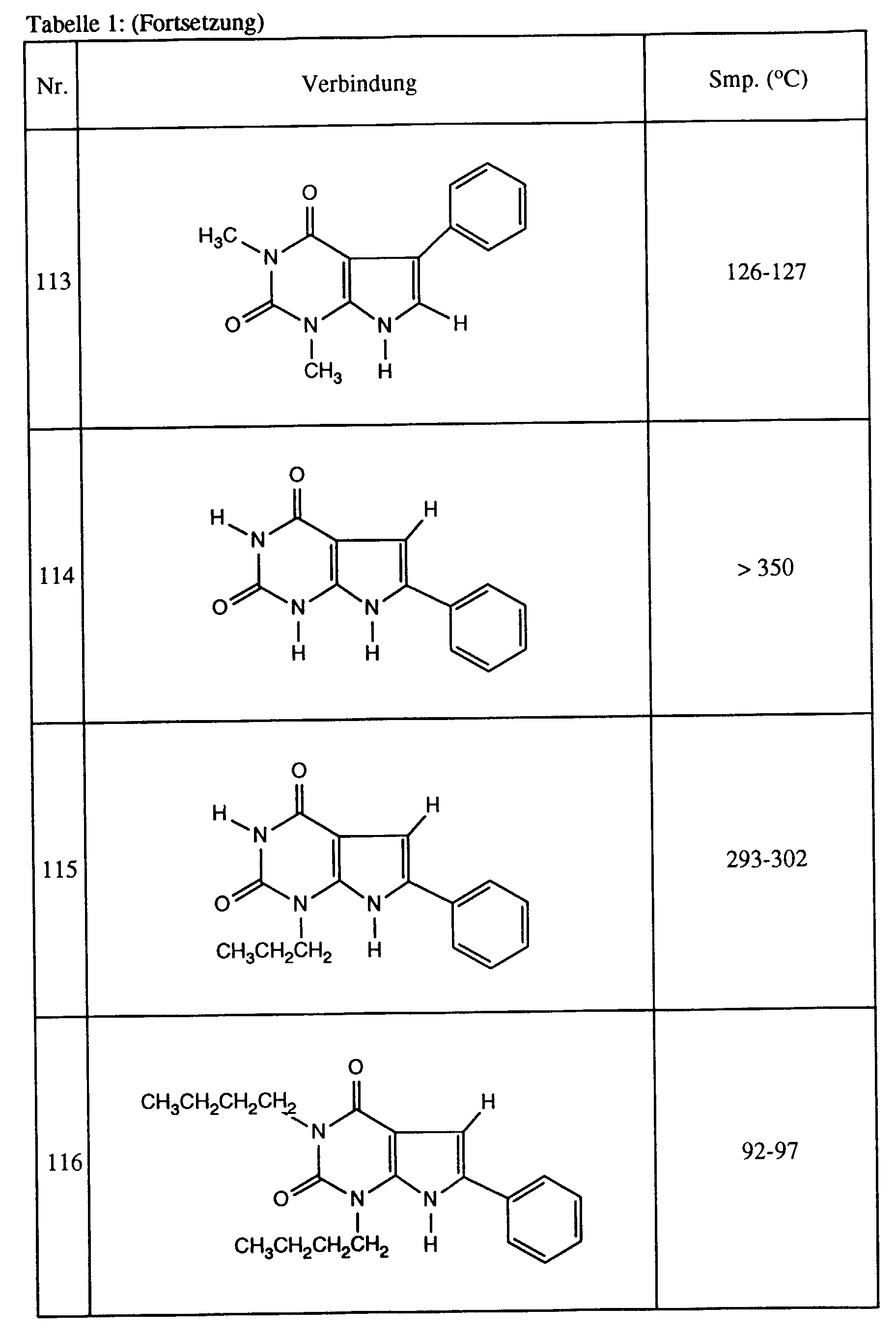

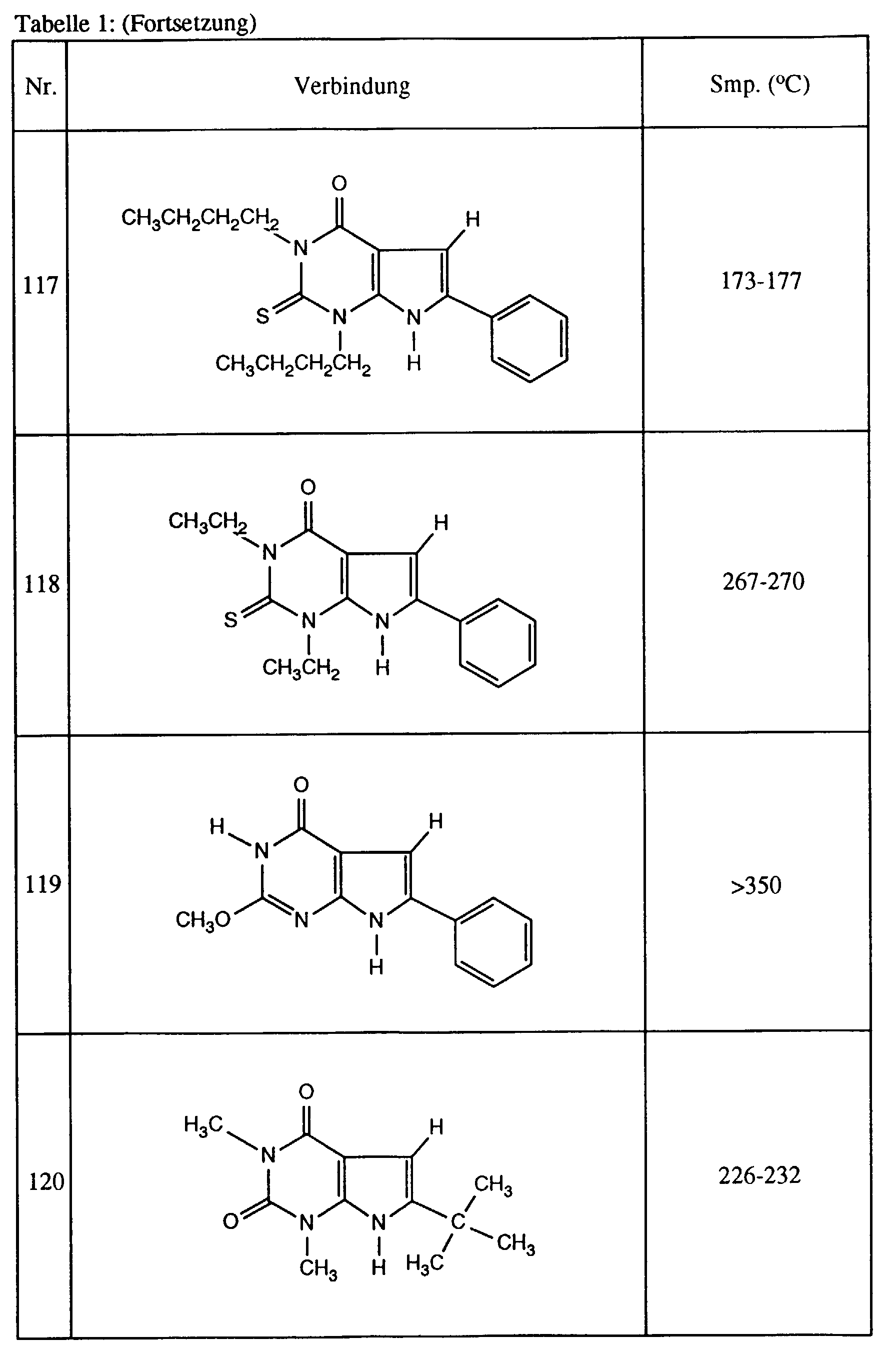

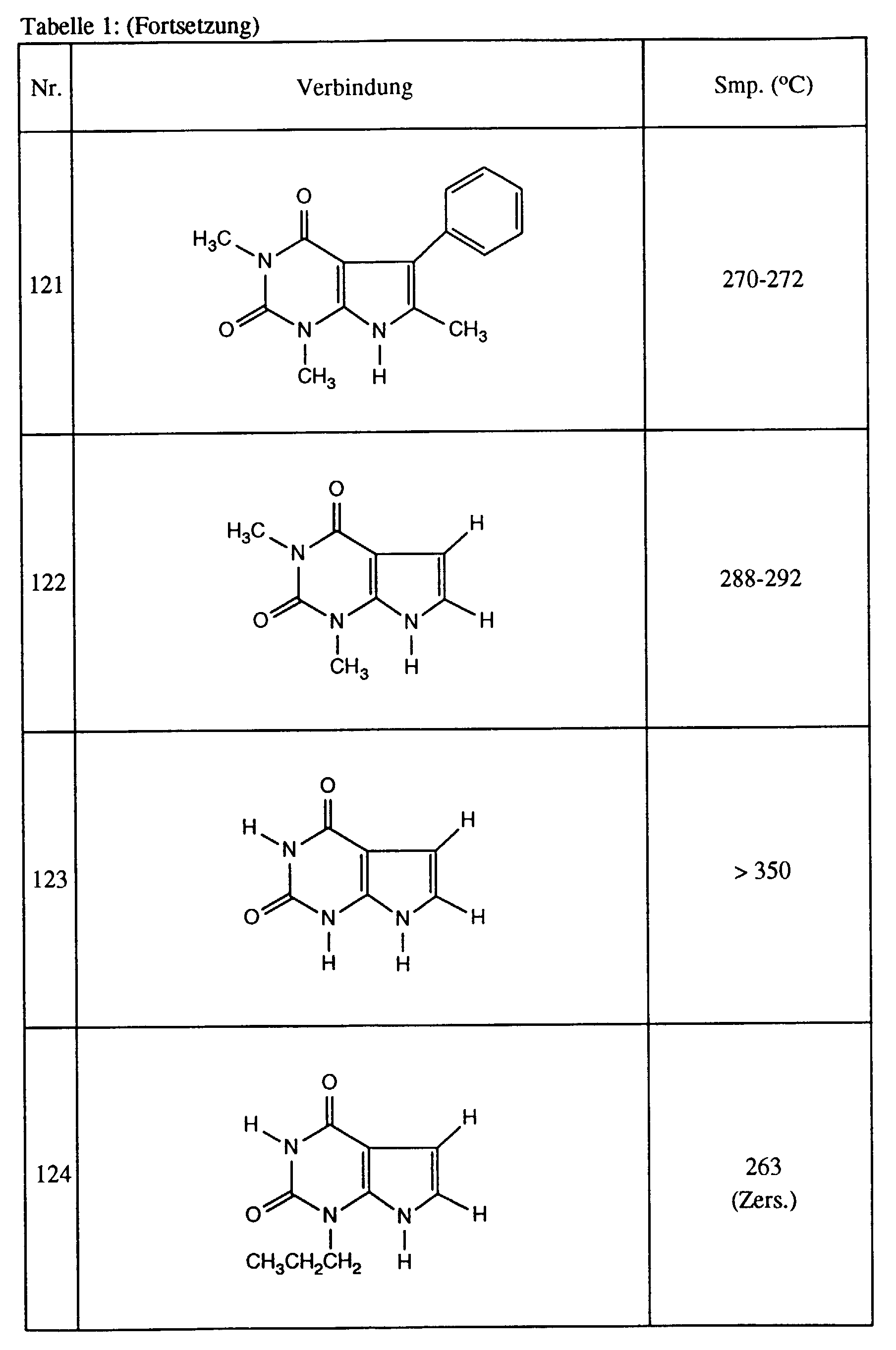

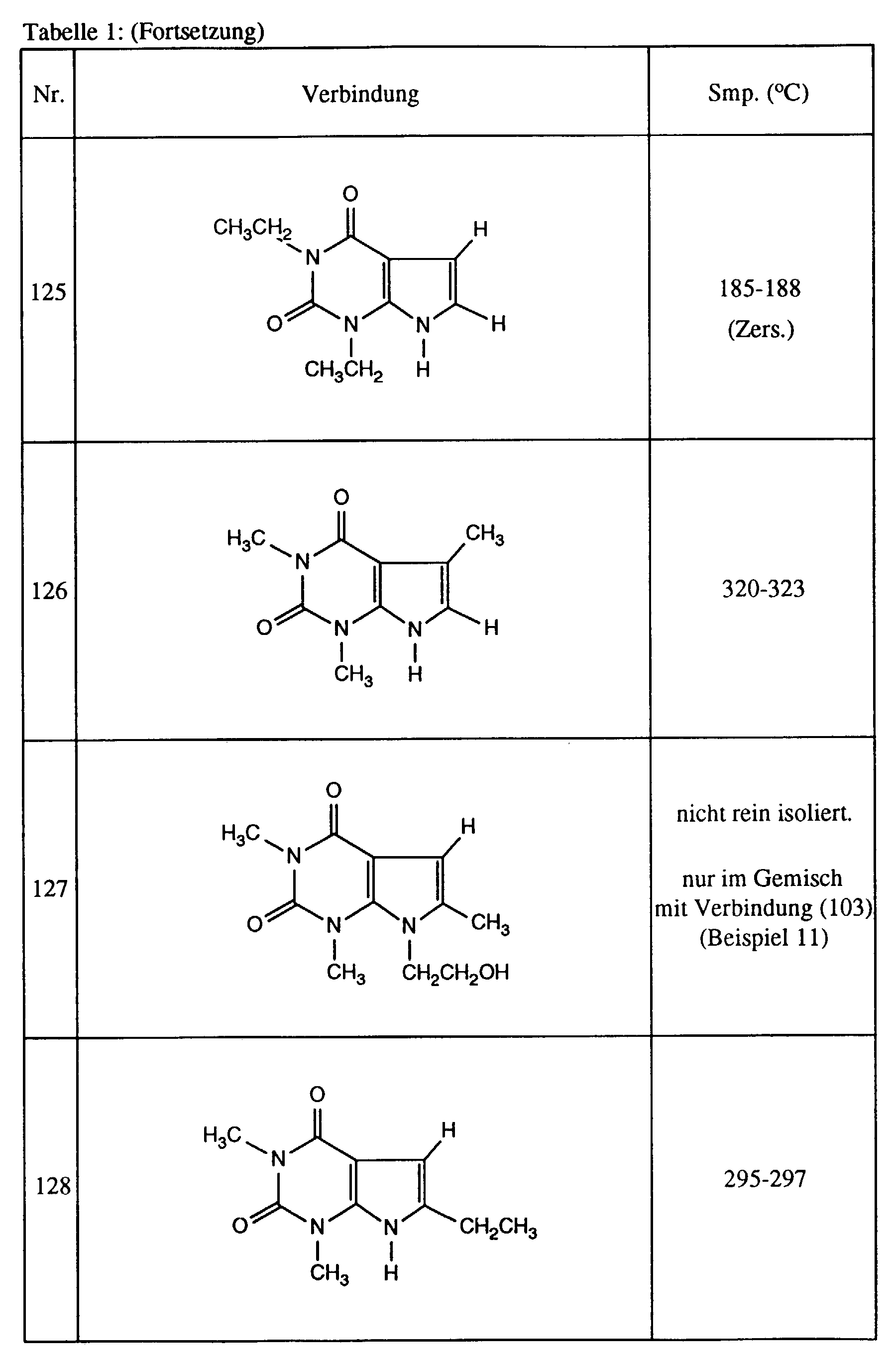

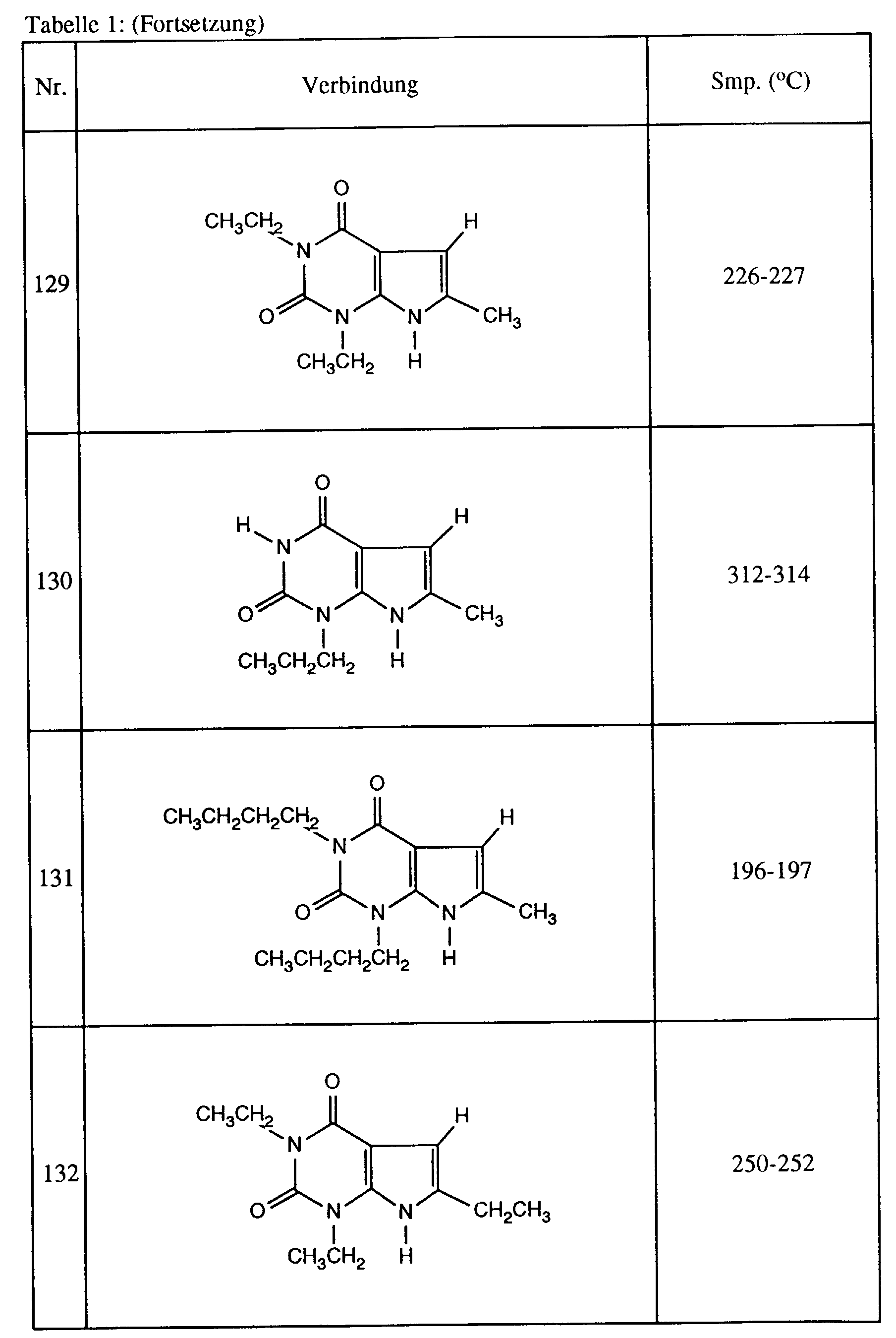

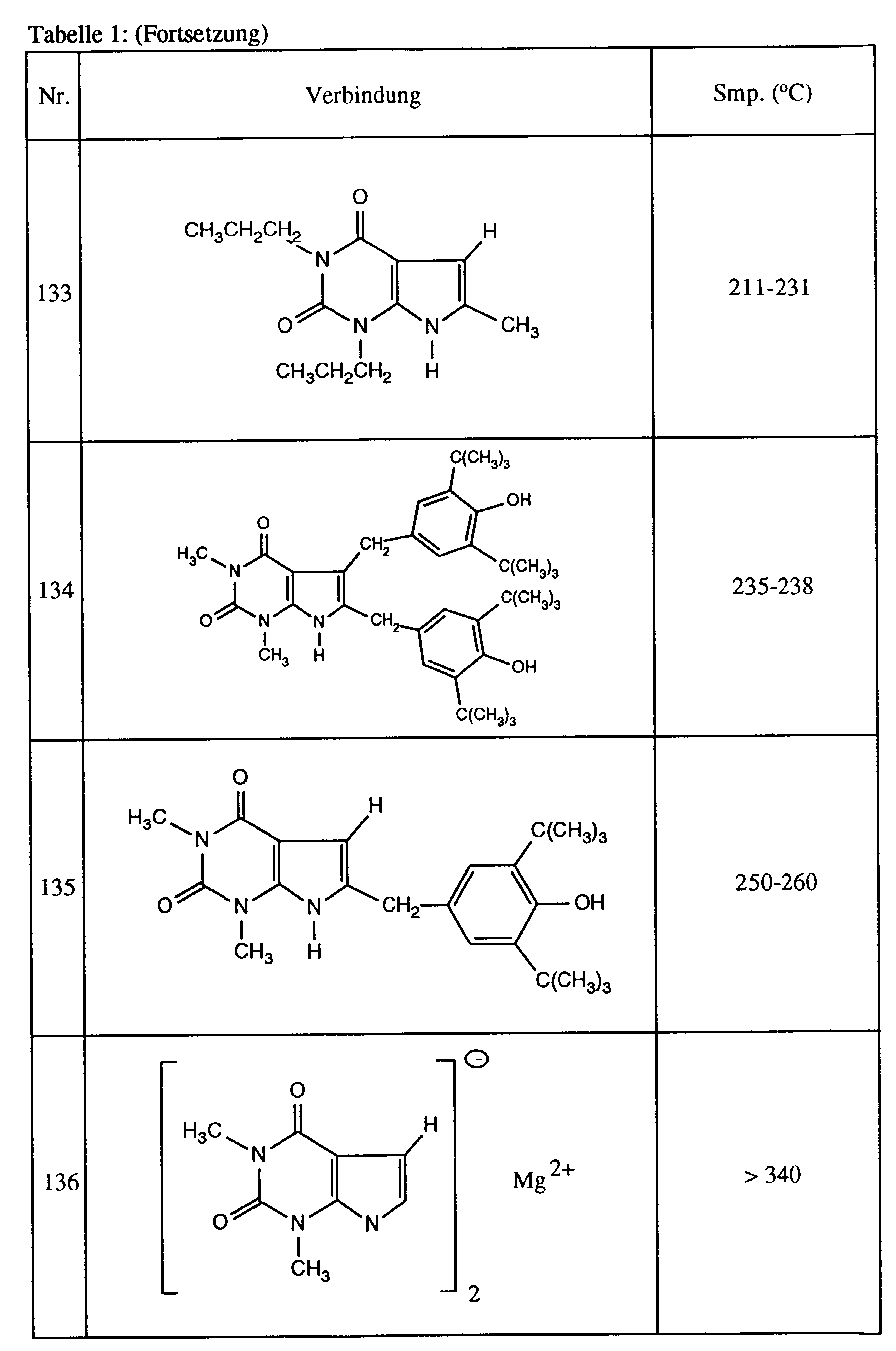

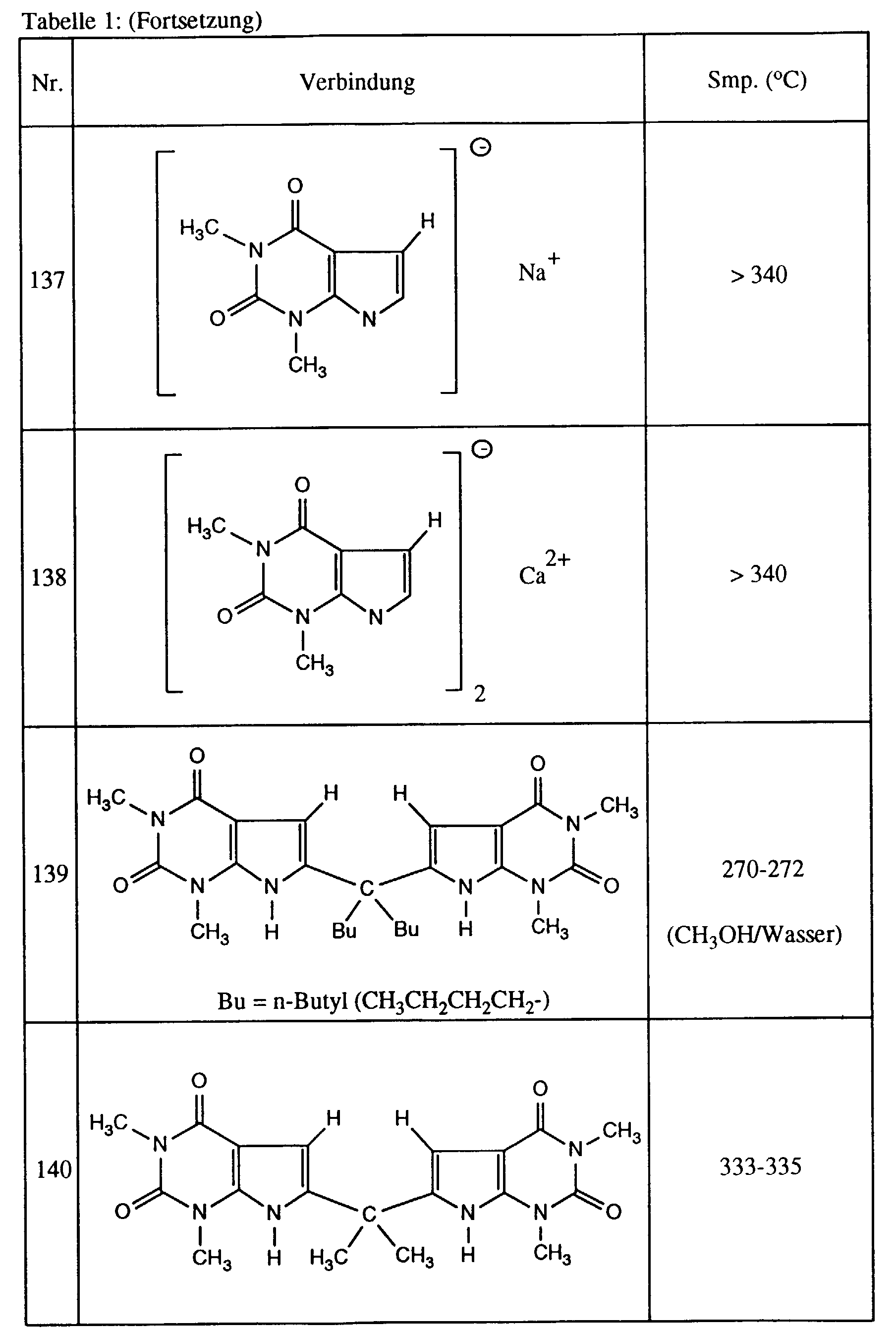

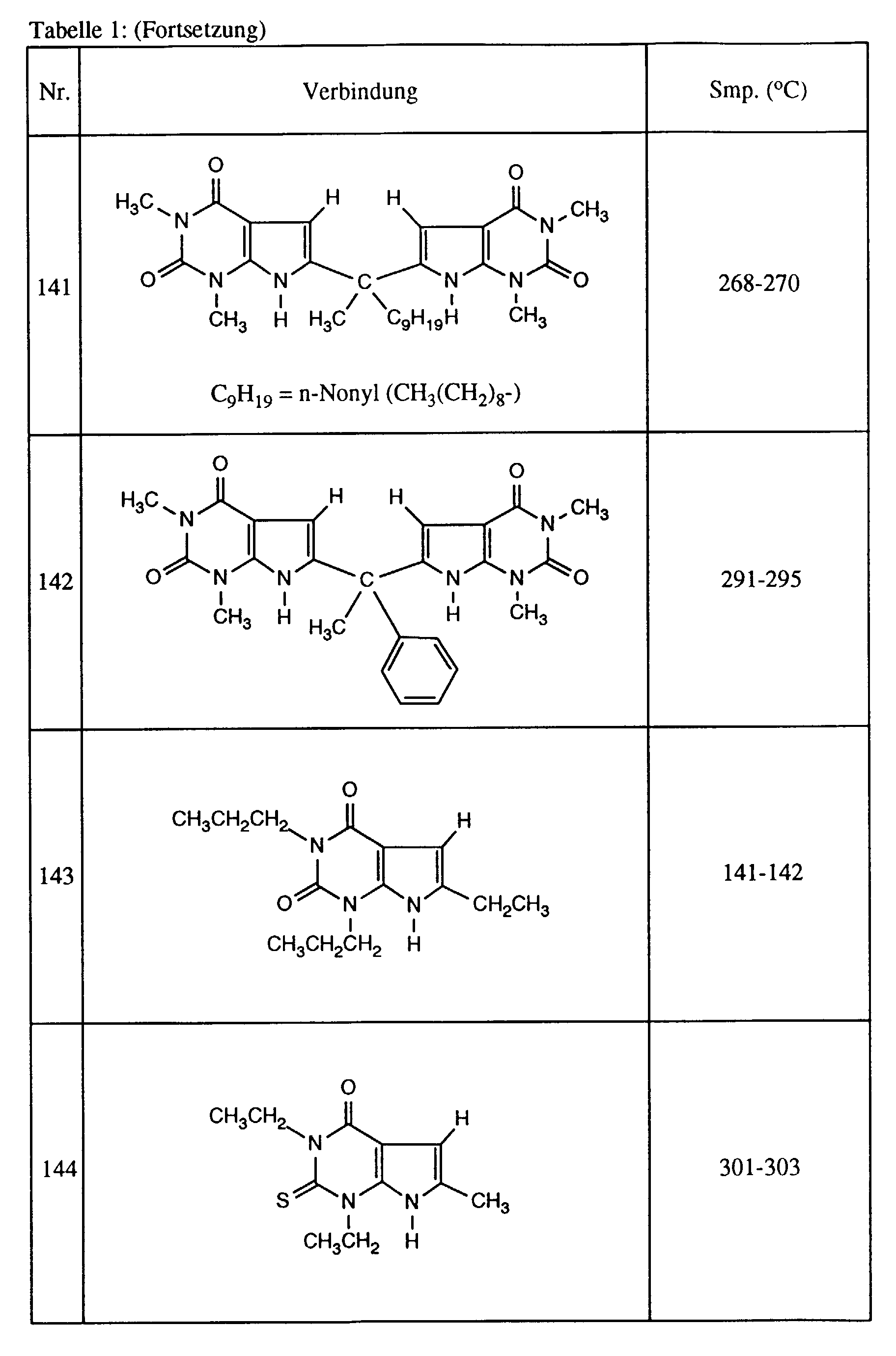

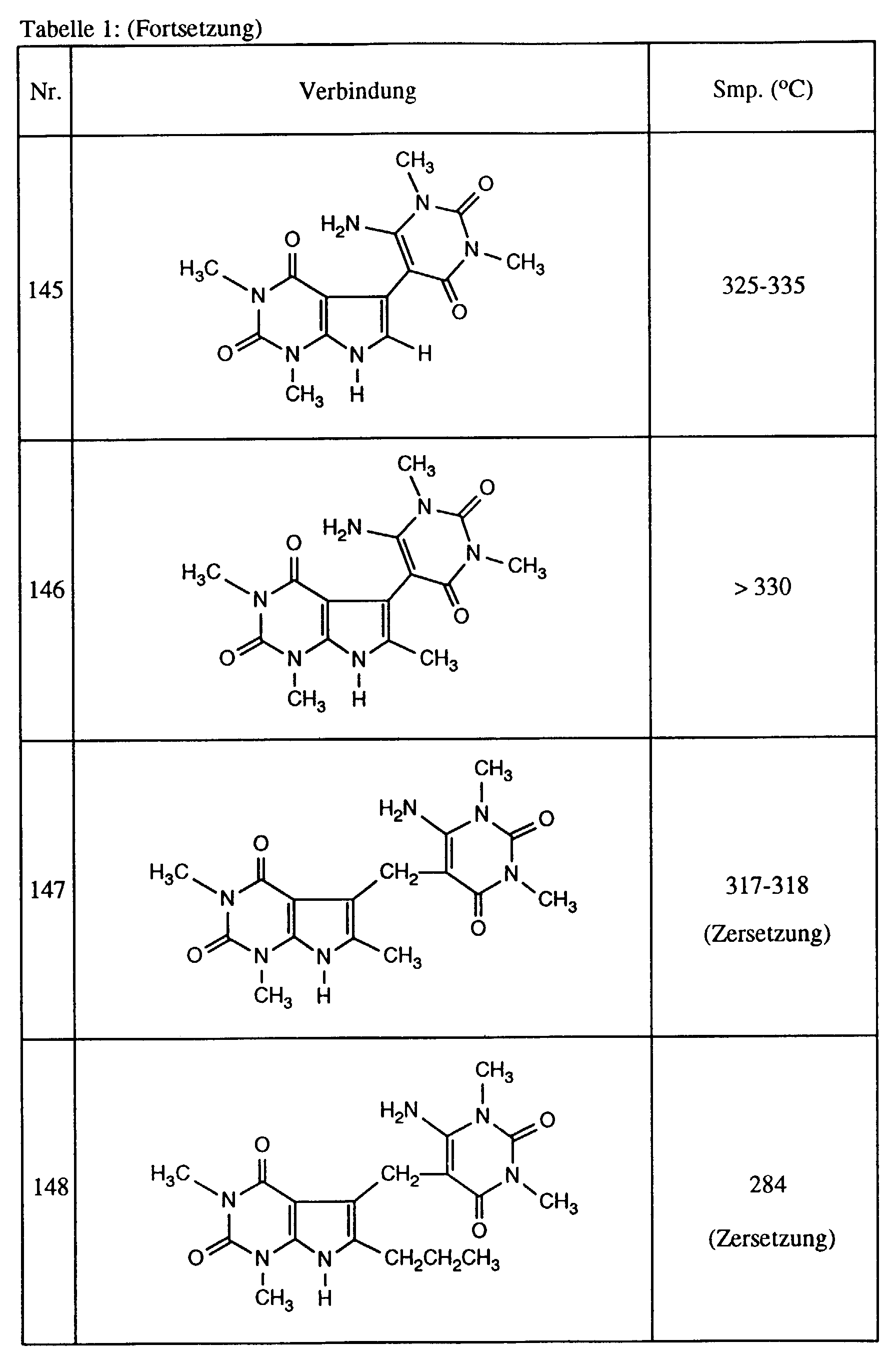

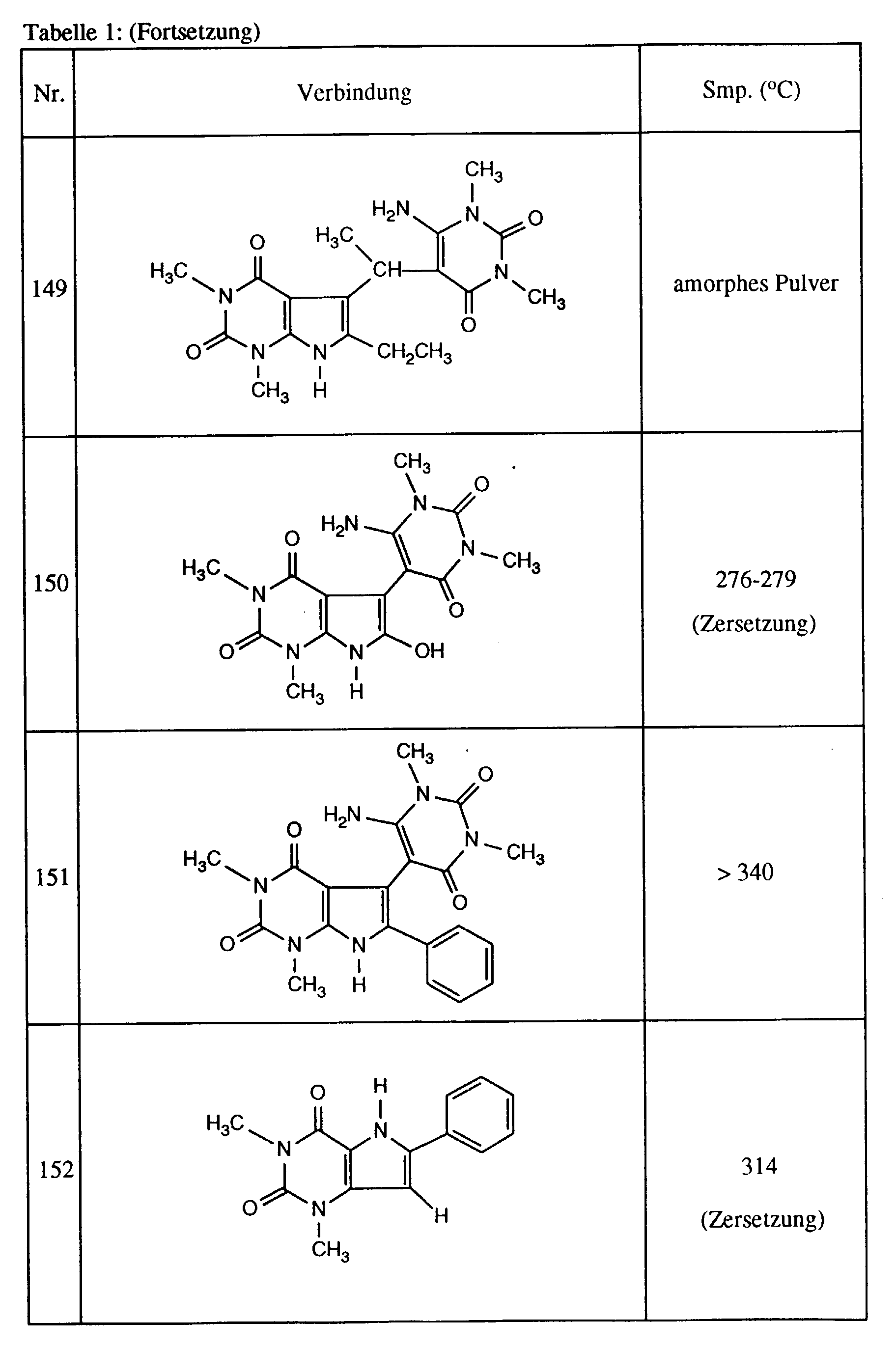

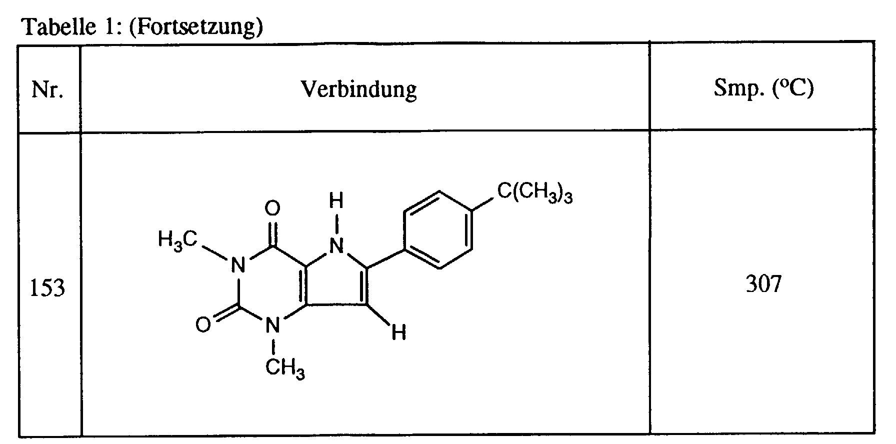

### Beispiel 28: Hitzetest bei 190°C.

Eine Trockenmischung bestehend aus 100,0 Teilen Polyvinylchlorid (S-PVC, K-Wert 60), 5,0 Teilen epoxidiertes Sojabohnenöl, 0,8 Teilen eines 1:1 Gemisches von Didecylphenylphosphit und Decyldiphenylphosphit; 0,2 Teilen Montansäureester und 1,0 Teilen eines erfindungsgemässen Stabilisators gemäss Tabelle 1 wird auf einem Mischwalzwerk 5 Minuten bei 170°C plastifiziert Anschliessend werden aus der Mitte der Folie 0,5 mm dicke Prüfmuster geschnitten und in einem Mathis-Thermo-Takter bei 190°C für die in der Tabelle 2 angegebenen Zeit thermisch belastet Von diesen Prüfmustern wird der Yellowness Index (YI) nach ASTM D 1925-70 bestimmt. Niedrige YI-Werte bedeuten wenig Verfärbung, hohe YI-Werte starke Verfärbung der Muster. Je geringer die Verfärbung ist, desto wirksamer ist der Stabilisator. Die Resultate sind in der Tabelle 2 zusammengefasst.

**Tabelle 2:**

| Hitzetest, YI-Werte nach Belastung bei 190°C | | | | | | | |
|---|---|---|---|---|---|---|---|
| Stabilisator | Dauer (Minuten) | | | | | | |
| | 0 | 5 | 10 | 15 | 20 | 25 | 30 |
| - | 53,4 | 66,5 | 81,7 | 114,6 | 125,8 | 143,4 | zerstört |
| 103 | 10,9 | 11,9 | 14,2 | 17,4 | 23,6 | 35,9 | 42,0 |
| 112 | 15,3 | 23,2 | 29,2 | 34,1 | 48,2 | 56,1 | 62,9 |
| 128 | 11,3 | 11,4 | 14,9 | 22,2 | 30,8 | 41,2 | 51,2 |
| 129 | 5,9 | 6,0 | 7,5 | 11,5 | 18,7 | 30,3 | 43,8 |
| 130 | 22,0 | 28,3 | 33,1 | 36,9 | 45,7 | 53,8 | 55,2 |
| 131 | 7,3 | 6,9 | 8,9 | 14,5 | 23,4 | 34,1 | 46,6 |
| 132 | 9,1 | 11,5 | 14,4 | 22,5 | 34,7 | 50,1 | 64,4 |

Eine weitere Bestimmung der Stabilität erfolgt durch den Dehydrochlorierungstest, welcher in Anlehnung an DIN 53381, Bl. 3, durchgeführt wird. Hierbei wird bei 190°C die Zeit bis zum Ansteigen der Dehydrochlorierungskurve (Induktionszeit, IT) und die Zeit bis zum Überschreiten einer Leitfähigkeit von 200 µS (Stabilisierungszeit, ST) bestimmt. Die Resultate sind in Tabelle 3 zusammengefasst Je grösser die Werte, desto wirksamer ist der Stabilisator.

**Tabelle 3:**

| Dehydrochlorierungstest bei 190°C | | |
|---|---|---|
| Stabilisator | Induktionszeit (IT) in Minuten | Stabilisierungszeit (ST) in Minuten |
| - | 46,0 | 50,5 |
| 103 | 83,0 | 89,5 |
| 112 | 69,0 | 76,0 |
| 128 | 75,0 | 82,5 |
| 129 | 79,0 | 86,0 |
| 130 | 63,5 | 68,0 |
| 131 | 74,0 | 81,5 |
| 132 | 71,5 | 79,5 |

Eine weitere Bestimmung der Stabilität erfolgt durch Verpressen der Filme bei 180°C und 200 bar während 3 Minuten zu 3 mm dicken Platten (Pressplattentest), deren Yellowness-Index gemäss ASTM D 1925-70 bestimmt wird. Die Resultate sind in Tabelle 4 zusammengefasst. Niedrige YI-Werte bedeuten wenig Verfärbung, hohe YI-Werte starke Verfärbung der Muster. Je geringer die Verfärbung ist, desto wirksamer ist der Stabilisator.

**Tabelle 4:**

| Pressplattentest | |
|---|---|
| Stabilisator | YI-Werte |
| - | 134,0 |
| 103 | 44,3 |
| 112 | 59,6 |
| 128 | 46,6 |
| 129 | 30,2 |
| 131 | 35,3 |
| 132 | 46,0 |

## Patentansprüche

1. Zusammensetzung enthaltend
a) ein chlorhaltiges Polymerisat und
b) mindestens eine Verbindung, die mindestens einen Rest der Formel I 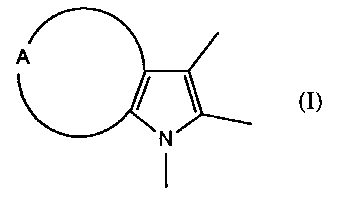
enthält, worin A die Ergänzung zu einem unsubstituierten oder substituierten sechs-gliedrigen heterocyclischen Ring mit zwei Stickstoffringatomen bedeutet.

2. Zusammensetzung gemäss Anspruch 1, enthaltend als Komponente b) mindestens eine Verbindung der Formel II 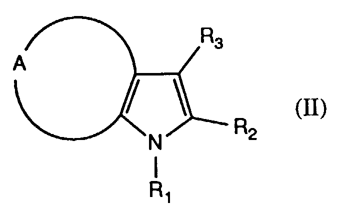 worin
A eine Gruppe der Formel III oder IV 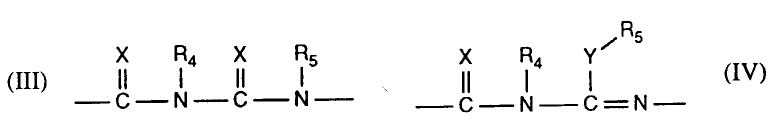 bedeutet,
X Sauerstoff oder Schwefel ist,
Y Sauerstoff, Schwefel oder 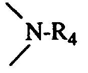 bedeutet,
R₁ Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; ein Alkalimetall oder ein Erdalkalimetall darstellt,
R₂ und R₃ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl;
unsubstituiertes oder am Phenylring durch ein bis drei Reste R₆ substituiertes C₇-C₉-Phenylalkyl; 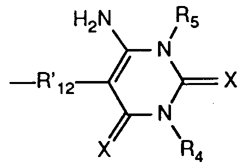 oder einen Rest der Formel V oder VI 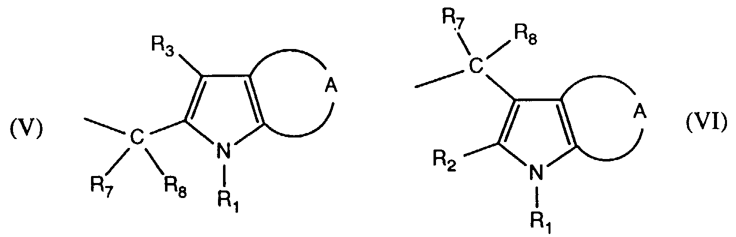
darstellen, worin A und R₁ die oben angegebene Bedeutung besitzen, oder ferner einer der Reste R₂ und R₃ Hydroxy, Formyl, C₁-C₁₂-Alkoxy, unsubstituiertes oder durch ein bis drei Reste R₆ substituiertes Phenoxy: C₁-C₁₂-Alkanoyloxy, unsubstituiertes oder durch ein bis drei Reste R₆ substituiertes Benzoyloxy; 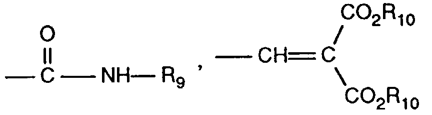 oder -CH=N-R₁₁ darstellt,
R₄ und R₅ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl;
unsubstituiertes oder am Phenylring durch ein bis drei Reste R₆ substituiertes C₇-C₉-Phenylalkyl bedeuten,
R₆ C₁-C₄-Alkyl, C₁-C₄-Alkoxy, C₅-C₈-Cycloalkyl, Hydroxy oder Chlor darstellt,
R₇ und R₈ unabhängig voneinander Wasserstoff, CF₃, C₁-C₁₂-Alkyl, Phenyl oder C₇-C₉-Phenylalkyl darstellen, oder R₇ und R₈ zusammen mit dem C-Atom, an das sie gebunden sind, einen unsubstituierten oder durch C₁-C₄-Alkyl substituierten C₅-C₁₂-Cycloalkylidenring bilden,
R₉ unsubstituiertes oder durch ein bis drei Reste R₆ substituiertes Phenyl bedeutet;
R₁₀ Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeutet,
R₁₁ C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl darstellt,
R'₁₂ eine direkte Bindung oder 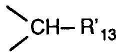 bedeutet, und
R'₁₃ Wasserstoff oder C₁-C₈-Alky darstellt.

3. Zusammensetzung gemäss Anspruch 2, worin die Verbindung der Formel II eine Verbindung der Formel VII, VIIa oder VIII 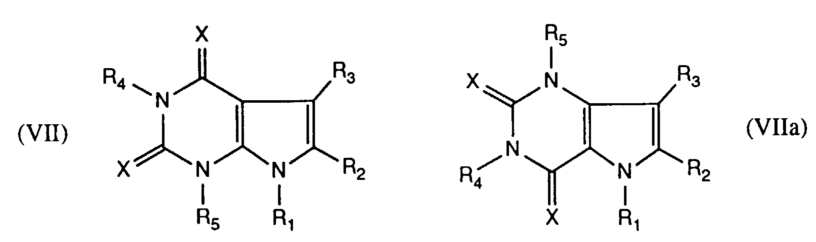 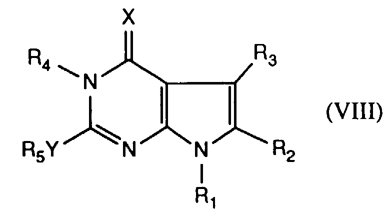 bedeutet, worin
X Sauerstoff oder Schwefel ist,
Y Sauerstoff, Schwefel oder 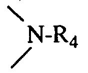 bedeutet,
R₁ Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, C₇-C₉-Phenylalkyl, ein Alkalimetall oder ein Erdalkalimetall darstellt,
R₂ und R₃ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, unsubstituiertes oder am Phenylring durch ein bis drei Reste R₆ substituiertes C₇-C₉-Phennylal; 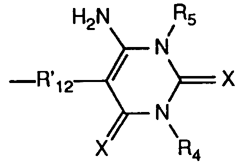 oder einen Rest der Formel IX, X, XI oder XII 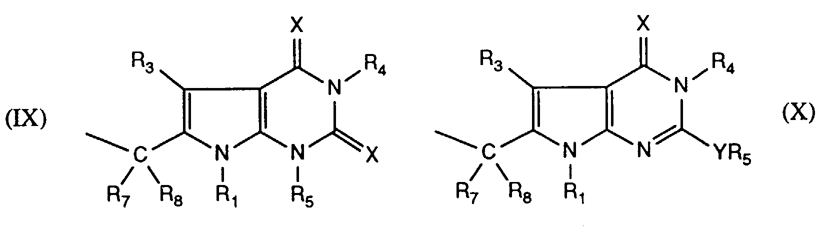 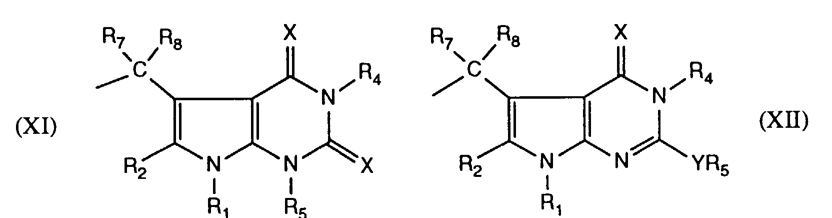 darstellt, worin X, Y, R₁, R₂ und R₃ die oben angegebene Bedeutung besitzen, oder ferner einer der Reste R₂ und R₃ Hydroxy, Formyl, C₁-C₁₀-Alkoxy, Phenoxy, C₁-C₁₀-Alkanoyloxy, Benzoyloxy, 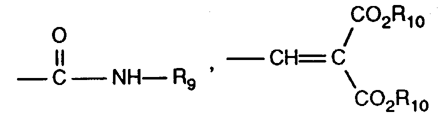 oder -CH=N-R₁₁ darstellt,
R₄ und R₅ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl oder C₇-C₉-Phenylalkyl bedeuten,
R₆ C₁-C₄-Alkyl, C₁-C₄-Alkoxy, Cyclohexyl, Hydroxy oder Chlor darstellt,
R₇ und R₈ unabhängig voneinander Wasserstoff, C₁-C₁₀-Alkyl, Phenyl oder C₇-C₉-Phenylalkyl darstellen,
R₉ Phenyl bedeutet,
R₁₀ C₁-C₁₀-Alkyl, durch Sauerstoff unterbrochenes C₂-C₁₂-Alkyl; C₃-C₆-Alkenyl, C₅-C₈-Cycloalkyl, Phenyl oder C₇-C₉-Phenylalkyl bedeutet,
R₁₁ C₁-C₁₀-Alkyl, C₅-C₈-Cycloalkyl, Phenyl oder C₇-C₉-Phenylalkyl darstellt,
R'₁₂ eine direkte Bindung oder 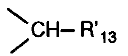 bedeutet, und
R'₁₃ Wasserstoff oder C₁-C₄-Alkyl darstellt.

4. Zusammensetzung gemäss Anspruch 2, worin X und Y Sauerstoff bedeuten.

5. Zusammensetzung gemäss Anspruch 2, worin R₁ Wasserstoff, C₁-C₄-Alkyl, Benzyl, 2-Hydroxyethyl, Natrium, Magnesium oder Calcium darstellt.

6. Zusammensetzung gemäss Anspruch 2, worin
R2 Wasserstoff, C₁-C₄-Alkyl, Phenyl, 2,6-Di-tert-butyl-4-hydroxybenzyl, Hydroxy, 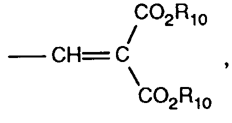 -CH=N-R₁₁ oder einen Rest der Formel IX 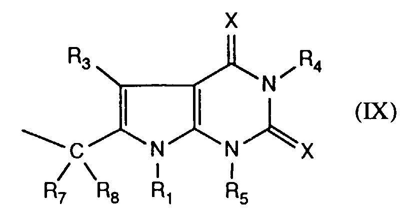 darstellt, worin X, R₁, R₄, R₅, R₇, R₈, R₁₀ und R₁₁ wie in Anspruch 2 definiert sind, und
R₃ Wasserstoff, Hydroxy, C₁-C₄-Alkyl, Phenyl, 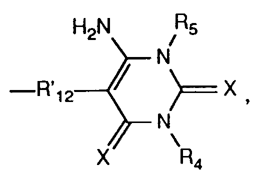 worin X, R₄, R₅ und R'₁₂ wie in Anspruch 2 definiert sind, oder 2,6-Di-tert-butyl-4-hydroxybenzyl bedeutet.

7. Zusammensetzung gemäss Anspruch 2, worin R₄ und R₅ unabhängig voneinander Wasserstoff oder C₁-C₄-Alkyl bedeuten.

8. Zusammensetzung gemäss Anspruch 3, worin
X und Y Sauerstoff sind,
R₁ Wasserstoff, C₁-C₄-Alkyl, durch Hydroxy substituiertes C₂-C₆-Alkyl, ein Alkalimetall oder Erdalkalimetall darstellt,
R₂ Wasserstoff, C₁-C₈-Alkyl, unsubstituiertes oder mit C₁-C₄-Alkyl substituiertes Phenyl; 2,6-Di-tert-butyl-4-hydroxybenzyl, Hydroxy, 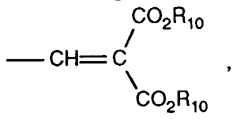 -CH=N-R₁₁ oder einen Rest der Formel IX 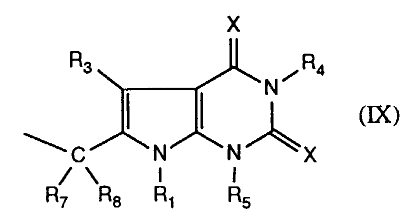 darstellt, worin X und R₁ die oben angegebene Bedeutung besitzen,
R₃ Wasserstoff, Hydroxy, C₁-C₈-Alkyl, unsubstituiertes oder mit C₁-C₄-Alkyl substituiertes Phenyl; 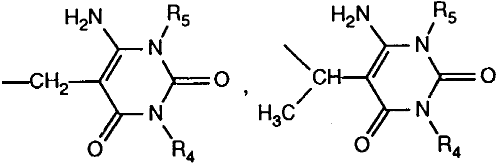 oder 2,6-Di-tert-butyl-4-hydroxybenzyl bedeutet,
R₄ und R₅ unabhängig voneinander Wasserstoff, C₁-C₄-Alkyl, C₃-C₆-Alkenyl oder Benzyl bedeuten,
R₇ und R₈ unabhängig voneinander Wasserstoff, C₁-C₉-Alkyl oder Phenyl darstellen, oder
R₇ und R₈ zusammen mit dem C-Atom, an das sie gebunden sind, einen C₅-C₈-Cycloalkylidenring bilden,
R₁₀ C₁-C₈-Alkyl bedeutet, und
R₁₁ C₇-C₉-Phenylalkyl darstellt.

9. Zusammensetzung gemäss Anspruch 3, worin
X und Y Sauerstoff sind,
R₁ Wasserstoff, 2-Hydroxyethyl, Natrium, Magnesium oder Calcium darstellt,
R₂ Wasserstoff, C₁-C₄-Alkyl, Phenyl, 2,6-Di-tert-butyl-4-hydroxybenzyl, Hydroxy, 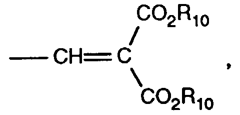 -CH=N-R₁₁ oder einen Rest der Formel IX 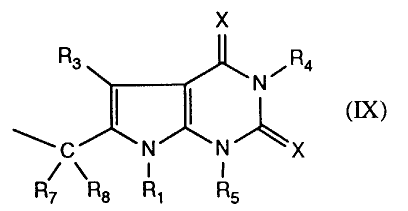
darstellt, worin X und R₁ die oben angegebene Bedeutung besitzen,
R₃ Wasserstoff, Hydroxy, C₁-C₄-Alkyl, Phenyl, 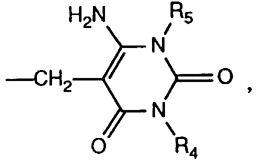 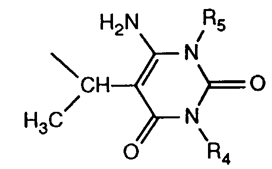 oder 2,6-Di-tert-butyl-4-hydroxybenzyl bedeutet,
R₄ und R₅ unabhängig voneinander Wasserstoff oder C₁-C₄-Alkyl bedeuten,
R₇ und R₈ unabhängig voneinander Wasserstoff, C₁-C₉-Alkyl oder Phenyl darstellen,
R₁₀ C₁-C₄-Alkyl bedeutet, und
R₁₁ Benzyl darstellt.

10. Zusammensetzung gemäss Anspruch 3, worin die Verbindung der Formel VII 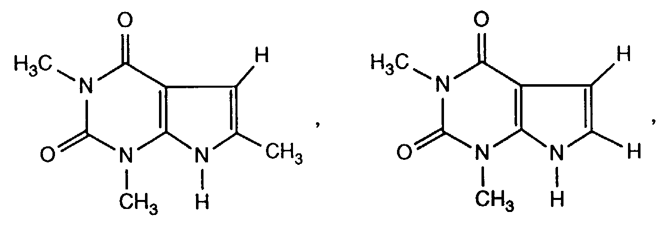 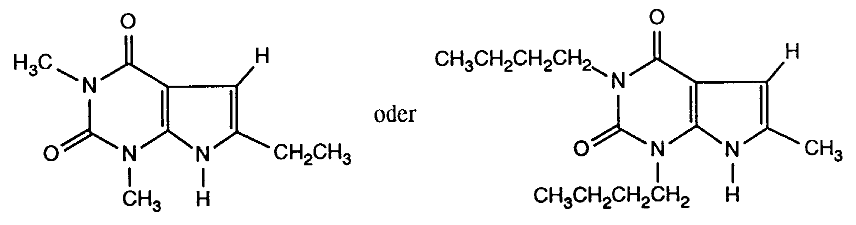 bedeutet.

11. Zusammensetzung gemäss Anspruch 1, worin das chlorhaltige Polymerisat Polyvinylchlorid ist.

12. Zusammensetzung gemäss Anspruch 1, enthaltend zusätzlich mindestens ein Me(II)-Carboxylat und/oder Me(II)-Phenolat, wobei Me(II) Ba, Ca, Mg oder Zn bedeutet

13. Zusammensetzung gemäss Anspruch 1, enthaltend zusätzlich eine Epoxyverbindung und/oder ein Phosphit.

14. Zusammensetzung gemäss Anspruch 1, enthaltend zusätzlich Gleitmittel, Pigmente, Modifikatoren, Verarbeitungshilfen, Füllstoffe, Antioxidantien und/oder Lichtschutzmittel.

15. Verwendung von Verbindungen, die mindestens einen in Anspruch 1 definierten Rest der Formel I enthalten, zum Stabilisieren eines chlorhaltigen Polymerisats gegen oxidativen, thermischen oder/und lichtinduzierten Abbau.

16. Verfahren zum Stabilisieren eines chlorhaltigen Polymerisats und/oder dessen Recyclats gegen oxidativen, thermischen oder/und lichtinduzierten Abbau, dadurch gekennzeichnet, dass man diesem mindestens eine Verbindung, die mindestens einen in Anspruch 1 definierten Rest der Formel I enthält, einverleibt oder auf dieses aufbringt.

17. Verfahren zur Herstellung von Verbindungen der Formel XIII oder XIV 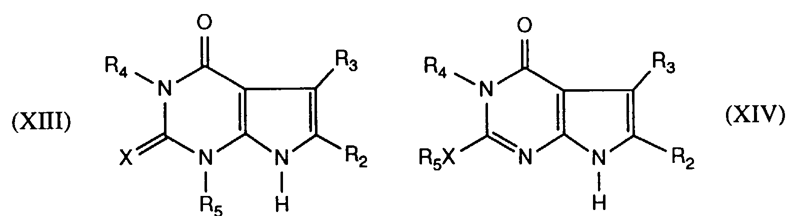 worin die allgemeinen Symbole wie in Anspruch 2 definiert sind, dadurch gekennzeichnet, dass eine Verbindung der Formel XV oder XVI 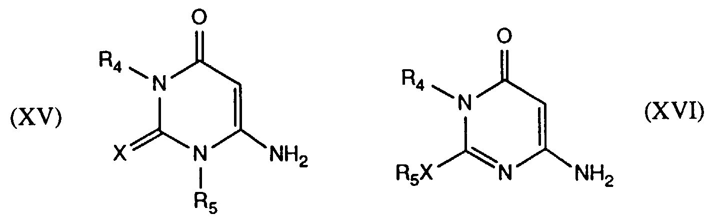 worin die allgemeinen Symbole wie in Anspruch 2 definiert sind, mit einer Verbindung Formel XVIIa, XVIIb, XVIIc, XVIId, XVIIe oder XVIIf 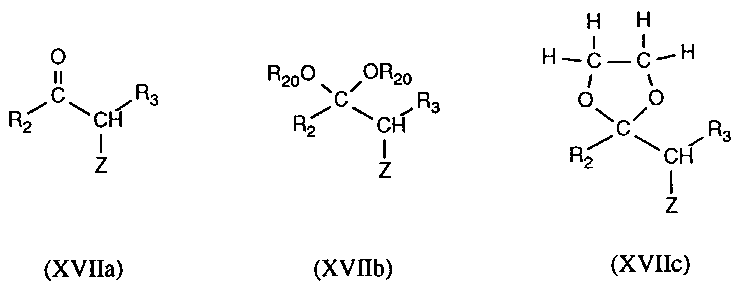 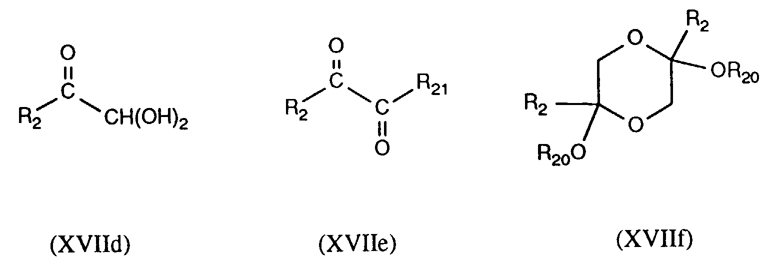 worin R₂ und R₃ wie in Anspruch 2 definiert sind, R₂₀ C₁-C₄-Alkyl darstellt, R₂₁ C₁-C₄-Alkyl bedeutet, und Z eine Abgangsgruppe darstellt, in Gegenwart eines Ammoniumsalzes umgesetzt wird.

18. Verfahren gemäss Anspruch 17, worin die Abgangsgruppe Z Halogen, Hydroxy oder 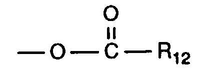 bedeutet,
R₁₂ Wasserstoff, C₁-C₄-Alkyl, unsubstituiertes oder mit R₁₃ substituiertes Phenyl darstellt; und
R₁₃ Halogen, Nitro, CF₃, C₁-C₄-Alkyl oder C₁-C₄-Alkoxy bedeutet.

19. Verfahren gemäss Anspruch 17, worin das Ammoniumsalz eine Verbindung der Formel XVIII 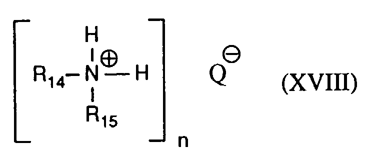 darstellt, worin
n 1 oder 2 bedeutet,
R₁₄ und R₁₅ unabhängig voneinander Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl;
C₃-C₆-Alkenyl, unsubstituiertes oder durch ein bis drei Reste R₁₆ substituiertes C₅-C₈-Cycloalkyl; unsubstituiertes oder durch ein bis drei Reste R₁₆ substituiertes Phenyl;
unsubstituiertes oder am Phenylring durch ein bis drei Reste R₁₆ substituiertes C₇-C₉-Phenylalkyl darstellt; oder R₁₄ und R₁₅ zusammen mit dem Stickstoffatom an das sie gebunden sind, einen C₄-C₇-Cycloalkylenring bilden, der zusätzlich mit Sauerstoff oder Schwefel unterbrochen sein kann,
R₁₆ C₁-C₄-Alkyl, C₁-C₄-Alkoxy, C₅-C₈-Cycloalkyl, Hydroxy oder Chlor darstellt, und
wenn n = 1 ist,
Q^{⊖} R₁₇-CO₂^{⊖} oder 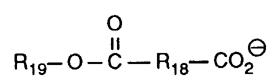 bedeutet,
wenn n = 2 ist,
Q^{⊖} Carbonat oder ^{⊖}O₂C-R₁₈-CO₂^{⊖} darstellt,
R₁₇ Wasserstoff, Hydroxy, C₁-C₁₈-Alkyl, Phenyl oder Benzyl bedeutet,
R₁₈ eine direkte Bindung oder C₁-C₆-Alkylen darstellt, und
R₁₉ Wasserstoff oder C₁-C₁₈-Alkyl bedeutet.

20. Verfahren gemäss Anspruch 17, worin nach Beendigung der Umsetzung ein Gemisch enthaltend einen Niederschlag der Verbindung der Formel XIII oder XIV und eine Mutterlauge gebildet wird, die Mutterlauge von dem Niederschlag getrennt wird und als Reaktionsmedium in einer weiteren Umsetzung gemäss Anspruch 17 verwendet wird.

21. Verbindungen der Formel II 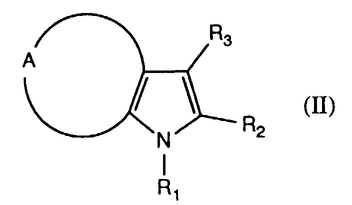 worin
A eine Gruppe der Formel III oder IV 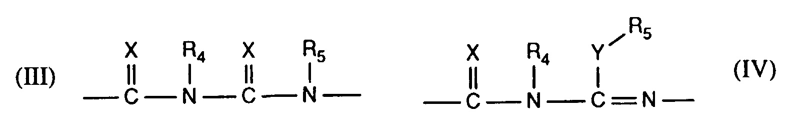 bedeutet,
X Sauerstoff oder Schwefel ist,
Y Sauerstoff, Schwefel oder 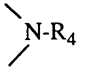 bedeutet,
R₁ Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; ein Alkalimetall oder ein Erdalkalimetall darstellt,
R₂ und R₃ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl; 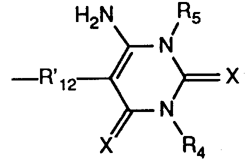 oder einen Rest der Formel V oder VI 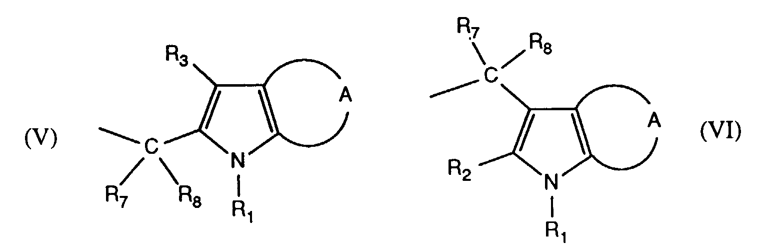 darstellen, worin A und R₁ die obcn angegebene Bedeutung besitzen, oder ferner einer der Reste R₂ und R₃ Hydroxy, Formyl, C₁-C₁₂-Alkoxy, unsubstituiertes oder durch ein bis drei Reste R₆ substituiertes Phenoxy; C₁-C₁₂-Alkanoyloxy, unsubstituiertes oder durch ein bis drei Reste R₆ substituiertes Benzoyloxy; 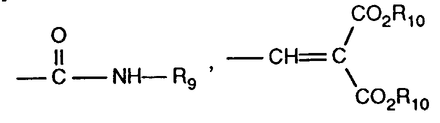 oder -CH=N-R₁₁ darstellt,
R₄ und R₅ unabhängig voneinander Wasserstoff, C₁-C₁₂-Alkyl, durch Sauerstoff, Schwefel oder Carboxy unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeuten,
R₆ C₁-C₄-Alkyl, C₁-C₄-Alkoxy, C₅-C₈-Cycloalkyl, Hydroxy oder Chlor darstellt,
R₇ und R₈ unabhängig voneinander Wasserstoff, CF₃, C₁-C₁₂-Alkyl, Phenyl oder C₇-C₉-Phenylalkyl darstellen, oder R₇ und R₈ zusammen mit dem C-Atom, an das sie gebunden sind, einen unsubstituierten oder durch C₁-C₄-Alkyl substituierten C₅-C₁₂-Cycloalkylidenring bilden,
R₉ unsubstituiertes oder durch ein bis drei Reste R₆ substituiertes Phenyl bedeutet;
R₁₀ Wasserstoff, C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl bedeutet,
R₁₁ C₁-C₁₂-Alkyl, durch Sauerstoff oder Schwefel unterbrochenes C₂-C₁₂-Alkyl; durch Hydroxy substituiertes C₁-C₁₂-Alkyl; C₃-C₆-Alkenyl, unsubstituiertes oder durch ein bis drei Reste R₆ substituiertes C₅-C₈-Cycloalkyl; unsubstituiertes oder durch ein bis drei Reste R₆ substituiertes Phenyl; unsubstituiertes oder am Phenylring durch ein bis drei Reste R₆ substituiertes C₇-C₉-Phenylalkyl darstellt,
R'₁₂ eine direkte Bindung oder 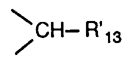 bedeutet, und
R'₁₃ Wasserstoff oder C₁-C₈-Alkyl darstellt; mit der Bedingung, dass die Verbindungen der Formel VII 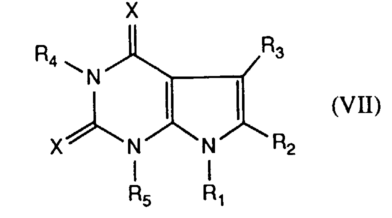 worin
X Sauerstoff bedeutet,
R₁ Wasserstoff, Methyl oder Phenyl darstellt,
R₂ Wasserstoff, Hydroxy, Formyl, C₁-C₃-Alkyl, Phenyl, 4-Chlorphenyl, 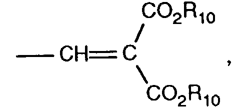 -CH=N-R₁₁ oder -CH₂CH(CO₂CH₂CH₃)₂ bedeutet,
R₃ Wasserstoff, Hydroxy, Formyl, C₁-C₅-Alkyl, Phenyl oder -CH₂CO₂R₁₀ darstellt,
R₄ und R₅ unabhängig voneinander Wasserstoff, C₁-C₄-Alkyl, Allyl, Phenyl, 4-Methylphenyl oder Benzyl bedeuten,
R₁₀ C₁-C₄-Alkyl darstellt,
R₁₁ Methyl, Allyl, Benzyl oder Phenyl bedeutet, und die Verbindung (152) 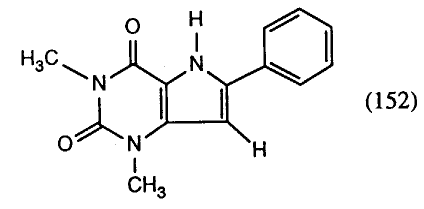 ausgeschlossen sind, wobei die Verbindungen 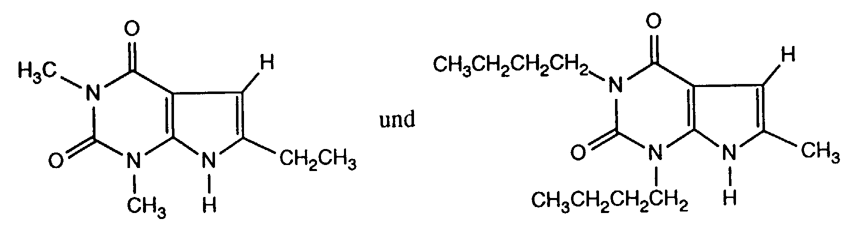 nicht ausgeschlossen sind.

22. Verbindungen gemäss Anspruch 21, worin die Verbindung der Formel II eine Verbindung der Formel VII, VIIa oder VIII 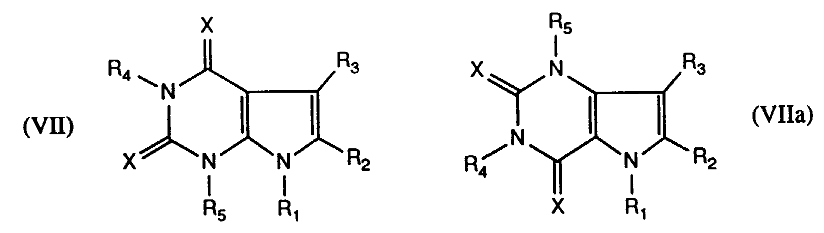 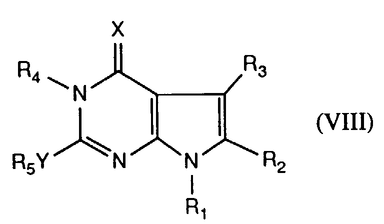 bedeutet, worin
X Sauerstoff oder Schwefel ist,
Y Sauerstoff, Schwefel oder 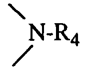 bedeutet,
R₁ Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl, C₇-C₉-Phenylalkyl, ein Alkalimetall oder ein Erdalkalimetall darstellt,
R₂ und R₃ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenvl. unsubstituiertes oder am Phenylring durch ein bis drei Reste R₆ substituiertes C₇-C₉-Phenylalkyl; 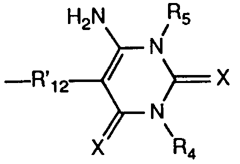 oder einen Rest der Formel IX, X, XI oder XII 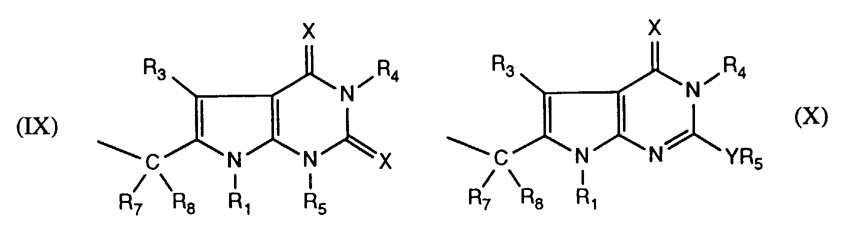 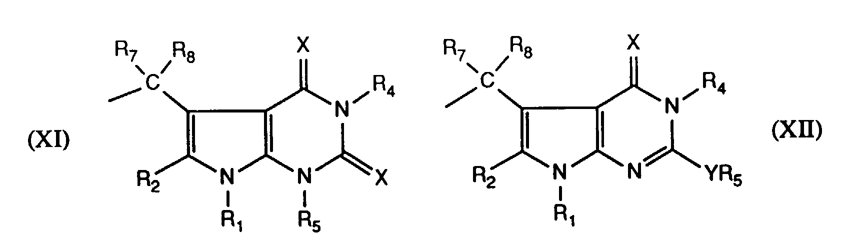 darstellt, worin X, Y, R₁, R₂ und R₃ die oben angegebene Bedeutung besitzen, oder ferner einer der Reste R₂ und R₃ Hydroxy, Formyl, C₁-C₁₀-Alkoxy, Phenoxy, C₁-C₁₀-Alkanoyloxy, Benzoyloxy, 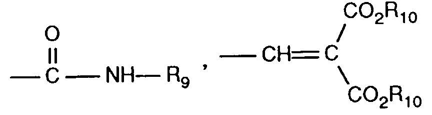 oder -CH=N-R₁₁ darstellt,
R₄ und R₅ unabhängig voneinander Wasserstoff, C₁-C₁₀-Alkyl, durch Sauerstoff unterbrochenes C₂-C₁₂-Alkyl; durch Hydroxy substituiertes C₁-C₁₀-Alkyl; C₃-C₆-Alkenyl, C₅-C₈-Cycloalkyl, Phenyl oder C₇-C₉-Phenylalkyl bedeuten,
R₆ C₁-C₄-Alkyl, C₁-C₄-Alkoxy, Cyclohexyl, Hydroxy oder Chlor darstellt,
R₇ und R₈ unabhängig voneinander Wasserstoff, C₁-C₁₀-Alkyl, Phenyl oder C₇-C₉-Phenylalkyl darstellen,
R₉ Phenyl bedeutet,
R₁₀ C₁-C₁₀-Alkyl, durch Sauerstoff unterbrochenes C₂-C₁₂-Alkyl; C₃-C₆-Alkenyl, C₅-C₈-Cycloalkyl, Phenyl oder C₇-C₉-Phenylalkyl bedeutet,
R₁₁ C₁-C₁₀-Alkyl, C₅-C₈-Cycloalkyl, Phenyl oder C₇-C₉-Phenylalkyl darstellt,
R'₁₂ eine direkte Bindung oder 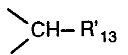 bedeutet, und
R'₁₃ Wasserstoff oder C₁-C₄-Alkyl darstellt.

23. Verbindungen gemäss Anspruch 22, worin R₄ und R₅ unabhängig voneinander Wasserstoff oder C₁-C₄-Alkyl bedeuten.

24. Verbindungen gemäss Anspruch 22, worin
X und Y Sauerstoff sind,
R₁ Wasserstoff, 2-Hydroxyethyl, Natrium, Magnesium oder Calcium darstellt,
R₂ Wasserstoff, C₁-C₄-Alkyl, Phenyl, 2,6-Di-tert-butyl-4-hydroxybenzyl, Hydroxy, 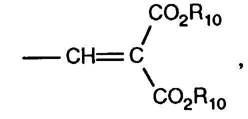 -CH=N-R₁₁ oder einen Rest der Formel IX 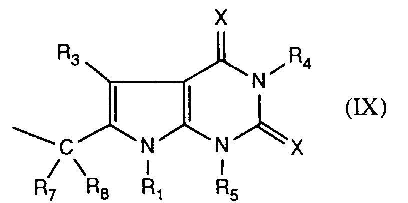 darstellt, worin X und R₁ die oben angegebene Bedeutung besitzen,
R₃ Wasserstoff, Hydroxy, C₁-C₄-Alkyl, Phenyl, 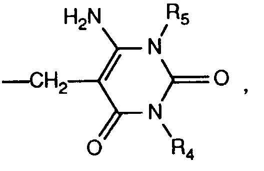 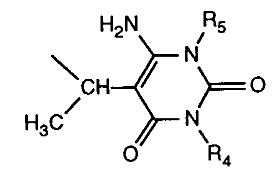 oder 2,6-Di-tert-butyl-4-hydroxybenzyl bedeutet,
R₄ und R₅ unabhängig voneinander Wasserstoff oder C₁-C₄-Alkyl bedeuten,
R₇ und R₈ unabhängig voneinander Wasserstoff, C₁-C₉-Alkyl oder Phenyl darstellen,
R₁₀ C₁-C₄-Alkyl bedeutet, und
R₁₁ Benzyl darstellt.
